(19) Europäisches Patentamt
European Patent Office
Office européen des brevets

(11) **EP 3 149 640 B1**

(12) **EUROPEAN PATENT SPECIFICATION**

(45) Date of publication and mention
of the grant of the patent:
**04.09.2019 Bulletin 2019/36**

(21) Application number: **15750841.7**

(22) Date of filing: **27.05.2015**

(51) Int Cl.:
*C12Q 1/6869* (2018.01)    *C12Q 1/6827* (2018.01)
*G16B 20/00* (2019.01)    *G16B 30/00* (2019.01)
*G16B 40/00* (2019.01)    *G16H 50/30* (2018.01)

(86) International application number:
**PCT/US2015/032550**

(87) International publication number:
**WO 2015/183872 (03.12.2015 Gazette 2015/48)**

(54) **CHROMOSOME REPRESENTATION DETERMINATIONS**

CHROMOSOMENDARSTELLUNGSBESTIMMUNGEN

DÉTERMINATIONS DE REPRÉSENTATION DE CHROMOSOMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.05.2014 US 201462005811 P**

(43) Date of publication of application:
**05.04.2017 Bulletin 2017/14**

(73) Proprietor: **Sequenom, Inc.
San Diego, CA 92121 (US)**

(72) Inventors:
• **ZHAO, Chen
San Diego, CA 92129 (US)**
• **DECIU, Cosmin
San Diego, CA 92121 (US)**

(74) Representative: **Vossius & Partner
Patentanwälte Rechtsanwälte mbB
Siebertstrasse 3
81675 München (DE)**

(56) References cited:
**WO-A1-2014/039556    US-A1- 2013 130 921
US-A1- 2013 150 253**

• **STEPHANIE C. Y. YU ET AL: "Noninvasive
Prenatal Molecular Karyotyping from Maternal
Plasma", PLOS ONE, vol. 8, no. 4, 17 April 2013
(2013-04-17), pages 1-8, XP055160833, DOI:
10.1371/journal.pone.0060968**
• **Sander Bollen: "BioConductor: Microarray
versus Next-Generation Sequencing toolsets", ,
11 February 2014 (2014-02-11), pages 1-14,
XP055215571, Retrieved from the Internet:
URL:http://dspace.library.uu.nl/bitstream/
handle/1874/290489/Sander_Bollen_writing_a
ssignment.pdf [retrieved on 2015-09-23]**

**Description**

Field

The present invention is defined by the claims. Technology described herein pertains in part to diagnostic tests that make use of sequence reads generated by a sequencing process. As described herein, a component used to generate a chromosome representation can be based on counts of sequence reads not aligned to a reference genome.

Background

Genetic information of living organisms (e.g., animals, plants and microorganisms) and other forms of replicating genetic information (e.g., viruses) is encoded in deoxyribonucleic acid (DNA) or ribonucleic acid (RNA). Genetic information is a succession of nucleotides or modified nucleotides representing the primary structure of chemical or hypothetical nucleic acids. In humans, the complete genome contains about 30,000 genes located on twenty-four (24) chromosomes (see The Human Genome, T. Strachan, BIOS Scientific Publishers, 1992). Each gene encodes a specific protein, which after expression via transcription and translation fulfills a specific biochemical function within a living cell.

Many medical conditions are caused by one or more genetic variations. Certain genetic variations cause medical conditions that include, for example, hemophilia, thalassemia, Duchenne Muscular Dystrophy (DMD), Huntington's Disease (HD), Alzheimer's Disease and Cystic Fibrosis (CF) (Human Genome Mutations, D. N. Cooper and M. Krawczak, BIOS Publishers, 1993). Such genetic diseases can result from an addition, substitution, or deletion of a single nucleotide in DNA of a particular gene. Certain birth defects are caused by a chromosomal abnormality, also referred to as an aneuploidy, such as Trisomy 21 (Down's Syndrome), Trisomy 13 (Patau Syndrome), Trisomy 18 (Edward's Syndrome), Monosomy X (Turner's Syndrome) and certain sex chromosome aneuploidies such as Klinefelter's Syndrome (XXY), for example. Another genetic variation is fetal gender, which can often be determined based on sex chromosomes X and Y. Some genetic variations may predispose an individual to, or cause, any of a number of diseases such as, for example, diabetes, arteriosclerosis, obesity, various autoimmune diseases and cancer (e.g., colorectal, breast, ovarian, lung).

Identifying one or more genetic variations (e.g., copy number variations) or variances can lead to diagnosis of, or determining predisposition to, a particular medical condition. Identifying a genetic variance can result in facilitating a medical decision and/or employing a helpful medical procedure. In certain easpects, identification of one or more genetic variations or variances involves the analysis of cell-free DNA. Cell-free DNA (CF-DNA) is composed of DNA fragments that originate from cell death and circulate in peripheral blood. High concentrations of CF-DNA can be indicative of certain clinical conditions such as cancer, trauma, burns, myocardial infarction, stroke, sepsis, infection, and other illnesses. Additionally, cell-free fetal DNA (CFF-DNA) can be detected in the maternal bloodstream and used for various noninvasive prenatal diagnostics. For example, US 2013/0130921 relates to a kit, a device and a method for detecting the copy number of fetal chromosomes and tumor cell chromosomes. Moreover, US 2013/0150253 provides methods, processes and apparatuses for the non-invasive assessment of genetic variations.

Summary

The present invention is defined by the claims. Accordingly, the present invention relates to a method for determining a sequence read count representation of a genome segment for a diagnostic test, comprising: (a) generating a count of nucleic acid sequence reads for a genome segment, which sequence reads are reads of nucleic acid from a test sample from a subject having the genome, thereby providing a count A for the segment; (b) generating a count of nucleic acid sequence reads for the genome or a subset of the genome, thereby providing a count B for the genome or subset of the genome, wherein the count B is a count of sequence reads that is determined by a process that does not include aligning the sequence reads to a reference genome; and (c) determining a count representation for the segment as a ratio of the count A to the count $B$, wherein one or more or all of (a), (b) and (c) are performed by a computer, wherein the count B is: (i) a count of total reads generated by a nucleic acid sequencing process used to sequence the nucleic acid from the test sample; (ii) a count of a fraction of total reads generated by a nucleic acid sequencing process used to sequence the nucleic acid from the test sample; (iii) a count of the total reads of (i) or the fraction of the total reads of (ii), less reads filtered according to a quality control metric for the sequencing process; (iv) a count of the total reads of (i) or the fraction of the total reads of (ii), weighted according to a quality control metric for the sequencing process; (v) a count of the total reads of (i) or the fraction of the total reads of (ii), less reads filtered according to read base content; (vi) a count of the total reads of (i) or the fraction of the total reads of (ii), weighted according to read base content; or (vii) a count of reads that match polynucleotides in a listing, wherein the reads are determined to match or not match the polynucleotides in the listing in a process comprising comparing reads to the polynucleotides in the listing, wherein the reads are the total reads in (i), the fraction of total reads in (ii), the total reads of (i) or the fraction of the total reads of (ii) less the reads filtered according to the quality control metric of (iii), the total reads of (i) or the fraction of the total reads of (ii) weighted according to the quality control metric of (iv), the total reads of (i) or the fraction of the total reads of (ii) less the reads filtered according to the read base content of (v), or the total reads of (i) or the fraction of the total reads of (ii) weighted according to the read base content of (vi).

Also provided herein, in certain aspects, are methods for determining a sequence read count representation of a genome segment for a diagnostic test, comprising (a) generating a count of nucleic acid sequence reads for a genome segment, which sequence reads are reads of nucleic acid from a test sample from a subject having the genome, thereby providing a count A for the segment; (b) generating a count of nucleic acid sequence reads for the genome or a subset of the genome, thereby providing a count $B$ for the genome or subset of the genome, where the count $B$ is a count of sequence reads not aligned to a reference genome; and (c) determining a count representation for the segment as a ratio of the count A to the count B.

Certain aspects of the technology are described further in the following description, examples, claims and drawings.

Brief Description of the Drawings

The drawings illustrate aspects of the technology and are not limiting. For clarity and ease of illustration, the drawings are not made to scale and, in some instances, various aspects may be shown exaggerated or enlarged to facilitate an understanding of particular aspects.

FIG. 1 shows a comparison between total number of reads (prior to alignment) and total number of reads (prior to alignment) which pass the chastity filtered.

FIG. 2 shows a comparison between total number of reads (prior to alignment) which pass the chastity filtered and reads which are aligned to all autosomes.

FIG. 3A, FIG. 3B and FIG. 3C show a comparison of z-scores derived from chromosome representation calculated using autosomes and calculated using pre-alignment reads, passing chastity-filter, using SPCA normalization, for chromosomes 21, 13, and 18.

FIG. 4 shows a non-limiting example of utilizing a sub-listing of polynucleotides to generate a count representation for a particular target chromosome.

FIG. 5 shows an illustrative aspects of a system in which certain aspects of the technology may be implemented.

Detailed Description

Certain diagnostic tests include processing sequence reads. Sequence reads are relatively short sub-sequences (e.g., about 20 to about 40 base pairs in length) generated by subjecting test sample nucleic acid to a sequencing process. Some diagnostic tests involve determining a chromosome count representation, which is a normalized version of the number of counts attributed to a test chromosome. A chromosome count representation sometimes is expressed as a ratio of (i) the number of sequence reads attributed to a test chromosome (Ntest), to (ii) the number of sequence reads for the genome (e.g., human autosomes and sex chromosomes X and Y) or a subset of the genome larger than the chromosome (e.g., autosomes) (Nref or Ntot). The Ntest and Nref values sometimes are determined by counting the number of reads aligned, or mapped, to a reference genome when determining a chromosome count representation.

It has been determined, as described in greater detail hereafter, that Ntest and/or Nref (also referred to as count A and count B, respectively), can be determined without aligning sequence reads to a reference genome. In addition, methods described herein can be used generally to generate a count representation for a genome segment, where the segment is smaller or larger than a target chromosome, or has the same size and sequence as a target chromosome.

Thus, provided herein are methods for determining a sequence read count representation of a genome segment (i.e., a target segment) for a diagnostic test, that include (a) generating a count of nucleic acid sequence reads for a genome segment, which sequence reads are reads of nucleic acid from a test sample from a subject having the genome, thereby providing a count A for the segment; (b) generating a count of nucleic acid sequence reads for the genome or a subset of the genome, thereby providing a count B for the genome or subset of the genome, where the count A is a count of sequence reads not aligned to a reference genome and/or the count B is a count of sequence reads not aligned to a reference genome; and (c) determining a count representation for the segment as a ratio of the count A to the count B.

Any suitable sample can be utilized for a method described herein. A sample can be from any suitable subject (e.g., human, ape, ungulate, bovine, ovine, equine, caprine, canine, feline, avian, reptilian, domestic animal, or the like). A sample sometimes is from a pregnant female subject bearing a fetus at any stage of gestation (e.g., first, second or third trimester for a human subject), and sometimes is from a post-natal subject. A sample sometimes is from a pregnant subject bearing a fetus that is euploid for all chromosomes, and sometimes is from a pregnant subject bearing a fetus having a chromosome aneuploidy (e.g., one, three (i.e., trisomy (e.g., T21, T18, T13)), or four copies of a chromosome) or other genetic variation. A sample sometimes is a subject having a cell proliferative condition, and sometimes is from a subject not having a cell proliferative condition. Non-limiting examples of cell proliferative conditions include cancers, tumors and dis-regulated cell proliferative conditions of liver cells (e.g., hepatocytes), lung cells, spleen cells, pancreas cells, colon cells, skin cells, bladder cells, eye cells, brain cells, esophagus cells, cells of the head, cells of the neck, cells of the ovary, cells of the testes, prostate cells, placenta cells, epithelial cells, endothelial cells, adipocyte cells, kidney/renal cells, heart cells, muscle cells, blood cells (e.g., white blood cells), central nervous system (CNS) cells, the like and combinations of the foregoing. A nucleic acid analyzed sometimes is isolated cellular nucleic acid from a suitable sample (e.g., buccal cells, biopsy tissue or cells, fetal cells). A nucleic acid analyzed sometimes is isolated circulating cell-free (ccf) nucleic acid from a suitable sample (e.g., blood serum, blood plasma, urine or other body fluid). Nucleic acid isolation processes are available and known in the art.

Processes suitable for sequencing nucleic acid for a diagnostic test are known in the art, and massively parallel sequencing (MPS) processes sometimes are utilized. Non-limiting examples of sequencing processes include Illumina/Solex/HiSeq (e.g., Illumina Genome Analyzer; Genome Analyzer II; HISEQ 2000; HISEQ), SOLiD, Roche/454, PACBIO and/or SMRT, Helicos True Single Molecule Sequencing, Ion Torrent and Ion semiconductor-based sequencing, WildFire, 5500, 5500xl W and/or 5500xl W Genetic Analyzer based technologies; Polony sequencing, Pyrosequencing, Massively Parallel Signature Sequencing (MPSS), RNA polymerase (RNAP) sequencing, LaserGen systems and methods, nanopore-based platforms, chemical-sensitive field effect transistor (CHEMFET) array, electron microscopy-based sequencing (e.g., ZS Genetics, Halcyon Molecular), and nanoball sequencing. Certain sequencing processes are implemented in combination with one or more nucleic acid amplification processes, non-limiting examples of which include polymerase chain reaction (PCR; AFLP-PCR, Allele-specific PCR, Alu-PCR, Asymmetric PCR, Colony PCR, Hot start PCR, Inverse PCR (IPCR), in situ PCR (ISH), Intersequence-specific PCR (ISSR-PCR), Long PCR, Multiplex PCR, Nested PCR, Quantitative PCR, Reverse Transcriptase PCR (RT-PCR), Real Time PCR, Single cell PCR, Solid phase PCR); ligation amplification (or ligase chain reaction (LCR)); amplification methods based on the use of Q-beta replicase or template-dependent polymerase; helicase-dependent isothermal amplification; strand displacement amplification (SDA); thermophilic SDA nucleic acid sequence based amplification (3SR or NASBA); transcription-associated amplification (TAA); the like and combinations thereof. A sequencing process that provides a sufficient depth of coverage for a diagnostic test generally is utilized, and sometimes the sequencing process provides about 0.1-fold to about 60-fold coverage (e.g., about 0.25-fold, 0.5-fold, 0.75 fold, 1-fold, 2-fold, 5-fold, 10-fold, 12-fold, 15-fold, 20-fold, 25-fold, 30-fold, 35-fold, 40-fold, 45-fold, 50-fold, 55-fold coverage) for a sample. A sequencing process can be performed using one or more sequencing runs (e.g., 1, 2, 3, 4 or 5 runs) for a sample.

A sequence read generally is a representation of a polynucleotide. For example, in a read containing an ATGC depiction of a sequence in a polynucleotide, "A" represents an adenine nucleotide, "T" represents a thymine nucleotide, "G" represents a guanine nucleotide and "C" represents a cytosine nucleotide. Sequence reads sometimes are paired-end reads and sometimes are single-end reads. A nominal, average, mean, median or absolute length of single-end reads sometimes is about 15 contiguous nucleotides to about 50 or more contiguous nucleotides, about 15 contiguous nucleotides to about 40 contiguous nucleotides, and sometimes about 15 contiguous nucleotides to about 36 contiguous nucleotides. A nominal, average, mean, median or absolute length of single-end reads sometimes is about 20 to about 30 bases, or about 24 to about 28 bases in length, and sometimes the nominal, average, mean or absolute length of single-end reads sometimes is about 1, 2, 3, 4, 5, 6, 7, 8, 9, 10, 11, 12, 13,14, 15, 16, 17, 18, 19, 21, 22, 23, 24, 25, 26, 27, 28 or about 29 bases or more in length. The nominal, average, mean or absolute length of the paired-end reads sometimes is about 10 contiguous nucleotides to about 25 contiguous nucleotides or more (e.g., about 10, 11, 12, 13, 14, 15, 16, 17, 18, 19, 20, 21, 22, 23, 24 or 25 nucleotides in length or more), about 15 contiguous nucleotides to about 20 contiguous nucleotides, and sometimes is about 17 contiguous nucleotides or about 18 contiguous nucleotides. Information for sequence reads can be included in one or more computer readable files having a suitable format, non-limiting examples of which are binary and/or text formats that include BAM, SAM, SRF, FASTQ, Gzip, the like, and combinations thereof.

A count A sometimes is determined by a process that does not include aligning the sequence reads to a reference genome, and a count B often is determined by a process that does not include aligning the sequence reads to a reference genome. A diagnostic test may include aligning sequence reads to a reference genome after a count B is determined, and/or sometimes after count A is determined. Processes suitable for aligning (e.g., mapping) sequence reads to a reference genome are known and include, without limitation, BLAST, BLITZ, FASTA, BOWTIE 1, BOWTIE 2, ELAND, MAQ, PROBEMATCH, SOAP or SEQMAP, DRAGEN, the like, or a variation or combination thereof. A reference genome can be obtained as known in the art, and can be obtained for example in GenBank, dbEST, dbSTS, EMBL (European Molecular Biology Laboratory) and DDBJ (DNA Databank of Japan) databases. Alignment of a sequence read to a reference genome can be a 100% sequence match. A sequence read alignment sometimes accommodates less than a 100% sequence match (i.e., non-perfect match, partial match, partial alignment) and sometime is about a 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76% or 75% match. Thus, a sequence read alignment sometimes accommodates a mismatch, and sometimes 1, 2, 3, 4 or 5 mismatches. An alignment process often includes or tracks information pertaining to a location of the reference genome at which a sequence read aligns (e.g., chromosome number to which a read aligns; chromosome position at which a read aligns), and such information can be stored in one or more computer readable files after an alignment is completed.

Sequence reads (e.g., aligned or non-aligned reads) can be counted by any suitable counting method known in the art. A count B sometimes is total reads generated by a nucleic acid sequencing process, or sometimes is a fraction of total reads generated by a nucleic acid sequencing process. As addressed herein, a count B sometimes is a count of the total reads or a fraction of the total reads, (i) less reads filtered according to a feature of the reads, or (ii) weighted according to a feature of the reads. A feature of the reads can be any suitable feature for filtering or weighting, non-limiting examples of which include read quality and read base content. Read base content sometimes is nucleotide base composition of a read and/or nucleotide base complexity of a read. Also as addressed herein, a count A and/or count B sometimes is a count of reads that match polynucleotides in a dictionary, and such a dictionary also is referred to herein as a listing or sub-listing of polynucleotides. A count A and/or count B may be a count of total reads or a fraction of total reads filtered according to a filter that removes reads aligned to one or more regions of a reference genome identified as having disproportionally low coverage or disproportionally high coverage of reads aligned thereto.

A count B may be (i) a count of total reads generated by a nucleic acid sequencing process used to sequence the nucleic acid from the test sample; (ii) a count of a fraction of total reads generated by a nucleic acid sequencing process used to sequence the nucleic acid from the test sample; (iii) a count of the total reads of (i) or the fraction of the total reads of (ii), less reads filtered according to a quality control metric for the sequencing process; (iv) a count of the total reads of (i) or the fraction of the total reads of (ii), weighted according to a quality control metric for the sequencing process; (v) a count of the total reads of (i) or the fraction of the total reads of (ii), less reads filtered according to read base content; (vi) a count of the total reads of (i) or the fraction of the total reads of (ii), weighted according to read base content; (vii) a count of reads that match polynucleotides in a listing, where the reads are determined to match or not match the polynucleotides in the listing in a process comprising comparing reads to the polynucleotides in the listing, where the reads are the total reads in (i), the fraction of total reads in (ii), the total reads of (i) or the fraction of the total reads of (ii) less the reads filtered according to the quality control metric of (iii), the total reads of (i) or the fraction of the total reads of (ii) weighted according to the quality control metric of (iv), the total reads of (i) or the fraction of the total reads of (ii) less the reads filtered according to the read base content of (v), or the total reads of (i) or the fraction of the total reads of (ii) weighted according to the read base content of (vi); (viii) the like, or (ix) combination of the foregoing (e.g., two or more of (i), (ii), (iii), (iv), (v), (vi) and (vii)).

A count A may be a count of reads that match polynucleotides in a listing or a subset of a listing, where the reads are determined to match or not match the polynucleotides in the listing or the subset of the listing in a process comprising comparing reads to the polynucleotides in the listing or the subset of the listing. The reads utilized for the comparison to the polynucleotides in the listing or the subset of the listing sometimes are reads are the total reads in (i), the fraction of total reads in (ii), the total reads of (i) or the fraction of the total reads of (ii) less the reads filtered according to the quality control metric of (iii), the total reads of (i) or the fraction of the total reads of (ii) weighted according to the quality control metric of (iv), the total reads of (i) or the fraction of the total reads of (ii) less the reads filtered according to the read base content of (v), or the total reads of (i) or the fraction of the total reads of (ii) weighted according to the read base content of (vi), where (i), (ii), (iii), (iv), (v) and (vi) are described in the foregoing paragraph.

A count A may be determined according to reads aligned to the target segment in a reference genome. The number of reads aligned to the target segment in the reference genome can be counted and the resulting total count for the segment can be utilized as count A. A fraction of the count of total reads also may be utilized, and sometimes total reads or a fraction of total reads is filtered or weighted as described herein for determining a count A. For example, coverage of reads aligned to regions in the target segment of the reference genome can be determined, and one or more regions covered by a disproportionally low or disproportionally high number of reads can be identified. Reads from such one or more regions may be filtered and removed from the total count of reads for the segment, for determining count A.

For aspects in which a count B is a count of total reads generated by a sequencing process, the total reads generally are not filtered (e.g., none of the reads are removed according to one or more criteria). In such aspects, total reads also generally are not weighted (e.g., none of the reads are multiplied by a weighting factor base on one or more criteria).

For aspects in which the count B is a count of a fraction of total reads generated by a sequencing process, the fraction often is a fraction of randomly selected reads from the total reads. The fraction in such aspects sometimes may be about 10% to about 90% of the total reads (e.g., about 15%, 20%, 25%, 30%, 35%, 40%, 45%, 50%, 55%, 60%, 65%, 70%, 75%, 80% or 85% of the total reads). Sometimes, about 50% to about 80% of the total reads are counted for a count $B$. For aspects in which a count $B$ is a fraction of the count of total reads generated by a sequencing process, the fraction of the total reads generally is not filtered and generally is not weighted.

For aspects in which a count $B$ is a count of the total reads or a fraction of the total reads, (i) less reads filtered according to a quality control metric for the sequencing process, or (ii) weighted according to a quality control metric for the sequencing process, the nucleic acid sequencing process that generates the sequence reads sometimes comprises image processing and the quality control metric is based on image quality. A non-limiting example of a MPS process that utilizes image processing to generate reads is an Illumina HiSeq/TruSeq process. Briefly, an image of solid-phase captured nucleic acid clusters is captured at each synthesis step of a sequencing-by-synthesis process. Image quality optionally can be assessed by a quality control metric according to whether the image generated by one cluster overlaps or does not overlap with the image of another cluster (e.g., metric used by a Chastity filter). Thus, a quality control metric sometimes may be based on an assessment of image overlap. The quality of the image, based on whether one cluster overlaps or does not overlap with another cluster, can be assessed using a score assigned by an image scoring module. A filter module may be utilized to filter out reads, from the total reads or fraction of total reads, attributed to clusters assigned a poor score. A weighting module may be utilized to multiply particular reads, or the count of particular reads, by their associated score assigned by the image scoring module, thereby weighting the reads, and the weighted reads or weighted read counts can be utilized for generating a segment count representation.

For aspects in which a count B is a count of the total reads or a fraction of the total reads, (i) less reads filtered according to read base content (e.g., base composition), or (ii) weighted according to the read base content, any suitable type of read base content can be utilized. Content of each of the four bases in DNA (A, T, C or G) or a combination thereof can be utilized for filtering or weighting by read base content. A read base content utilized for filtering or weighting sometimes is guanine and cytosine (GC) content. The amount of base content (e.g., GC content) can be assigned to each read by a base content module and the amount can be expressed in any suitable manner (e.g., percent GC content, GC score). Base content may be assessed by the number of base repeats or polynucleotide repeats in a read (e.g., a stretch of consecutive G bases in a read; three GCCG polynucleotide repeats in a read), and a repeat score or repeat value (e.g., % repeated element) can be assigned to each read by a repeat scoring module. A base content module and a repeat scoring module collectively are referred to as a base content module. A base content filter module may be utilized in to filter out reads, from the total reads or fraction of total reads, based on a base content assessment or score from a base content module. Reads may be filtered away from total reads or a fraction of total reads based on whether the reads (i) have a base content (e.g., GC content) less than a first base content threshold (e.g., a first threshold of about 40% GC content or less (e.g., a first threshold of about 30% GC content)) and/or (ii) have a base content (e.g., GC content) greater than a second base content threshold (e.g., a second threshold of about 60% GC content or more (e.g., a second threshold of about 70% GC content)). Reads may be filtered away from total reads or a fraction of total reads based on whether the reads have a repeat content (e.g., a base repeat content) greater than a repeat content threshold (e.g., threshold of about 50% repeats). A weighting module may be utilized to multiply particular reads, or the count of particular reads, by their associated score or value assigned by a repeat scoring module or base content module, thereby weighting the reads, and the weighted reads or weighted read counts can be utilized for generating a segment count representation.

For aspects in which reads are determined to match or not match polynucleotides in a listing or subset of the listing (i.e., a sub-listing), a count A and/or count B often is a count of reads that exactly match sequence and size of the polynucleotides in the listing or sub-listing. Polynucleotides often are selected for a listing or sub-listing based on an alignment of reads from a sample or samples (e.g., not the test sample) to a reference genome, or a subset in a reference genome, prior to comparing test sample reads to the polynucleotides and counting the matching test sample reads. Reads aligned in this prior alignment generally correspond to (e.g., are the same as) as the polynucleotides in the listing or sub-listing. Reads that align uniquely to a particular segment or region often are selected for inclusion as polynucleotides in a listing or sub-listing. For example, reads that align to a target segment (e.g., target chromosome) in the reference genome and do not align to other segments in the reference genome (e.g., do not align to other chromosomes) often are selected for inclusion as polynucleotides in a sub-listing.

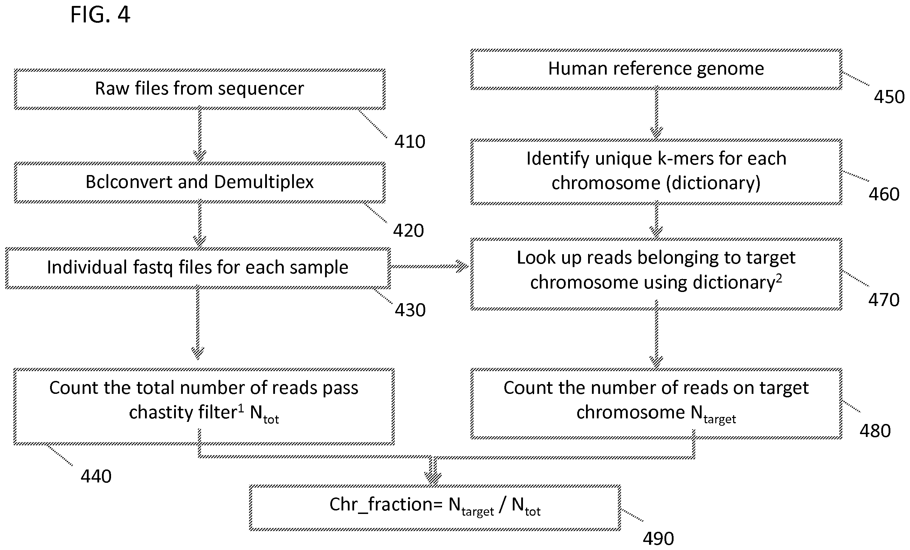

For determining a count $B$, a listing sometimes includes polynucleotides corresponding to reads that aligned in the prior alignment to all chromosomes, all autosomes, or a subset of all autosomes in the reference genome. For determining count A, a sub-listing often is utilized that includes polynucleotides corresponding to reads that aligned in the prior alignment to the target segment for which the count representation is determined (e.g., a target chromosome as the target segment) in the reference genome. A listing and sub-listing may be utilized, where the listing includes polynucleotides mapped to all autosomes, which can be utilized to determine count $B$, and where the sub-listing includes polynucleotides mapped to the segment, which can be utilized to determine count $A$. Thus, count $A$ and count $B$ may be determined, for generating a count representation for a target segment, without aligning reads from a test sample to a reference genome. A non-limiting example of utilizing a sub-listing of polynucleotides to generate a count representation for a particular target chromosome is illustrated in FIG. 4 and described in Example 2.

A process utilized to compare reads to polynucleotides in a listing or sub-listing (a comparison) generally is different than a process used to align reads to a reference genome (an alignment). For example, a process utilized for a comparison often does not track or record information pertaining to (i) a chromosome to which each read or polynucleotide aligns, and/or (ii) a chromosome position number at which each read or polynucleotide aligns. Also, a process utilized for a comparison often is binary, and for example, may assess whether the sequence and length of the read is, or is not, a 100% match to a polynucleotide in the listing and/or sub-listing. A binary process generally is less complex than a process for aligning reads to a reference genome as an alignment process often utilizes higher complexity algorithms.

Reads generated from test sample nucleic acid (i) sometimes are not subjected to an alignment process that aligns the sequence reads to the reference genome prior to generating count $A$ and/or count $B$; (ii) sometimes are not subjected to an alignment process that aligns the sequence reads to the reference genome in a diagnostic test being performed; or (iii) sometimes are subjected to an alignment process that aligns reads with a reference genome, where count $A$ and/or count $B$ is/are determined prior to subjecting the reads to the alignment process. Reads generated for the test sample nucleic acid may be subjected to an alignment process that aligns reads with a reference genome, the count $A$ is a count of reads aligned to the segment in the reference genome, and the count $B$ is a count of reads not aligned, or determined prior to alignment of reads, to the reference genome. The count $A$ and/or the count $B$ may be determined by a process that does not include aligning the sequence reads to a reference genome.

Reads generated from a test sample may be subjected to an alignment process that aligns reads with a reference genome, and the count $B$ is a count of reads not aligned to the reference genome by the alignment process. Reads that cannot be aligned to a reference genome (unalignable reads) sometimes are reads that contain a repeated polynucleotide and/or originate from centromeres.

A target segment, for which a count representation is determined, may be a chromosome, and the chromosome sometimes is chromosome 13, chromosome 18 and chromosome 21. The segment sometimes is a segment of a chromosome, and sometimes is a microduplication or microdeletion region.

A count $A$ may be normalized counts and/or a count $B$ may be normalized counts. Any suitable normalization process or suitable combination of normalization processes can be used to generate normalized counts. Non-limiting examples of normalization processes include portion-wise normalization (e.g., bin-wise normalization), normalization by GC content, linear and nonlinear least squares regression, LOESS, GC-LOESS, LOWESS, PERUN, ChAI, RM, GCRM, cQn, the like and combinations thereof. Normalized counts sometimes are generated by (i) a normalization process comprising a LOESS normalization process, (ii) a normalization process comprising a guanine and cytosine (GC) bias normalization, (iii) a normalization process comprising LOESS normalization of GC bias (GC-LOESS), (iv) a normalization process comprising principal component normalization (e.g., ChAI normalization process), the like and combinations of the foregoing. A normalization process may includes a GC-LOESS normalization followed by a principal component normalization. Specific aspects of certain normalization processes (e.g., ChAI normalization, principal component normalization, PERUN normalization) are described, for example, in patent application no. PCT/US2014/039389 filed on May 23, 2014 and published as WO 2014/190286; and patent application no. PCT/US2014/058885 filed on October 2, 2014 and published as WO 2015/051163 on April 9, 2015.

A normalization process that includes a principal component normalization may include: (a) providing a read density profile, which can be generated by filtering, according to a read density distribution prepared for multiple samples, and (b) adjusting the read density profile for the test sample according to one or more principal components, which principal components are obtained from a set of reference samples, by a principal component analysis, thereby providing a test sample profile comprising adjusted read densities.

A normalization process that includes a PERUN normalization may include: (1) determining a guanine and cytosine (GC) bias coefficient for a test sample based on a fitted relation between (i) the counts of the sequence reads mapped to each of the portions and (ii) GC content for each of the portions, where the GC bias coefficient is a slope for a linear fitted relation or a curvature estimation for a non-linear fitted relation; and (2) calculating, using a microprocessor, a genomic section level for each of the portions based on the counts of (a), the GC bias coefficient of (b) and a fitted relation, for each of the portions, between (i) the GC bias coefficient for each of multiple samples and (ii) the counts of the sequence reads mapped to each of the portions for the multiple samples, thereby providing calculated genomic section levels

A diagnostic method may include determining a statistic of a count representation for a segment, and/or may include determining a statistic using a count representation for a segment. Any suitable statistic can be generated, non-limiting examples of which include mean, median, mode, average, p-value, a measure of deviation (e.g., standard deviation (SD), sigma, absolute deviation, mean absolute deviation (MAD), calculated variance, and the like), a suitable measure of error (e.g., standard error, mean squared error, root mean squared error, and the like), a suitable measure of variance, a suitable standard score (e.g., standard deviation, cumulative percentage, percentile equivalent, Z-score, T-score, R-score, standard nine (stanine), percent in stanine, and the like), or combination thereof. Any suitable statistical method may be used to generate a statistic of a count representation or generate a statistic using a count representation, non-limiting examples of which include exact test, F-test, Z-test, T-test, calculating and/or comparing a measure of uncertainty, a null hypothesis, counternulls and the like, a chisquare test, omnibus test, calculating and/or comparing level of significance (e.g., statistical significance), a meta analysis, a multivariate analysis, a regression, simple linear regression, robust linear regression least squares regression, principle component analysis, linear discriminant analysis, quadratic discriminant analysis, bagging, neural networks, support vector machine models, random forests, classification tree models, K-nearest neighbors, logistic regression, loss smoothing, Behrens-Fisher approach, bootstrapping, Fisher's method for combining independent tests of significance, Neyman-Pearson testing, confirmatory data analysis, exploratory data analysis, the like or combination thereof.

A z-score sometimes is generated as a statistic, which sometimes is a quotient of (a) a subtraction product of (i) the count representation for the segment for the test sample, less (ii) a median of a count representation for the segment for a sample set, divided by (b) a MAD of the count representation for the segment for the sample set. A diagnostic test sometimes may be a prenatal genetic diagnostic test, a test sample is from a pregnant female bearing a fetus, and a sample set is a set of samples for subjects having euploid fetus pregnancies. A diagnostic test may be a prenatal diagnostic test, a test sample is from a pregnant female bearing a fetus, and a sample set is a set of samples for subjects having trisomy fetus pregnancies. A diagnostic test may be a genetic test for presence, absence, increased risk, or decreased risk of a cell proliferative condition, and a sample set is a set of samples for subjects having the cell proliferative condition. A diagnostic test may be for presence, absence, increased risk, or decreased risk of a cell proliferative condition, and a sample set is a set of samples for subjects not having the cell proliferative condition.

A diagnostic test may be a genetic prenatal diagnostic test, a test sample is from a pregnant female bearing a fetus, and the diagnostic test includes determining presence of absence of a genetic variation (e.g., a fetal genetic variation). A genetic variation sometimes is a chromosome aneuploidy, and sometimes a chromosome aneuploidy is one (monosomy), three (trisomy) or four copies of a whole chromosome. A genetic variation in certain prenatal diagnostic testssometimes may be a microduplication or microdeletion.

A diagnostic test may be a genetic diagnostic test for presence, absence, increased risk, or decreased risk of a cell proliferative condition, and the diagnostic test includes determining presence of absence of a genetic variation. A genetic variation in some cancer diagnostic tests sometimes may a microduplication or microdeletion.

Determining presence or absence of a genetic variation (determining an outcome) using a segment count representation, or statistic derived therefrom, can be performed in any suitable manner. Any suitable statistic can be utilized for determining an outcome, non-limiting examples of which include standard deviation, average absolute deviation, median absolute deviation, maximum absolute deviation, standard score (e.g., z-value, z-score, normal score, standardized variable) the like and combinations thereof. An outcome may be determined when the number of deviations between two statistics (e.g., one for test sample (e.g., test counts) and another for reference samples (e.g., reference counts)) is greater than about 1, greater than about 1.5, greater than about 2, greater than about 2.5, greater than about 2.6, greater than about 2.7, greater than about 2.8, greater than about 2.9, greater than about 3, greater than about 3.1, greater than about 3.2, greater than about 3.3, greater than about 3.4, greater than about 3.5, greater than about 4, greater than about 5, or greater than about 6. Determining an outcome sometimes is performed by comparing a statistic derived from the count representation (e.g., z-score) to a predetermined threshold value for the statistic (e.g., z-score threshold; z-score threshold of about 3.95).

Determining an outcome sometimes is performed using a decision analysis. Non-limiting examples of decision analyses are described in patent application no. PCT/US2014/039389 filed on May 23, 2014 and published as WO 2014/190286. A decision analysis may include (a) providing a count representation for a test segment (e.g., test chromosome) for a test sample as described herein; (b) determining fetal fraction for the test sample; (c) calculating a log odds ratio (LOR), which LOR is the log of the quotient of (i) a first multiplication product of (1) a conditional probability of having a genetic variation and (2) a prior probability of having the genetic variation, and (ii) a second multiplication product of (1) a conditional probability of not having the genetic variation and (2) a prior probability of not having the genetic variation, where: the conditional probability of having the genetic variation is determined according to the fetal fraction of (b) and the count representation of (a); and (d) identifying an outcome (e.g., presence or absence of the genetic variation) according to the LOR and the count representation. A count representation sometimes is a normalized count representation, and a genetic variation may be a chromosome aneuploidy, microduplication or microdeletion. The conditional probability of having the genetic variation sometimes is (i) determined according to fetal fraction determined for the test sample in (b), a z-score for the count representation for the test sample in (a), and a fetal fraction-specific distribution of z-scores for the count representation; (ii) determined by the relationship in equation 23:

$$Z \sim Normal\left(\frac{\mu_X}{\sigma_X}\frac{f}{2}, 1\right) \qquad (23)$$

where $f$ is fetal fraction, X is the summed portions for the chromosome, X - f($\mu$X,$\sigma$X), where $\mu$X and $\sigma$X are the mean and standard deviation of X, respectively, and f($\cdot$) is a distribution function; and/or (iii) is the intersection between the z-score for the test sample count representation of (a) and a fetal fraction-specific distribution of z-scores for the count representation. The conditional probability of not having the genetic variation sometimes is (i) determined according to the count representation of (a) and count representations for euploids; and/or (ii) is the intersection between the z-score of the count representation and a distribution of z-scores for the count representation in subjects not having the genetic variation. The prior probability of having the genetic variation and the prior probability of not having the genetic variation sometimes are determined from multiple samples that do not include the test subject. A decision analysis sometimes includes (1) determining whether the LOR is greater than or less than zero; (2) determining a z-score quantification of the count representation of (a) and determining whether it is less than, greater than or equal to a value of 3.95; (3) determining the presence of a genetic variation if, for the test sample, (i) the z-score quantification of the count representation is greater than or equal to the value of 3.95, and (ii) the LOR is greater than zero; and/or (4) determining the absence of a genetic variation if, for the test sample, (i) the z-score quantification of the count representation is less than the value of 3.95, and/or (ii) the LOR is less than zero.

Fetal fraction can be expressed in any suitable manner (e.g., ratio of an amount of fetal nucleic acid to total nucleic acid amount or amount of maternal nucleic acid in a test sample), and can be determined using any suitable method known in the art. An amount of fetal nucleic acid may be determined according to markers specific to a male fetus (e.g., Y-chromosome STR markers (e.g., DYS 19, DYS 385, DYS 392 markers); RhD marker in RhD-negative females), allelic ratios of polymorphic sequences, or according to one or more markers specific to fetal nucleic acid and not maternal nucleic acid (e.g., differential epigenetic biomarkers (e.g., methylation) between mother and fetus, or fetal RNA markers in maternal blood plasma.

Fetal fraction may be determined using methods that incorporate fragment length information (e.g., fragment length ratio (FLR) analysis, fetal ratio statistic (FRS) analysis as described in International Application Publication No. WO2013/177086). Cell-free fetal nucleic acid fragments generally are shorter than maternally-derived nucleic acid fragments and fetal fraction may be determined, by counting fragments under a particular length threshold and comparing the counts, for example, to counts from fragments over a particular length threshold and/or to the amount of total nucleic acid in the sample. Methods for counting nucleic acid fragments of a particular length are described in further detail in International Application Publication No. WO2013/177086.

Fetal fraction may be determined using an assay that discriminates fetal nucleic acid according to methylation status (see, e.g., fetal quantifier assay (FQA); U.S. Patent Application Publication No. 2010/0105049). In certain assays, a concentration of fetal DNA in a maternal test sample may be determined by the following method: (a) determine the total amount of DNA present in a maternal test sample; (b) selectively digest the maternal DNA in a maternal sample using one or more methylation sensitive restriction enzymes thereby enriching the fetal DNA; (c) determine the amount of fetal DNA from (b); and (d) compare the amount of fetal DNA from step c) to the total amount of DNA from (a), thereby determining the concentration of fetal DNA in the maternal sample. The absolute copy number of fetal nucleic acid in a maternal test sample can be determined, for example, using mass spectrometry and/or a system that uses a competitive PCR approach for absolute copy number measurements.

A genetic test sometimes is performed in whole or in part within a system. Some or all steps for determining a count representation sometimes are performed by (i) a microprocessor in a system, (ii) in conjunction with memory in a system, and/or (iii) by a computer.

*Samples*

Provided herein are systems, methods and products for analyzing nucleic acids. Nucleic acid fragments in a mixture of nucleic acid fragments may be analyzed. A mixture of nucleic acids can comprise two or more nucleic acid fragment species having different nucleotide sequences, different fragment lengths, different origins (e.g., genomic origins, fetal vs. maternal origins, cell or tissue origins, cancer vs. non-cancer origin, tumor vs. non-tumor origin, sample origins, subject origins, and the like), or combinations thereof.

Nucleic acid or a nucleic acid mixture utilized in systems, methods and products described herein often is isolated from a sample obtained from a subject. A subject can be any living or non-living organism, including but not limited to a human, a non-human animal, a plant, a bacterium, a fungus or a protist. Any human or non-human animal can be selected, including but not limited to mammal, reptile, avian, amphibian, fish, ungulate, ruminant, bovine (e.g.,

cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female (e.g., woman, a pregnant woman). A subject may be any age (e.g., an embryo, a fetus, infant, child, adult).

Nucleic acid may be isolated from any type of suitable biological specimen or sample (e.g., a test sample). A sample or test sample can be any specimen that is isolated or obtained from a subject or part thereof (e.g., a human subject, a pregnant female, a fetus). Non-limiting examples of specimens include fluid or tissue from a subject, including, without limitation, blood or a blood product (e.g., serum, plasma, or the like), umbilical cord blood, chorionic villi, amniotic fluid, cerebrospinal fluid, spinal fluid, lavage fluid (e.g., bronchoalveolar, gastric, peritoneal, ductal, ear, arthroscopic), biopsy sample (e.g., from pre-implantation embryo; cancer biopsy), celocentesis sample, cells (blood cells, placental cells, embryo or fetal cells, fetal nucleated cells or fetal cellular remnants) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, the like or combinations thereof. A biological sample may be a cervical swab from a subject. A biological sample may be blood and sometimes plasma or serum. The term "blood" as used herein refers to a blood sample or preparation from a pregnant woman or a woman being tested for possible pregnancy. The term encompasses whole blood, blood product or any fraction of blood, such as serum, plasma, buffy coat, or the like as conventionally defined. Blood or fractions thereof often comprise nucleosomes (e.g., maternal and/or fetal nucleosomes). Nucleosomes comprise nucleic acids and are sometimes cell-free or intracellular. Blood also comprises buffy coats. Buffy coats are sometimes isolated by utilizing a ficoll gradient. Buffy coats can comprise white blood cells (e.g., leukocytes, T-cells, B-cells, platelets, and the like). Buffy coats may comprise maternal and/or fetal nucleic acid. Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow. For blood, an appropriate amount of peripheral blood (e.g., between 3-40 milliliters) often is collected and can be stored according to standard procedures prior to or after preparation. A fluid or tissue sample from which nucleic acid is extracted may be acellular (e.g., cell-free). A fluid or tissue sample may contain cellular elements or cellular remnants. Fetal cells or cancer cells may be included in the sample.

A sample can be a liquid sample. A liquid sample can comprise extracellular nucleic acid (e.g., circulating cell-free DNA). Non-limiting examples of liquid samples, include, blood or a blood product (e.g., serum, plasma, or the like), umbilical cord blood, amniotic fluid, cerebrospinal fluid, spinal fluid, lavage fluid (e.g., bronchoalveolar, gastric, peritoneal, ductal, ear, arthroscopic), biopsy sample (e.g., liquid biopsy for the detection of cancer), celocentesis sample, washings of female reproductive tract, urine, sputum, saliva, nasal mucous, prostate fluid, lavage, semen, lymphatic fluid, bile, tears, sweat, breast milk, breast fluid, the like or combinations thereof. A sample may be a liquid biopsy, which generally refers to an assessment of a liquid sample from a subject for the presence, absence, progression or remission of a disease (e.g., cancer). A liquid biopsy can be used in conjunction with, or as an alternative to, a sold biopsy (e.g., tumor biopsy). In certain instances, extracellular nucleic acid is analyzed in a liquid biopsy.

A sample often is heterogeneous, by which is meant that more than one type of nucleic acid species is present in the sample. For example, heterogeneous nucleic acid can include, but is not limited to, (i) cancer and non-cancer nucleic acid, (ii) pathogen and host nucleic acid, (iii) fetal derived and maternal derived nucleic acid, and/or more generally, (iv) mutated and wild-type nucleic acid. A sample may be heterogeneous because more than one cell type is present, such as a fetal cell and a maternal cell, a cancer and non-cancer cell, or a pathogenic and host cell. A minority nucleic acid species and a majority nucleic acid species may be present.

For prenatal applications of technology described herein, fluid or tissue sample may be collected from a female at a gestational age suitable for testing, or from a female who is being tested for possible pregnancy. Suitable gestational age may vary depending on the prenatal test being performed. A pregnant female subject sometimes may be in the first trimester of pregnancy, at times in the second trimester of pregnancy, or sometimes in the third trimester of pregnancy. A fluid or tissue may be collected from a pregnant female between about 1 to about 45 weeks of fetal gestation (e.g., at 1-4, 4-8, 8-12, 12-16, 16-20, 20-24, 24-28, 28-32, 32-36, 36-40 or 40-44 weeks of fetal gestation), and sometimes between about 5 to about 28 weeks of fetal gestation (e.g., at 6, 7, 8, 9, 10, 11, 12, 13, 14, 15, 16, 17, 18, 19, 20, 21, 22, 23, 24, 25, 26 or 27 weeks of fetal gestation). A fluid or tissue sample may be collected from a pregnant female during or just after (e.g., 0 to 72 hours after) giving birth (e.g., vaginal or non-vaginal birth (e.g., surgical delivery)).

*Acquisition of Blood Samples and Extraction of DNA*

Methods described herein may comprise separating, enriching, sequencing and/or analyzing DNA found in the blood of a subject as a non-invasive means to detect the presence or absence of a chromosome alteration in a subject's genome and/or to monitor the health of a subject.

*Acquisition of Blood Samples*

A blood sample can be obtained from a subject (e.g., a male or female subject) of any age using a method of the present technology. A blood sample can be obtained from a pregnant woman at a gestational age suitable for testing using a method of the present technology. A suitable gestational age may vary depending on the disorder tested, as discussed below. Collection of blood from a subject (e.g., a pregnant woman) often is performed in accordance with the standard protocol hospitals or clinics generally follow. An appropriate amount of peripheral blood, e.g., typically between 5-50 ml, often is collected and may be stored according to standard procedure prior to further preparation. Blood samples may be collected, stored or transported in a manner that minimizes degradation or the quality of nucleic acid present in the sample.

*Preparation of Blood Samples*

An analysis of DNA found in a subjects blood may be performed using, e.g., whole blood, serum, or plasma. An analysis of fetal DNA found in maternal blood may be performed using, e.g., whole blood, serum, or plasma. An analysis of tumor DNA found in a patient's blood may be performed using, e.g., whole blood, serum, or plasma. Methods for preparing serum or plasma from blood obtained from a subject (e.g., a maternal subject; cancer patient) are known. For example, a subject's blood (e.g., a pregnant woman's blood; cancer patient's blood) can be placed in a tube containing EDTA or a specialized commercial product such as Vacutainer SST (Becton Dickinson, Franklin Lakes, N.J.) to prevent blood clotting, and plasma can then be obtained from whole blood through centrifugation. Serum may be obtained with or without centrifugation-following blood clotting. If centrifugation is used then it is typically, though not exclusively, conducted at an appropriate speed, e.g., 1,500-3,000 times g. Plasma or serum may be subjected to additional centrifugation steps before being transferred to a fresh tube for DNA extraction.

In addition to the acellular portion of the whole blood, DNA may also be recovered from the cellular fraction, enriched in the buffy coat portion, which can be obtained following centrifugation of a whole blood sample from the woman or patient and removal of the plasma.

*Extraction of DNA*

There are numerous known methods for extracting DNA from a biological sample including blood. The general methods of DNA preparation (e.g., described by Sambrook and Russell, Molecular Cloning: A Laboratory Manual 3d ed., 2001) can be followed; various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep™ Blood DNA Isolation Kit (Promega, Madison, Wis.), and GFX™ Genomic Blood DNA Purification Kit (Amersham, Piscataway, N.J.), may also be used to obtain DNA from a blood sample from a subject. Combinations of more than one of these methods may also be used.

A sample obtained from a subject may first be enriched or relatively enriched for tumor nucleic acid by one or more methods. For example, the discrimination of tumor and normal patient DNA can be performed using the compositions and processes of the present technology alone or in combination with other discriminating factors.

A sample obtained from a pregnant female subject may first be enriched or relatively enriched for fetal nucleic acid by one or more methods. For example, the discrimination of fetal and maternal DNA can be performed using the compositions and processes of the present technology alone or in combination with other discriminating factors. Examples of these factors include, but are not limited to, single nucleotide differences between chromosome X and Y, chromosome Y-specific sequences, polymorphisms located elsewhere in the genome, size differences between fetal and maternal DNA and differences in methylation pattern between maternal and fetal tissues.

Other methods for enriching a sample for a particular species of nucleic acid are described in PCT Patent Application Number PCT/US07/69991, filed May 30, 2007, PCT Patent Application Number PCT/US2007/071232, filed June 15, 2007, US Provisional Application Numbers 60/968,876 and 60/968,878 (assigned to the Applicant), (PCT Patent Application Number PCT/EP05/012707, filed November 28, 2005). Maternal nucleic acid may be selectively removed (either partially, substantially, almost completely or completely) from the sample.

The terms "nucleic acid" and "nucleic acid molecule" may be used interchangeably throughout the disclosure. The terms refer to nucleic acids of any composition from, such as DNA (e.g., complementary DNA (cDNA), genomic DNA (gDNA) and the like), RNA (e.g., message RNA (mRNA), short inhibitory RNA (siRNA), ribosomal RNA (rRNA), tRNA, microRNA, RNA highly expressed by the fetus or placenta, and the like), and/or DNA or RNA analogs (e.g., containing base analogs, sugar analogs and/or a non-native backbone and the like), RNA/DNA hybrids and polyamide nucleic acids (PNAs), all of which can be in single- or double-stranded form, and unless otherwise limited, can encompass known analogs of natural nucleotides that can function in a similar manner as naturally occurring nucleotides. A nucleic acid may be, or may be from, a plasmid, phage, virus, autonomously replicating sequence (ARS), centromere, artificial chromosome, chromosome, or other nucleic acid able to replicate or be replicated in vitro or in a host cell, a cell, a cell nucleus or cytoplasm of a cell. A template nucleic acid may be from a single chromosome (e.g., a nucleic acid sample may be from one chromosome of a sample obtained from a diploid organism). Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with locus, gene, cDNA, and mRNA encoded by a gene. The term also may include, as equivalents, derivatives, variants and analogs of RNA or DNA synthesized from nucleotide analogs, single-stranded ("sense" or "antisense", "plus" strand or "minus" strand, "forward" reading frame or "reverse" reading frame) and double-stranded polynucleotides. The term "gene" means the segment of DNA involved in producing a polypeptide chain; it includes regions preceding and following the coding region (leader and trailer) involved in the transcription/translation of the gene product and the regulation of the transcription/translation, as well as intervening sequences (introns) between individual coding segments (exons). Deoxyribonucleotides include deoxyadenosine, deoxycytidine, deoxyguanosine and deoxythymidine. For RNA, the base cytosine is replaced with uracil. A template nucleic acid may be prepared using a nucleic acid obtained from a subject as a template.

*Nucleic Acid Isolation and Processing*

Nucleic acid may be derived from one or more sources (e.g., cells, serum, plasma, buffy coat, lymphatic fluid, skin, soil, and the like) by methods known in the art. Any suitable method can be used for isolating, extracting and/or purifying DNA from a biological sample (e.g., from blood or a blood product), non-limiting examples of which include methods of DNA preparation (e.g., described by Sambrook and Russell, Molecular Cloning: A Laboratory Manual 3d ed., 2001), various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep™ Blood DNA Isolation Kit (Promega, Madison, Wis.), and GFX™ Genomic Blood DNA Purification Kit (Amersham, Piscataway, N.J.), the like or combinations thereof.

Cell lysis procedures and reagents are known in the art and may generally be performed by chemical (e.g., detergent, hypotonic solutions, enzymatic procedures, and the like, or combination thereof), physical (e.g., French press, sonication, and the like), or electrolytic lysis methods. Any suitable lysis procedure can be utilized. For example, chemical methods generally employ lysing agents to disrupt cells and extract the nucleic acids from the cells, followed by treatment with chaotropic salts. Physical methods such as freeze/thaw followed by grinding, the use of cell presses and the like also are useful. High salt lysis procedures also are commonly used. For example, an alkaline lysis procedure may be utilized. The latter procedure traditionally incorporates the use of phenol-chloroform solutions, and an alternative phenol-chloroform-free procedure involving three solutions can be utilized. In the latter procedures, one solution can contain 15mM Tris, pH 8.0; 10mM EDTA and 100 $\mu$g/ml Rnase A; a second solution can contain 0.2N NaOH and 1% SDS; and a third solution can contain 3M KOAc, pH 5.5. These procedures can be found in Current Protocols in Molecular Biology, John Wiley & Sons, N.Y., 6.3.1-6.3.6 (1989).

Nucleic acid may be isolated at a different time point as compared to another nucleic acid, where each of the samples is from the same or a different source. A nucleic acid may be from a nucleic acid library, such as a cDNA or RNA library, for example. A nucleic acid may be a result of nucleic acid purification or isolation and/or amplification of nucleic acid molecules from the sample. Nucleic acid provided for processes described herein may contain nucleic acid from one sample or from two or more samples (e.g., from 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more samples).

Nucleic acids may include extracellular nucleic acid. The term "extracellular nucleic acid" as used herein can refer to nucleic acid isolated from a source having substantially no cells and also is referred to as "cell-free" nucleic acid, "circulating cell-free nucleic acid" (e.g., CCF fragments, ccf DNA) and/or "cell-free circulating nucleic acid". Extracellular nucleic acid can be present in and obtained from blood (e.g., from the blood of a human, e.g., from the blood of a pregnant female). Extracellular nucleic acid often includes no detectable cells and may contain cellular elements or cellular remnants. Non-limiting examples of acellular sources for extracellular nucleic acid are blood, blood plasma, blood serum and urine. As used herein, the term "obtain cell-free circulating sample nucleic acid" includes obtaining a sample directly (e.g., collecting a sample, e.g., a test sample) or obtaining a sample from another who has collected a sample. Without being limited by theory, extracellular nucleic acid may be a product of cell apoptosis and cell breakdown, which provides basis for extracellular nucleic acid often having a series of lengths across a spectrum (e.g., a "ladder").

Extracellular nucleic acid may include different nucleic acid species, and therefore is referred to herein as "heterogeneous". For example, blood serum or plasma from a person having cancer can include nucleic acid from cancer cells (e.g., tumor, neoplasia) and nucleic acid from non-cancer cells. In another example, blood serum or plasma from a pregnant female can include maternal nucleic acid and fetal nucleic acid. In some instances, cancer or fetal nucleic acid sometimes is about 5% to about 50% of the overall nucleic acid (e.g., about 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49% of the total nucleic acid is cancer or fetal nucleic acid). The majority of cancer or fetal nucleic acid in nucleic acid may be of a length of about 500 base pairs or less (e.g., about 80, 85, 90, 91, 92, 93, 94, 95, 96, 97, 98, 99 or 100% of cancer or fetal nucleic acid is of a length of about 500 base pairs or less). Tthe majority of cancer or fetal nucleic acid in nucleic acid may be of a length of about 250 base pairs or less (e.g., about 80, 85, 90, 91, 92, 93, 94, 95, 96, 97, 98, 99 or 100% of cancer or fetal nucleic acid is of a length of about 250 base pairs or less). The majority of cancer or fetal nucleic acid in nucleic acid may be of a length of about 200 base pairs or less (e.g., about 80, 85, 90, 91, 92, 93, 94, 95, 96, 97, 98, 99 or 100% of cancer or fetal nucleic acid is of a length of about 200 base pairs or less). The majority of cancer or fetal nucleic acid in nucleic acid may be of a length of about 150 base pairs or less (e.g., about 80, 85, 90, 91, 92, 93, 94, 95, 96, 97, 98, 99 or 100% of cancer or fetal nucleic acid is of a length of about 150 base pairs or less). The majority of cancer or fetal nucleic acid in nucleic acid may be of a length of about 100 base pairs or less (e.g., about 80, 85, 90, 91, 92, 93, 94, 95, 96, 97, 98, 99 or 100% of cancer or fetal nucleic acid is of a length of about 100 base pairs or less). The majority of cancer or fetal nucleic acid in nucleic acid is of a length of about 50 base pairs or less (e.g., about 80, 85, 90, 91, 92, 93, 94, 95, 96, 97, 98, 99 or 100% of cancer or fetal nucleic acid is of a length of about 50 base pairs or less). The majority of cancer or fetal nucleic acid in nucleic acid may be of a length of about 25 base pairs or less (e.g., about 80, 85, 90, 91, 92, 93, 94, 95, 96, 97, 98, 99 or 100% of cancer or fetal nucleic acid is of a length of about 25 base pairs or less).

Nucleic acid may be provided for conducting methods described herein without processing of the sample(s) containing the nucleic acid. Nucleic acid may be provided for conducting methods described herein after processing of the sample(s) containing the nucleic acid. For example, a nucleic acid can be extracted, isolated, purified, partially purified or amplified from the sample(s). The term "isolated" as used herein refers to nucleic acid removed from its original environment (e.g., the natural environment if it is naturally occurring, or a host cell if expressed exogenously), and thus is altered by human intervention (e.g., "by the hand of man") from its original environment. The term "isolated nucleic acid" as used herein can refer to a nucleic acid removed from a subject (e.g., a human subject). An isolated nucleic acid can be provided with fewer non-nucleic acid components (e.g., protein, lipid) than the amount of components present in a source sample. A composition comprising isolated nucleic acid can be about 50% to greater than 99% free of non-nucleic acid components. A composition comprising isolated nucleic acid can be about 90%, 91%, 92%, 93%, 94%, 95%, 96%, 97%, 98%, 99% or greater than 99% free of non-nucleic acid components. The term "purified" as used herein can refer to a nucleic acid provided that contains fewer non-nucleic acid components (e.g., protein, lipid, carbohydrate) than the amount of non-nucleic acid components present prior to subjecting the nucleic acid to a purification procedure. A composition comprising purified nucleic acid may be about 80%, 81%, 82%, 83%, 84%, 85%, 86%, 87%, 88%, 89%, 90%, 91%, 92%, 93%, 94%, 95%, 96%, 97%, 98%, 99% or greater than 99% free of other non-nucleic acid components. The term "purified" as used herein can refer to a nucleic acid provided that contains fewer nucleic acid species than in the sample source from which the nucleic acid is derived. A composition comprising purified nucleic acid may be about 90%, 91%, 92%, 93%, 94%, 95%, 96%, 97%, 98%, 99% or greater than 99% free of other nucleic acid species. For example, fetal nucleic acid can be purified from a mixture comprising maternal and fetal nucleic acid. In certain examples, small fragments of fetal nucleic acid (e.g., 30 to 500 bp fragments) can be purified, or partially purified, from a mixture comprising both fetal and maternal nucleic acid fragments. In certain examples, nucleosomes comprising smaller fragments of fetal nucleic acid can be purified from a mixture of larger nucleosome complexes comprising larger fragments of maternal nucleic acid. In certain examples, cancer cell nucleic acid can be purified from a mixture comprising cancer cell and non-cancer cell nucleic acid. In certain examples, nucleosomes comprising small fragments of cancer cell nucleic acid can be purified from a mixture of larger nucleosome complexes comprising larger fragments of non-cancer nucleic acid.

Nucleic acids may be sheared or cleaved prior to, during or after a method described herein. Sheared or cleaved nucleic acids may have a nominal, average or mean length of about 5 to about 10,000 base pairs, about 100 to about 1,000 base pairs, about 100 to about 500 base pairs, or about 10, 15, 20, 25, 30, 35, 40, 45, 50, 55, 60, 65, 70, 75, 80, 85, 90, 95, 100, 200, 300, 400, 500, 600, 700, 800, 900, 1000, 2000, 3000, 4000, 5000, 6000, 7000, 8000 or 9000 base pairs. Sheared or cleaved nucleic acids can be generated by a suitable method known in the art, and the average, mean or nominal length of the resulting nucleic acid fragments can be controlled by selecting an appropriate fragment-generating method.

Nucleic acid may be sheared or cleaved by a suitable method, non-limiting examples of which include physical methods (e.g., shearing, e.g., sonication, French press, heat, UV irradiation, the like), enzymatic processes (e.g., enzymatic cleavage agents (e.g., a suitable nuclease, a suitable restriction enzyme, a suitable methylation sensitive restriction enzyme)), chemical methods (e.g., alkylation, DMS, piperidine, acid hydrolysis, base hydrolysis, heat, the like, or combinations thereof), processes described in U.S. Patent Application Publication No. 20050112590, the like or combinations thereof.

As used herein, "shearing" or "cleavage" refers to a procedure or conditions in which a nucleic acid molecule, such as a nucleic acid template gene molecule or amplified product thereof, may be severed into two or more smaller nucleic acid molecules. Such shearing or cleavage can be sequence specific, base specific, or nonspecific, and can be accomplished by any of a variety of methods, reagents or conditions, including, for example, chemical, enzymatic, physical shearing (e.g., physical fragmentation). As used herein, "cleavage products", "cleaved products" or grammatical variants thereof, refers to nucleic acid molecules resultant from a shearing or cleavage of nucleic acids or amplified products thereof.

The term "amplified" as used herein refers to subjecting a target nucleic acid in a sample to a process that linearly or exponentially generates amplicon nucleic acids having the same or substantially the same nucleotide sequence as the target nucleic acid, or segment thereof. The term "amplified" may refer to a method that comprises a polymerase chain reaction (PCR). For example, an amplified product can contain one or more nucleotides more than the amplified nucleotide region of a nucleic acid template sequence (e.g., a primer can contain "extra" nucleotides such as a transcriptional initiation sequence, in addition to nucleotides complementary to a nucleic acid template gene molecule, resulting in an amplified product containing "extra" nucleotides or nucleotides not corresponding to the amplified nucleotide region of the nucleic acid template gene molecule).

As used herein, the term "complementary cleavage reactions" refers to cleavage reactions that are carried out on the same nucleic acid using different cleavage reagents or by altering the cleavage specificity of the same cleavage reagent such that alternate cleavage patterns of the same target or reference nucleic acid or protein are generated. Nucleic acid may be treated with one or more specific cleavage agents (e.g., 1, 2, 3, 4, 5, 6, 7, 8, 9, 10 or more specific cleavage agents) in one or more reaction vessels (e.g., nucleic acid is treated with each specific cleavage agent in a separate vessel). The term "specific cleavage agent" as used herein refers to an agent, sometimes a chemical or an enzyme that can cleave a nucleic acid at one or more specific sites.

Nucleic acid also may be exposed to a process that modifies certain nucleotides in the nucleic acid before providing nucleic acid for a method described herein. A process that selectively modifies nucleic acid based upon the methylation state of nucleotides therein can be applied to nucleic acid, for example. In addition, conditions such as high temperature, ultraviolet radiation, x-radiation, can induce changes in the sequence of a nucleic acid molecule. Nucleic acid may be provided in any suitable form useful for conducting a suitable sequence analysis.

Nucleic acid may be single or double stranded. Single stranded DNA, for example, can be generated by denaturing double stranded DNA by heating or by treatment with alkali, for example. Nucleic acid may be in a D-loop structure, formed by strand invasion of a duplex DNA molecule by an oligonucleotide or a DNA-like molecule such as peptide nucleic acid (PNA). D loop formation can be facilitated by addition of E. Coli RecA protein and/or by alteration of salt concentration, for example, using methods known in the art.

*Minority vs. Majority Species*

At least two different nucleic acid species can exist in different amounts in extracellular (e.g., circulating cell-free) nucleic acid and sometimes are referred to as minority species and majority species. In certain instances, a minority species of nucleic acid is from an affected cell type (e.g., cancer cell, wasting cell, cell attacked by immune system). A chromosome alteration may be determined for a minority nucleic acid species. A chromosome alteration may be determined for a majority nucleic acid species. As used herein, it is not intended that the terms "minority" or "majority" be rigidly defined in any respect. In one aspect, a nucleic acid that is considered "minority", for example, can have an abundance of at least about 0.1% of the total nucleic acid in a sample to less than 50% of the total nucleic acid in a sample. A minority nucleic acid may have an abundance of at least about 1% of the total nucleic acid in a sample to about 40% of the total nucleic acid in a sample. A minority nucleic acid may have an abundance of at least about 2% of the total nucleic acid in a sample to about 30% of the total nucleic acid in a sample. A minority nucleic acid may have an abundance of at least about 3% of the total nucleic acid in a sample to about 25% of the total nucleic acid in a sample. For example, a minority nucleic acid can have an abundance of about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% of the total nucleic acid in a sample. In some instances, a minority species of extracellular nucleic acid sometimes is about 1% to about 40% of the overall nucleic acid (e.g., about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40% of the nucleic acid is minority species nucleic acid). The minority nucleic acid may be extracellular DNA. The minority nucleic acid may be extracellular DNA from apoptotic tissue. The minority nucleic acid may be extracellular DNA from tissue affected by a cell proliferative disorder. The minority nucleic acid may be extracellular DNA from a tumor cell. The minority nucleic acid may be extracellular fetal DNA.

In another aspect, a nucleic acid that is considered "majority", for example, can have an abundance greater than 50% of the total nucleic acid in a sample to about 99.9% of the total nucleic acid in a sample. A majority nucleic acid may have an abundance of at least about 60% of the total nucleic acid in a sample to about 99% of the total nucleic acid in a sample. A majority nucleic acid may have an abundance of at least about 70% of the total nucleic acid in a sample to about 98% of the total nucleic acid in a sample. A majority nucleic acid may have an abundance of at least about 75% of the total nucleic acid in a sample to about 97% of the total nucleic acid in a sample. For example, a majority nucleic acid can have an abundance of at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% of the total nucleic acid in a sample. The majority nucleic acid may be extracellular DNA. The majority nucleic acid may be extracellular maternal DNA. The majority nucleic acid may be DNA from healthy tissue. The majority nucleic acid may be DNA from non-tumor cells.

A minority species of extracellular nucleic acid may be of a length of about 500 base pairs or less (e.g., about 80, 85, 90, 91, 92, 93, 94, 95, 96, 97, 98, 99 or 100% of minority species nucleic acid is of a length of about 500 base pairs or less). A minority species of extracellular nucleic acid may be of a length of about 300 base pairs or less (e.g., about 80, 85, 90, 91, 92, 93, 94, 95, 96, 97, 98, 99 or 100% of minority species nucleic acid is of a length of about 300 base pairs or less). A minority species of extracellular nucleic acid may be of a length of about 200 base pairs or less (e.g., about 80, 85, 90, 91, 92, 93, 94, 95, 96, 97, 98, 99 or 100% of minority species nucleic acid is of a length of about 200 base pairs or less). A minority species of extracellular nucleic acid may be of a length of about 150 base pairs or less (e.g., about 80, 85, 90, 91, 92, 93, 94, 95, 96, 97, 98, 99 or 100% of minority species nucleic acid is of a length of about 150 base pairs or less).

*Cell types*

As used herein, a "cell type" refers to a type of cell that can be distinguished from another type of cell. Extracellular nucleic acid can include nucleic acid from several different cell types. Non-limiting examples of cell types that can contribute nucleic acid to circulating cell-free nucleic acid include liver cells (e.g., hepatocytes), lung cells, spleen cells, pancreas cells, colon cells, skin cells, bladder cells, eye cells, brain cells, esophagus cells, cells of the head, cells of the neck, cells of the ovary, cells of the testes, prostate cells, placenta cells, epithelial cells, endothelial cells, adipocyte cells, kidney/renal cells, heart cells, muscle cells, blood cells (e.g., white blood cells), central nervous system (CNS) cells, the like and combinations of the foregoing. Cell types that contribute nucleic acid to circulating cell-free nucleic acid analyzed may include white blood cells, endothelial cells and hepatocyte liver cells. Different cell types can be screened as part of identifying and selecting nucleic acid loci for which a marker state is the same or substantially the same for a cell type in subjects having a medical condition and for the cell type in subjects not having the medical condition, as described in further detail herein.

A particular cell type sometimes remains the same or substantially the same in subjects having a medical condition and in subjects not having a medical condition. In a non-limiting example, the number of living or viable cells of a particular cell type may be reduced in a cell degenerative condition, and the living, viable cells are not modified, or are not modified significantly, in subjects having the medical condition.

A particular cell type sometimes is modified as part of a medical condition and has one or more different properties than in its original state. In a non-limiting example, a particular cell type may proliferate at a higher than normal rate, may transform into a cell having a different morphology, may transform into a cell that expresses one or more different cell surface markers and/or may become part of a tumor, as part of a cancer condition. In aspects for which a particular cell type (i.e., a progenitor cell) is modified as part of a medical condition, the marker state for each of the one or more markers assayed often is the same or substantially the same for the particular cell type in subjects having the medical condition and for the particular cell type in subjects not having the medical condition. Thus, the term "cell type" sometimes pertains to a type of cell in subjects not having a medical condition, and to a modified version of the cell in subjects having the medical condition. A "cell type" may be a progenitor cell only and not a modified version arising from the progenitor cell. A "cell type" sometimes pertains to a progenitor cell and a modified cell arising from the progenitor cell. A marker state for a marker analyzed often may be the same or substantially the same for a cell type in subjects having a medical condition and for the cell type in subjects not having the medical condition.

A cell type may be a cancer cell. Certain cancer cell types include, for example, leukemia cells (e.g., acute myeloid leukemia, acute lymphoblastic leukemia, chronic myeloid leukemia, chronic lymphoblastic leukemia); cancerous kidney/renal cells (e.g., renal cell cancer (clear cell, papillary type 1, papillary type 2, chromophobe, oncocytic, collecting duct), renal adenocarcinoma, hypernephroma, Wilm's tumor, transitional cell carcinoma); brain tumor cells (e.g., acoustic neuroma, astrocytoma (grade I: pilocytic astrocytoma, grade II: low-grade astrocytoma, grade III: anaplastic astrocytoma, grade IV: glioblastoma (GBM)), chordoma, cns lymphoma, craniopharyngioma, glioma (brain stem glioma, ependymoma, mixed glioma, optic nerve glioma, subependymoma), medulloblastoma, meningioma, metastatic brain tumors, oligodendroglioma, pituitary tumors, primitive neuroectodermal (PNET), schwannoma, juvenile pilocytic astrocytoma (JPA), pineal tumor, rhabdoid tumor).

Different cell types can be distinguished by any suitable characteristic, including without limitation, one or more different cell surface markers, one or more different morphological features, one or more different functions, one or more different protein (e.g., histone) modifications and one or more different nucleic acid markers. Non-limiting examples of nucleic acid markers include single-nucleotide polymorphisms (SNPs), methylation state of a nucleic acid locus, short tandem repeats, insertions (e.g., micro-insertions), deletions (micro-deletions) the like and combinations thereof. Non-limiting examples of protein (e.g., histone) modifications include acetylation, methylation, ubiquitylation, phosphorylation, sumoylation, the like and combinations thereof.

As used herein, the term a "related cell type" refers to a cell type having multiple characteristics in common with another cell type. In related cell types, 75% or more cell surface markers sometimes are common to the cell types (e.g., about 80%, 85%, 90% or 95% or more of cell surface markers are common to the related cell types).

*Enriching nucleic acids*

Nucleic acid (e.g., extracellular nucleic acid) is enriched or relatively enriched for a subpopulation or species of nucleic acid. Nucleic acid subpopulations can include, for example, fetal nucleic acid, maternal nucleic acid, cancer nucleic acid, patient nucleic acid, nucleic acid comprising fragments of a particular length or range of lengths, or nucleic acid from a particular genome region (e.g., single chromosome, set of chromosomes, and/or certain chromosome regions). Such enriched samples can be used in conjunction with a method provided herein. Thus, methods of the technology may comprise an additional step of enriching for a subpopulation of nucleic acid in a sample, such as, for example, cancer or fetal nucleic acid. A method for determining cancer or fetal fraction also may be used to enrich for cancer or fetal nucleic acid. Maternal nucleic acid may be selectively removed (partially, substantially, almost completely or completely) from the sample. Enriching for a particular low copy number species nucleic acid (e.g., cancer or fetal nucleic acid) may improve quantitative sensitivity. Methods for enriching a sample for a particular species of nucleic acid are described, for example, in United States Patent No. 6,927,028, International Patent Application Publication No. WO2007/140417, International Patent Application Publication No. WO2007/147063, International Patent Application Publication No. WO2009/032779, International Patent Application Publication No. WO2009/032781, International Patent Application Publication No. WO2010/033639, International Patent Application Publication No. WO2011/034631, International Patent Application Publication No. WO2006/056480, and International Patent Application Publication No. WO2011/143659.

Nucleic acid may be enriched for certain target fragment species and/or reference fragment species. Nucleic acid may be enriched for a specific nucleic acid fragment length or range of fragment lengths using one or more length-based separation methods described below. Nucleic acid may be enriched for fragments from a select genomic region (e.g., chromosome) using one or more sequence-based separation methods described herein and/or known in the art. Certain methods for enriching for a nucleic acid subpopulation (e.g., fetal nucleic acid) in a sample are described in detail below.

Some methods for enriching for a nucleic acid subpopulation (e.g., fetal nucleic acid) that can be used with a method described herein include methods that exploit epigenetic differences between maternal and fetal nucleic acid. For example, fetal nucleic acid can be differentiated and separated from maternal nucleic acid based on methylation differences. Methylation-based fetal nucleic acid enrichment methods are described in U.S. Patent Application Publication No. 2010/0105049. Such methods sometimes involve binding a sample nucleic acid to a methylation-specific binding agent (methyl-CpG binding protein (MBD), methylation specific antibodies, and the like) and separating bound nucleic acid from unbound nucleic acid based on differential methylation status. Such methods also can include the use of methylation-sensitive restriction enzymes (as described above; e.g., Hhal and Hpall), which allow for the enrichment of fetal nucleic acid regions in a maternal sample by selectively digesting nucleic acid from the maternal sample with an enzyme that selectively and completely or substantially digests the maternal nucleic acid to enrich the sample for at least one fetal nucleic acid region.

Another method for enriching for a nucleic acid subpopulation (e.g., fetal nucleic acid) that can be used with a method described herein is a restriction endonuclease enhanced polymorphic sequence approach, such as a method described in U.S. Patent Application Publication No. 2009/0317818. Such methods include cleavage of nucleic acid comprising a non-target allele with a restriction endonuclease that recognizes the nucleic acid comprising the non-target allele but not the target allele; and amplification of uncleaved nucleic acid but not cleaved nucleic acid, where the uncleaved, amplified nucleic acid represents enriched target nucleic acid (e.g., fetal nucleic acid) relative to non-target nucleic acid (e.g., maternal nucleic acid). Nucleic acid may be selected such that it comprises an allele having a polymorphic site that is susceptible to selective digestion by a cleavage agent, for example.

Some methods for enriching for a nucleic acid subpopulation (e.g., fetal nucleic acid) that can be used with a method described herein include selective enzymatic degradation approaches. Such methods involve protecting target sequences from exonuclease digestion thereby facilitating the elimination in a sample of undesired sequences (e.g., maternal DNA). For example, in one approach, sample nucleic acid is denatured to generate single stranded nucleic acid, single stranded nucleic acid is contacted with at least one target-specific primer pair under suitable annealing conditions, annealed primers are extended by nucleotide polymerization generating double stranded target sequences, and digesting single stranded nucleic acid using a nuclease that digests single stranded (e.g., non-target) nucleic acid. The method can be repeated for at least one additional cycle. The same target-specific primer pair may be used to prime each of the first and second cycles of extension, and different target-specific primer pairs may be used for the first and second cycles.

Some methods for enriching for a nucleic acid subpopulation (e.g., fetal nucleic acid) that can be used with a method described herein include massively parallel signature sequencing (MPSS) approaches. MPSS typically is a solid phase method that uses adapter (e.g., tag) ligation, followed by adapter decoding, and reading of the nucleic acid sequence in small increments. Tagged PCR products are typically amplified such that each nucleic acid generates a PCR product with a unique tag. Tags are often used to attach the PCR products to microbeads. After several rounds of ligation-based sequence determination, for example, a sequence signature can be identified from each bead. Each signature sequence (MPSS tag) in a MPSS dataset is analyzed, compared with all other signatures, and all identical signatures are counted.

Certain enrichment methods (e.g., certain MPS and/or MPSS-based enrichment methods) may include amplification (e.g., PCR)-based approaches. Loci-specific amplification methods may be be used (e.g., using loci-specific amplification primers). A multiplex SNP allele PCR approach may be used. A multiplex SNP allele PCR approach may be used in combination with uniplex sequencing. For example, such an approach can involve the use of multiplex PCR (e.g., MASSARRAY system) and incorporation of capture probe sequences into the amplicons followed by sequencing using, for example, the Illumina MPSS system. A multiplex SNP allele PCR approach may be be used in combination with a three-primer system and indexed sequencing. For example, such an approach can involve the use of multiplex PCR (e.g., MASSARRAY system) with primers having a first capture probe incorporated into certain loci-specific forward PCR primers and adapter sequences incorporated into loci-specific reverse PCR primers, to thereby generate amplicons, followed by a secondary PCR to incorporate reverse capture sequences and molecular index barcodes for sequencing using, for example, the Illumina MPSS system. A multiplex SNP allele PCR approach may be used in combination with a four-primer system and indexed sequencing. For example, such an approach can involve the use of multiplex PCR (e.g., MASSARRAY system) with primers having adaptor sequences incorporated into both loci-specific forward and loci-specific reverse PCR primers, followed by a secondary PCR to incorporate both forward and reverse capture sequences and molecular index barcodes for sequencing using, for example, the Illumina MPSS system. A microfluidics approach may be used. An array-based microfluidics approach may be used. For example, such an approach can involve the use of a microfluidics array (e.g., Fluidigm) for amplification at low plex and incorporation of index and capture probes, followed by sequencing. An emulsion microfluidics approach may be used, such as, for example, digital droplet PCR.

Universal amplification methods may be used (e.g., using universal or non-loci-specific amplification primers). Universal amplification methods may be used in combination with pull-down approaches. A method may include biotinylated ultramer pull-down (e.g., biotinylated pull-down assays from Agilent or IDT) from a universally amplified sequencing library. For example, such an approach can involve preparation of a standard library, enrichment for selected regions by a pull-down assay, and a secondary universal amplification step. Pull-down approaches may be used in combination with ligation-based methods. A method may include biotinylated ultramer pull down with sequence specific adapter ligation (e.g., HALOPLEX PCR, Halo Genomics). For example, such an approach can involve the use of selector probes to capture restriction enzyme-digested fragments, followed by ligation of captured products to an adaptor, and universal amplification followed by sequencing. Pull-down approaches may be used in combination with extension and ligation-based methods. A method may include molecular inversion probe (MIP) extension and ligation. For example, such an approach can involve the use of molecular inversion probes in combination with sequence adapters followed by universal amplification and sequencing. Complementary DNA may be synthesized and sequenced without amplification.

Extension and ligation approaches may be performed without a pull-down component. A method may include loci-specific forward and reverse primer hybridization, extension and ligation. Such methods can further include universal amplification or complementary DNA synthesis without amplification, followed by sequencing. Such methods may reduce or exclude background sequences during analysis.

Pull-down approaches may be used with an optional amplification component or with no amplification component. A method may include a modified pull-down assay and ligation with full incorporation of capture probes without universal amplification. For example, such an approach can involve the use of modified selector probes to capture restriction enzyme-digested fragments, followed by ligation of captured products to an adaptor, optional amplification; and sequencing. A method may include a biotinylated pull-down assay with extension and ligation of adaptor sequence in combination with circular single stranded ligation. For example, such an approach can involve the use of selector probes to capture regions of interest (e.g., target sequences), extension of the probes, adaptor ligation, single stranded circular ligation, optional amplification, and sequencing. The analysis of the sequencing result may separate target sequences form background.

Nucleic acid may be enriched for fragments from a select genomic region (e.g., chromosome) using one or more sequence-based separation methods described herein. Sequence-based separation generally is based on nucleotide sequences present in the fragments of interest (e.g., target and/or reference fragments) and substantially not present in other fragments of the sample or present in an insubstantial amount of the other fragments (e.g., 5% or less). Sequence-based separation may generate separated target fragments and/or separated reference fragments. Separated target fragments and/or separated reference fragments often are isolated away from the remaining fragments in the nucleic acid sample. The separated target fragments and the separated reference fragments also may be isolated away from each other (e.g., isolated in separate assay compartments). The separated target fragments and the separated reference fragments may be isolated together (e.g., isolated in the same assay compartment). Unbound fragments may be differentially removed or degraded or digested.

A selective nucleic acid capture process may be used to separate target and/or reference fragments away from the nucleic acid sample. Commercially available nucleic acid capture systems include, for example, Nimblegen sequence capture system (Roche NimbleGen, Madison, WI); Illumina BEADARRAY platform (Illumina, San Diego, CA); Affymetrix GENECHIP platform (Affymetrix, Santa Clara, CA); Agilent SureSelect Target Enrichment System (Agilent Technologies, Santa Clara, CA); and related platforms. Such methods typically involve hybridization of a capture oligonucleotide to a segment or all of the nucleotide sequence of a target or reference fragment and can include use of a solid phase (e.g., solid phase array) and/or a solution based platform. Capture oligonucleotides (sometimes referred to as "bait") can be selected or designed such that they preferentially hybridize to nucleic acid fragments from selected genomic regions or loci (e.g., one of chromosomes 21, 18, 13, X or Y, or a reference chromosome). A hybridization-based method (e.g., using oligonucleotide arrays) may be be used to enrich for nucleic acid sequences from certain chromosomes (e.g., a potentially aneuploid chromosome, reference chromosome or other chromosome of interest) or segments of interest thereof.

Nucleic acid may be enriched for a particular nucleic acid fragment length, range of lengths, or lengths under or over a particular threshold or cutoff using one or more length-based separation methods. Nucleic acid fragment length typically refers to the number of nucleotides in the fragment. Nucleic acid fragment length also is sometimes referred to as nucleic acid fragment size. A length-based separation method may be performed without measuring lengths of individual fragments. A length based separation method may be performed in conjunction with a method for determining length of individual fragments. Length-based separation may refer to a size fractionation procedure where all or part of the fractionated pool can be isolated (e.g., retained) and/or analyzed. Size fractionation procedures are known in the art (e.g., separation on an array, separation by a molecular sieve, separation by gel electrophoresis, separation by column chromatography (e.g., size-exclusion columns), and microfluidics-based approaches). Length-based separation approaches may include fragment circularization, chemical treatment (e.g., formaldehyde, polyethylene glycol (PEG)), mass spectrometry and/or size-specific nucleic acid amplification, for example.

Certain length-based separation methods that can be used with methods described herein employ a selective sequence tagging approach, for example. The term "sequence tagging" refers to incorporating a recognizable and distinct sequence into a nucleic acid or population of nucleic acids. The term "sequence tagging" as used herein has a different meaning than the term "sequence tag" described later herein. In such sequence tagging methods, a fragment size species (e.g., short fragments) nucleic acids are subjected to selective sequence tagging in a sample that includes long and short nucleic acids. Such methods typically involve performing a nucleic acid amplification reaction using a set of nested primers which include inner primers and outer primers. One or both of the inner may be be tagged to thereby introduce a tag onto the target amplification product. The outer primers generally do not anneal to the short fragments that carry the (inner) target sequence. The inner primers can anneal to the short fragments and generate an amplification product that carries a tag and the target sequence. Typically, tagging of the long fragments is inhibited through a combination of mechanisms which include, for example, blocked extension of the inner primers by the prior annealing and extension of the outer primers. Enrichment for tagged fragments can be accomplished by any of a variety of methods, including for example, exonuclease digestion of single stranded nucleic acid and amplification of the tagged fragments using amplification primers specific for at least one tag.

Another length-based separation method that can be used with methods described herein involves subjecting a nucleic acid sample to polyethylene glycol (PEG) precipitation. Examples of methods include those described in International Patent Application Publication Nos. WO2007/140417 and WO2010/115016, . This method in general entails contacting a nucleic acid sample with PEG in the presence of one or more monovalent salts under conditions sufficient to substantially precipitate large nucleic acids without substantially precipitating small (e.g., less than 300 nucleotides) nucleic acids.

Another size-based enrichment method that can be used with methods described herein involves circularization by ligation, for example, using circligase. Short nucleic acid fragments typically can be circularized with higher efficiency than long fragments. Non-circularized sequences can be separated from circularized sequences, and the enriched short fragments can be used for further analysis.

*Nucleic acid library*

A nucleic acid library may be a plurality of polynucleotide molecules (e.g., a sample of nucleic acids) that are prepared, assemble and/or modified for a specific process, non-limiting examples of which include immobilization on a solid phase (e.g., a solid support, e.g., a flow cell, a bead), enrichment, amplification, cloning, detection and/or for nucleic acid sequencing. A nucleic acid library may be prepared prior to or during a sequencing process. A nucleic acid library (e.g., sequencing library) can be prepared by a suitable method as known in the art. A nucleic acid library can be prepared by a targeted or a non-targeted preparation process.

A library of nucleic acids may be modified to comprise a chemical moiety (e.g., a functional group) configured for immobilization of nucleic acids to a solid support. A library of nucleic acids may be modified to comprise a biomolecule (e.g., a functional group) and/or member of a binding pair configured for immobilization of the library to a solid support, non-limiting examples of which include thyroxin-binding globulin, steroid-binding proteins, antibodies, antigens, haptens, enzymes, lectins, nucleic acids, repressors, protein A, protein G, avidin, streptavidin, biotin, complement component C1q, nucleic acid-binding proteins, receptors, carbohydrates, oligonucleotides, polynucleotides, complementary nucleic acid sequences, the like and combinations thereof. Some examples of specific binding pairs include, without limitation: an avidin moiety and a biotin moiety; an antigenic epitope and an antibody or immunologically reactive fragment thereof; an antibody and a hapten; a digoxigen moiety and an anti-digoxigen antibody; a fluorescein moiety and an anti-fluorescein antibody; an operator and a repressor; a nuclease and a nucleotide; a lectin and a polysaccharide; a steroid and a steroid-binding protein; an active compound and an active compound receptor; a hormone and a hormone receptor; an enzyme and a substrate; an immunoglobulin and protein A; an oligonucleotide or polynucleotide and its corresponding complement; the like or combinations thereof.

A library of nucleic acids may be modified to comprise one or more polynucleotides of known composition, non-limiting examples of which include an identifier (e.g., a tag, an indexing tag), a capture sequence, a label, an adapter, a restriction enzyme site, a promoter, an enhancer, an origin of replication, a stem loop, a complimentary sequence (e.g., a primer binding site, an annealing site), a suitable integration site (e.g., a transposon, a viral integration site), a modified nucleotide, the like or combinations thereof. Polynucleotides of known sequence can be added at a suitable position, for example on the 5' end, 3' end or within a nucleic acid sequence. Polynucleotides of known sequence can be the same or different sequences. A polynucleotide of known sequence may be configured to hybridize to one or more oligonucleotides immobilized on a surface (e.g., a surface in flow cell). For example, a nucleic acid molecule comprising a 5' known sequence may hybridize to a first plurality of oligonucleotides while the 3' known sequence may hybridize to a second plurality of oligonucleotides. A library of nucleic acid may comprise chromosome-specific tags, capture sequences, labels and/or adaptors. A library of nucleic acids may comprise one or more detectable labels. One or more detectable labels may be incorporated into a nucleic acid library at a 5' end, at a 3' end, and/or at any nucleotide position within a nucleic acid in the library. A library of nucleic acids may comprise hybridized oligonucleotides. Hybridized oligonucleotides may be labeled probes. A library of nucleic acids may comprise hybridized oligonucleotide probes prior to immobilization on a solid phase.

A polynucleotide of known sequence may comprise a universal sequence. A universal sequence is a specific nucleotide acid sequence that is integrated into two or more nucleic acid molecules or two or more subsets of nucleic acid molecules where the universal sequence is the same for all molecules or subsets of molecules that it is integrated into. A universal sequence is often designed to hybridize to and/or amplify a plurality of different sequences using a single universal primer that is complementary to a universal sequence. Two (e.g., a pair) or more universal sequences and/or universal primers may be used. A universal primer often comprises a universal sequence. Adapters (e.g., universal adapters) may comprise universal sequences. One or more universal sequences may be used to capture, identify and/or detect multiple species or subsets of nucleic acids.

In certain aspects of preparing a nucleic acid library, (e.g., in certain sequencing by synthesis procedures), nucleic acids are size selected and/or fragmented into lengths of several hundred base pairs, or less (e.g., in preparation for library generation). Library preparation may be performed without fragmentation (e.g., when using ccfDNA).

A ligation-based library preparation method may be used (e.g., ILLUMINA TRUSEQ, Illumina, San Diego CA). Ligation-based library preparation methods often make use of an adaptor (e.g., a methylated adaptor) design which can incorporate an index sequence at the initial ligation step and often can be used to prepare samples for single-read sequencing, paired-end sequencing and multiplexed sequencing. For example, sometimes nucleic acids (e.g., fragmented nucleic acids or ccfDNA) are end repaired by a fill-in reaction, an exonuclease reaction or a combination thereof. The resulting blunt-end repaired nucleic acid may then be extended by a single nucleotide, which is complementary to a single nucleotide overhang on the 3' end of an adapter/primer. Any nucleotide can be used for the extension/overhang nucleotides. Nucleic acid library preparation may comprise ligating an adapter oligonucleotide. Adapter oligonucleotides are often complementary to flow-cell anchors, and sometimes are utilized to immobilize a nucleic acid library to a solid support, such as the inside surface of a flow cell, for example. An adapter oligonucleotide may comprise an identifier, one or more sequencing primer hybridization sites (e.g., sequences complementary to universal sequencing primers, single end sequencing primers, paired end sequencing primers, multiplexed sequencing primers, and the like), or combinations thereof (e.g., adapter/sequencing, adapter/identifier, adapter/identifier/sequencing).

An identifier can be a suitable detectable label incorporated into or attached to a nucleic acid (e.g., a polynucleotide) that allows detection and/or identification of nucleic acids that comprise the identifier. An identifier may be incorporated into or attached to a nucleic acid during a sequencing method (e.g., by a polymerase). Non-limiting examples of identifiers include nucleic acid tags, nucleic acid indexes or barcodes, a radiolabel (e.g., an isotope), metallic label, a fluorescent label, a chemiluminescent label, a phosphorescent label, a fluorophore quencher, a dye, a protein (e.g., an enzyme, an antibody or part thereof, a linker, a member of a binding pair), the like or combinations thereof. An identifier (e.g., a nucleic acid index or barcode) may be a unique, known and/or identifiable sequence of nucleotides or nucleotide analogues. Identifiers may be six or more contiguous nucleotides. A multitude of fluorophores are available with a variety of different excitation and emission spectra. Any suitable type and/or number of fluorophores can be used as an identifier. 1 or more, 2 or more, 3 or more, 4 or more, 5 or more, 6 or more, 7 or more, 8 or more, 9 or more, 10 or more, 20 or more, 30 or more or 50 or more different identifiers may be utilized in a method described herein (e.g., a nucleic acid detection and/or sequencing method). One or two types of identifiers (e.g., fluorescent labels) may be linked to each nucleic acid in a library. Detection and/or quantification of an identifier can be performed by a suitable method, apparatus or machine, non-limiting examples of which include flow cytometry, quantitative polymerase chain reaction (qPCR), gel electrophoresis, a luminometer, a fluorometer, a spectrophotometer, a suitable gene-chip or microarray analysis, Western blot, mass spectrometry, chromatography, cytofluorimetric analysis, fluorescence microscopy, a suitable fluorescence or digital imaging method, confocal laser scanning microscopy, laser scanning cytometry, affinity chromatography, manual batch mode separation, electric field suspension, a suitable nucleic acid sequencing method and/or nucleic acid sequencing apparatus, the like and combinations thereof.

A transposon-based library preparation method may be used (e.g., EPICENTRE NEXTERA, Epicentre, Madison WI). Transposon-based methods typically use in vitro transposition to simultaneously fragment and tag DNA in a single-tube reaction (often allowing incorporation of platform-specific tags and optional barcodes); and prepare sequencer-ready libraries.

A nucleic acid library or parts thereof may be amplified (e.g., amplified by a PCR-based method). A sequencing method may comprise amplification of a nucleic acid library. A nucleic acid library can be amplified prior to or after immobilization on a solid support (e.g., a solid support in a flow cell). Nucleic acid amplification includes the process of amplifying or increasing the numbers of a nucleic acid template and/or of a complement thereof that are present (e.g., in a nucleic acid library), by producing one or more copies of the template and/or its complement. Amplification can be carried out by a suitable method. A nucleic acid library can be amplified by a thermocycling method or by an isothermal amplification method. A rolling circle amplification method may be used. Amplification may take place on a solid support (e.g., within a flow cell) where a nucleic acid library or portion thereof is immobilized. In certain sequencing methods, a nucleic acid library is added to a flow cell and immobilized by hybridization to anchors under suitable conditions. This type of nucleic acid amplification is often referred to as solid phase amplification. In some aspects of solid phase amplification, all or a portion of the amplified products are synthesized by an extension initiating from an immobilized primer. Solid phase amplification reactions are analogous to standard solution phase amplifications except that at least one of the amplification oligonucleotides (e.g., primers) is immobilized on a solid support.

Solid phase amplification may comprise a nucleic acid amplification reaction comprising only one species of oligonucleotide primer immobilized to a surface. Solid phase amplification may comprise a plurality of different immobilized oligonucleotide primer species. Solid phase amplification may comprise a nucleic acid amplification reaction comprising one species of oligonucleotide primer immobilized on a solid surface and a second different oligonucleotide primer species in solution. Multiple different species of immobilized or solution based primers can be used. Non-limiting examples of solid phase nucleic acid amplification reactions include interfacial amplification, bridge amplification, emulsion PCR, WildFire amplification (e.g., US patent publication US20130012399), the like or combinations thereof.

*Sequencing*

Nucleic acids (e.g., nucleic acid fragments, sample nucleic acid, cell-free nucleic acid) may be sequenced. A full or substantially full sequence may be obtained and sometimes a partial sequence is obtained.

Some or all nucleic acids in a sample may be enriched and/or amplified (e.g., non-specifically, e.g., by a PCR based method) prior to or during sequencing. Specific nucleic acid portions or subsets in a sample may be enriched and/or amplified prior to or during sequencing. A portion or subset of a pre-selected pool of nucleic acids may be sequenced randomly. Nucleic acids in a sample may not be enriched and/or amplified prior to or during sequencing.

As used herein, "reads" (e.g., "a read", "a sequence read") are short nucleotide sequences produced by any sequencing process described herein or known in the art. Reads can be generated from one end of nucleic acid fragments ("single-end reads"), and sometimes are generated from both ends of nucleic acids (e.g., paired-end reads, double-end reads).

The length of a sequence read is often associated with the particular sequencing technology. High-throughput methods, for example, provide sequence reads that can vary in size from tens to hundreds of base pairs (bp). Nanopore sequencing, for example, can provide sequence reads that can vary in size from tens to hundreds to thousands of base pairs. Sequence reads may be of a mean, median, average or absolute length of about 15 bp to about 900 bp long. Sequence reads may be of a mean, median, average or absolute length about 1000 bp or more.

The nominal, average, mean or absolute length of single-end reads sometimes may be about 15 contiguous nucleotides to about 50 or more contiguous nucleotides, about 15contiguous nucleotides to about 40 or more contiguous nucleotides, and sometimes about 15 contiguous nucleotides or about 36 or more contiguous nucleotides. The nominal, average, mean or absolute length of single-end reads may be about 20 to about 30 bases, or about 24 to about 28 bases in length. The nominal, average, mean _ or absolute length of single-end reads may be about 1, 2, 3, 4, 5, 6, 7, 8, 9, 10, 11, 12, 13, 14, 15, 16, 17, 18, 19, 21, 22, 23, 24, 25, 26, 27, 28 or about 29 bases or more in length.

The nominal, average, mean or absolute length of the paired-end reads sometimes may be about 10 contiguous nucleotides to about 25 contiguous nucleotides or more (e.g., about 10, 11, 12, 13, 14, 15, 16, 17, 18, 19, 20, 21, 22, 23, 24 or 25 nucleotides in length or more), about 15 contiguous nucleotides to about 20 contiguous nucleotides or more, and sometimes is about 17 contiguous nucleotides or about 18 contiguous nucleotides.

Reads generally are representations of nucleotide sequences in a physical nucleic acid. For example, in a read containing an ATGC depiction of a sequence, "A" represents an adenine nucleotide, "T" represents a thymine nucleotide, "G" represents a guanine nucleotide and "C" represents a cytosine nucleotide, in a physical nucleic acid. Sequence reads obtained from the blood of a pregnant female can be reads from a mixture of fetal and maternal nucleic acid. A mixture of relatively short reads can be transformed by processes described herein into a representation of a genomic nucleic acid present in the pregnant female and/or in the fetus. A mixture of relatively short reads can be transformed into a representation of a copy number variation (e.g., a maternal and/or fetal copy number variation), genetic variation or an aneuploidy, for example. Reads of a mixture of maternal and fetal nucleic acid can be transformed into a representation of a composite chromosome or a segment thereof comprising features of one or both maternal and fetal chromosomes. "Obtaining" nucleic acid sequence reads of a sample from a subject and/or "obtaining" nucleic acid sequence reads of a biological specimen from one or more reference persons may involve directly sequencing nucleic acid to obtain the sequence information. "Obtaining" may involve receiving sequence information obtained directly from a nucleic acid by another.

A representative fraction of a genome may be sequenced and is sometimes referred to as "coverage" or "fold coverage". For example, a 1-fold coverage indicates that roughly 100% of the nucleotide sequences of the genome are represented by reads. "Fold coverage" is a relative term referring to a prior sequencing run as a reference. For example, a second sequencing run may have 2-fold less coverage than a first sequencing run. A genome may be sequenced with redundancy, where a given region of the genome can be covered by two or more reads or overlapping reads (e.g., a "fold coverage" greater than 1, e.g., a 2-fold coverage).

One nucleic acid sample from one individual may be sequenced. Nucleic acids from each of two or more samples may be sequenced, where samples are from one individual or from different individuals. Nucleic acid samples from two or more biological samples may be pooled, where each biological sample is from one individual or two or more individuals, and the pool is sequenced. In the latter case, a nucleic acid sample from each biological sample often is identified by one or more unique identifiers.

A sequencing method may utilize identifiers that allow multiplexing of sequence reactions in a sequencing process. The greater the number of unique identifiers, the greater the number of samples and/or chromosomes for detection, for example, that can be multiplexed in a sequencing process. A sequencing process can be performed using any suitable number of unique identifiers (e.g., 4, 8, 12, 24, 48, 96, or more).

A sequencing process sometimes makes use of a solid phase, and sometimes the solid phase comprises a flow cell on which nucleic acid from a library can be attached and reagents can be flowed and contacted with the attached nucleic acid. A flow cell sometimes includes flow cell lanes, and use of identifiers can facilitate analyzing a number of samples in each lane. A flow cell often is a solid support that can be configured to retain and/or allow the orderly passage of reagent solutions over bound analytes. Flow cells frequently are planar in shape, optically transparent, generally in the millimeter or sub-millimeter scale, and often have channels or lanes in which the analyte/reagent interaction occurs. The number of samples analyzed in a given flow cell lane may be dependent on the number of unique identifiers utilized during library preparation and/or probe design. Multiplexing using 12 identifiers, for example, allows simultaneous analysis of 96 samples (e.g., equal to the number of wells in a 96 well microwell plate) in an 8 lane flow cell. Similarly, multiplexing using 48 identifiers, for example, allows simultaneous analysis of 384 samples (e.g., equal to the number of wells in a 384 well microwell plate) in an 8 lane flow cell. Non-limiting examples of commercially available multiplex sequencing kits include Illumina's multiplexing sample preparation oligonucleotide kit and multiplexing sequencing primers and PhiX control kit (e.g., Illumina's catalog numbers PE-400-1001 and PE-400-1002, respectively).

Any suitable method of sequencing nucleic acids can be used, non-limiting examples of which include Maxim & Gilbert, chain-termination methods, sequencing by synthesis, sequencing by ligation, sequencing by mass spectrometry, microscopy-based techniques, the like or combinations thereof. A first generation technology, such as, for example, Sanger sequencing methods including automated Sanger sequencing methods, including microfluidic Sanger sequencing, may be used in a method provided herein. Sequencing technologies that include the use of nucleic acid imaging technologies (e.g., transmission electron microscopy (TEM) and atomic force microscopy (AFM)), may be used. A high-throughput sequencing method may be used. High-throughput sequencing methods generally involve clonally amplified DNA templates or single DNA molecules that are sequenced in a massively parallel fashion, sometimes within a flow cell. Next generation (e.g., 2nd and 3rd generation) sequencing techniques capable of sequencing DNA in a massively parallel fashion can be used for methods described herein and are collectively referred to herein as "massively parallel sequencing" (MPS). MPS sequencing methods may utilize a targeted approach, where specific chromosomes, genes or regions of interest are sequences. A non-targeted approach may be used where most or all nucleic acids in a sample are sequenced, amplified and/or captured randomly.

A targeted enrichment, amplification and/or sequencing approach may be used. A targeted approach often isolates, selects and/or enriches a subset of nucleic acids in a sample for further processing by use of sequence-specific oligonucleotides. A library of sequence-specific oligonucleotides may be utilized to target (e.g., hybridize to) one or more sets of nucleic acids in a sample. Sequence-specific oligonucleotides and/or primers are often selective for particular sequences (e.g., unique nucleic acid sequences) present in one or more chromosomes, genes, exons, introns, and/or regulatory regions of interest. Any suitable method or combination of methods can be used for enrichment, amplification and/or sequencing of one or more subsets of targeted nucleic acids. Targeted sequences may be isolated and/or enriched by capture to a solid phase (e.g., a flow cell, a bead) using one or more sequence-specific anchors. Targeted sequences may be enriched and/or amplified by a polymerase-based method (e.g., a PCR-based method, by any suitable polymerase based extension) using sequence-specific primers and/or primer sets. Sequence specific anchors often can be used as sequence-specific primers.

MPS sequencing sometimes makes use of sequencing by synthesis and certain imaging processes. A nucleic acid sequencing technology that may be used in a method described herein is sequencing-by-synthesis and reversible terminator-based sequencing (e.g., Illumina's Genome Analyzer; Genome Analyzer II; HISEQ 2000; HISEQ 2500 (Illumina, San Diego CA)). With this technology, millions of nucleic acid (e.g., DNA) fragments can be sequenced in parallel. In one example of this type of sequencing technology, a flow cell is used which contains an optically transparent slide with 8 individual lanes on the surfaces of which are bound oligonucleotide anchors (e.g., adaptor primers). A flow cell often is a solid support that can be configured to retain and/or allow the orderly passage of reagent solutions over bound analytes. Flow cells frequently are planar in shape, optically transparent, generally in the millimeter or sub-millimeter scale, and often have channels or lanes in which the analyte/reagent interaction occurs.

Sequencing by synthesis may comprise iteratively adding (e.g., by covalent addition) a nucleotide to a primer or preexisting nucleic acid strand in a template directed manner. Each iterative addition of a nucleotide is detected and the process is repeated multiple times until a sequence of a nucleic acid strand is obtained. The length of a sequence obtained depends, in part, on the number of addition and detection steps that are performed. In some aspects of sequencing by synthesis, one, two, three or more nucleotides of the same type (e.g., A, G, C or T) are added and detected in a round of nucleotide addition. Nucleotides can be added by any suitable method (e.g., enzymatically or chemically). For example, a polymerase or a ligase adds a nucleotide to a primer or to a preexisting nucleic acid strand in a template directed manner. In some aspects of sequencing by synthesis, different types of nucleotides, nucleotide analogues and/or identifiers are used. Reversible terminators and/or removable (e.g., cleavable) identifiers may be used. Fluorescent labeled nucleotides and/or nucleotide analogues may be used. Sequencing by synthesis may comprise a cleavage (e.g., cleavage and removal of an identifier) and/or a washing step. The addition of one or more nucleotides may be detected by a suitable method described herein or known in the art, non-limiting examples of which include any suitable imaging apparatus, a suitable camera, a digital camera, a CCD (Charge Couple Device) based imaging apparatus (e.g., a CCD camera), a CMOS (Complementary Metal Oxide Silicon)based imaging apparatus (e.g., a CMOS camera), a photo diode (e.g., a photomultiplier tube), electron microscopy, a field-effect transistor (e.g., a DNA field-effect transistor), an ISFET ion sensor (e.g., a CHEMFET sensor), the like or combinations thereof. Other sequencing methods that may be used to conduct methods herein include digital PCR and sequencing by hybridization.

Other sequencing methods that may be used to conduct methods herein include digital PCR and sequencing by hybridization. Digital polymerase chain reaction (digital PCR or dPCR) can be used to directly identify and quantify nucleic acids in a sample. Digital PCR may be performed in an emulsion. For example, individual nucleic acids are separated, e.g., in a microfluidic chamber device, and each nucleic acid is individually amplified by PCR. Nucleic acids can be separated such that there is no more than one nucleic acid per well. Different probes may be used to distinguish various alleles (e.g., fetal alleles and maternal alleles). Alleles can be enumerated to determine copy number.

Sequencing by hybridization may be used. The method involves contacting a plurality of polynucleotide sequences with a plurality of polynucleotide probes, where each of the plurality of polynucleotide probes can be optionally tethered to a substrate. The substrate may be a flat surface with an array of known nucleotide sequences. The pattern of hybridization to the array can be used to determine the polynucleotide sequences present in the sample. Each probe may be tethered to a bead, e.g., a magnetic bead or the like. Hybridization to the beads can be identified and used to identify the plurality of polynucleotide sequences within the sample.

Nanopore sequencing may be be used in a method described herein. Nanopore sequencing is a single-molecule sequencing technology whereby a single nucleic acid molecule (e.g., DNA) is sequenced directly as it passes through a nanopore.

A suitable MPS method, system or technology platform for conducting methods described herein can be used to obtain nucleic acid sequence reads. Non-limiting examples of MPS platforms include Illumina/Solex/HiSeq (e.g., Illumina's Genome Analyzer; Genome Analyzer II; HISEQ 2000; HISEQ), SOLiD, Roche/454, PACBIO and/or SMRT, Helicos True Single Molecule Sequencing, Ion Torrent and Ion semiconductor-based sequencing (e.g., as developed by Life Technologies), WildFire, 5500, 5500xl W and/or 5500xl W Genetic Analyzer based technologies (e.g., as developed and sold by Life Technologies, US patent publication no. US20130012399); Polony sequencing, Pyrosequencing, Massively Parallel Signature Sequencing (MPSS), RNA polymerase (RNAP) sequencing, LaserGen systems and methods, Nanopore-based platforms, chemical-sensitive field effect transistor (CHEMFET) array, electron microscopy-based sequencing (e.g., as developed by ZS Genetics, Halcyon Molecular), nanoball sequencing, the like or combinations thereof.

Chromosome-specific sequencing may be performed. Chromosome-specific sequencing may be performed utilizing DANSR (digital analysis of selected regions). Digital analysis of selected regions enables simultaneous quantification of hundreds of loci by cfDNA-dependent catenation of two locus-specific oligonucleotides via an intervening 'bridge' oligonucleotide to form a PCR template. Chromosome-specific sequencing may be performed by generating a library enriched in chromosome-specific sequences. Sequence reads may be obtained only for a selected set of chromosomes. Sequence reads may be obtained only for chromosomes 21, 18 and 13. Sequence reads may be obtained for and/or and mapped to an entire reference genome or a segment of a genome.

Sequence reads may be generated, obtained, gathered, assembled, manipulated, transformed, processed, and/or provided by a sequence module. A machine comprising a sequence module can be a suitable machine and/or apparatus that determines the sequence of a nucleic acid utilizing a sequencing technology known in the art. A sequence module may align, assemble, fragment, complement, reverse complement, and/or error check (e.g., error correct sequence reads).

Nucleotide sequence reads obtained from a sample may be partial nucleotide sequence reads. As used herein, "partial nucleotide sequence reads" refers to sequence reads of any length with incomplete sequence information, also referred to as sequence ambiguity. Partial nucleotide sequence reads may lack information regarding nucleobase identity and/or nucleobase position or order. Partial nucleotide sequence reads generally do not include sequence reads in which the only incomplete sequence information (or in which less than all of the bases are sequenced or determined) is from inadvertent or unintentional sequencing errors. Such sequencing errors can be inherent to certain sequencing processes and include, for example, incorrect calls for nucleobase identity, and missing or extra nucleobases. Thus, for partial nucleotide sequence reads herein, certain information about the sequence is often deliberately excluded. That is, one deliberately obtains sequence information with respect to less than all of the nucleobases or which might otherwise be characterized as or be a sequencing error. A partial nucleotide sequence read may span a portion of a nucleic acid fragment. A partial nucleotide sequence read may span the entire length of a nucleic acid fragment. Partial nucleotide sequence reads are described, for example, in International Patent Application Publication no. WO2013/052907.

*Mapping reads*

Sequence reads can be mapped and the number of reads mapping to a specified nucleic acid region (e.g., a chromosome, portion or segment thereof) are referred to as counts. Any suitable mapping method (e.g., process, algorithm, program, software, module, the like or combination thereof) can be used. Sequence reads may not be mapped. Certain aspects of mapping processes are described hereafter.

Mapping nucleotide sequence reads (i.e., sequence information from a fragment whose physical genomic position is unknown) can be performed in a number of ways, and often comprises alignment of the obtained sequence reads with a matching sequence in a reference genome. In such alignments, sequence reads generally are aligned to a reference sequence and those that align are designated as being "mapped", "a mapped sequence read" or "a mapped read". A mapped sequence read may be referred to as a "hit" or "count". Mapped sequence reads may be grouped together according to various parameters and assigned to particular portions, which are discussed in further detail below.

As used herein, the terms "aligned", "alignment", or "aligning" refer to two or more nucleic acid sequences that can be identified as a match (e.g., 100% identity) or partial match. Alignments can be done manually or by a computer (e.g., a software, program, module, or algorithm), non-limiting examples of which include the Efficient Local Alignment of Nucleotide Data (ELAND) computer program distributed as part of the Illumina Genomics Analysis pipeline. Alignment of a sequence read can be a 100% sequence match. In some cases, an alignment is less than a 100% sequence match (i.e., non-perfect match, partial match, partial alignment). An alignment may be about a 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76% or 75% match. An alignment may comprise a mismatch. An alignment may comprise 1, 2, 3, 4 or 5 mismatches. Two or more sequences can be aligned using either strand. A nucleic acid sequence may be aligned with the reverse complement of another nucleic acid sequence. Sequence reads may be aligned to a reference sequence or a reference genome. Sequence reads may not aligned to a reference sequence or a reference genome.

Various computational methods can be used to map each sequence read to a portion. Non-limiting examples of computer algorithms that can be used to align sequences include, without limitation, BLAST, BLITZ, FASTA, BOWTIE 1, BOWTIE 2, ELAND, MAQ, PROBEMATCH, SOAP or SEQMAP, or variations thereof or combinations thereof. Sequence reads may be aligned with sequences in a reference genome. The sequence reads may be found and/or aligned with sequences in nucleic acid databases known in the art including, for example, GenBank, dbEST, dbSTS, EMBL (European Molecular Biology Laboratory) and DDBJ (DNA Databank of Japan). BLAST or similar tools can be used to search the identified sequences against a sequence database. Search hits can then be used to sort the identified sequences into appropriate portions (described hereafter), for example.

Mapped sequence reads and/or information associated with a mapped sequence read may be stored on and/or accessed from a non-transitory computer-readable storage medium in a suitable computer-readable format. A "computer-readable format" is sometimes referred to generally herein as a format. Mapped sequence reads may be stored and/or accessed in a suitable binary format, a text format, the like or a combination thereof. A binary format may sometimes be a BAM format. A text format is sometimes a sequence alignment/map (SAM) format. Non-limiting examples of binary and/or text formats include BAM, SAM, SRF, FASTQ, Gzip, the like, or combinations thereof. Mapped sequence reads may be stored in and/or are converted to a format that requires less storage space (e.g., less bytes) than a traditional format (e.g., a SAM format or a BAM format). Mapped sequence reads in a first format may be compressed into a second format requiring less storage space than the first format. The term "compressed" as used herein refers to a process of data compression, source coding, and/or bit-rate reduction where a computer readable data file is reduced in size. Mapped sequence reads may be compressed from a SAM format in a binary format. Some data sometimes is lost after a file is compressed. Sometimes no data is lost in a compression process. In some file compression aspects, some data is replaced with an index and/or a reference to another data file comprising information regarding a mapped sequence read. A mapped sequence read may be stored in a binary format comprising or consisting of a read count, a chromosome identifier (e.g., that identifies a chromosome to which a read is mapped) and a chromosome position identifier (e.g., that identifies a position on a chromosome to which a read is mapped). A binary format may comprise a 20 byte array, a 16 byte array, an 8 byte array, a 4 byte array or a 2 byte array. Mapped read information may be stored in an array in a 10 byte format, 9 byte format, 8 byte format, 7 byte format, 6 byte format, 5 byte format, 4 byte format, 3 byte format or 2 byte format. Sometimes mapped read data is stored in a 4 byte array comprising a 5 byte format. A binary format may comprise a 5-byte format comprising a 1-byte chromosome ordinal and a 4-byte chromosome position. Mapped reads may be stored in a compressed binary format that is about 100 times, about 90 times, about 80 times, about 70 times, about 60 times, about 55 times, about 50 times, about 45 times, about 40 times or about 30 times smaller than a sequence alignment/map (SAM) format. Mapped reads may be stored in a compress binary format that is about 2 times smaller to about 50 times smaller than (e.g., about 30, 25, 20, 19, 18, 17, 16, 15, 14, 13, 12, 11, 10, 9, 8, 7, 6, or about 5 times smaller than) a GZip format.

A system may comprise a compression module. Mapped sequence read information stored on a non-transitory computer-readable storage medium in a computer-readable format may be compressed by a compression module. A compression module sometimes converts mapped sequence reads to and from a suitable format. A compression module may accept mapped sequence reads in a first format, convert them into a compressed format (e.g., a binary format) and transfer the compressed reads to another module (e.g., a bias density module). A compression module often provides sequence reads in a binary format (e.g., a BReads format). Non-limiting examples of a compression module include GZIP, BGZF, and BAM, the like or modifications thereof).

The following provides an example of converting an integer into a 4-byte array using java:

```
public static final byte[ ]
convertToByteArray(int value)
{
return new byte[ ] {
(byte)(value >>> 24),
(byte)(value >>> 16),
(byte)(value >>> 8),
(byte)value};
}
```

A read may uniquely or non-uniquely map to portions in a reference genome. A read is considered as "uniquely mapped" if it aligns with a single sequence in the reference genome. A read is considered as "non-uniquely mapped" if it aligns with two or more sequences in the reference genome. Non-uniquely mapped reads may be eliminated from further analysis (e.g. quantification). A certain, small degree of mismatch (0-1) may be allowed to account for single nucleotide polymorphisms that may exist between the reference genome and the reads from individual samples being mapped. No degree of mismatch may be allowed for a read mapped to a reference sequence.

As used herein, the term "reference genome" can refer to any particular known, sequenced or characterized genome, whether partial or complete, of any organism or virus which may be used to reference identified sequences from a subject. For example, a reference genome used for human subjects as well as many other organisms can be found at the National Center for Biotechnology Information at World Wide Web URL ncbi.nlm.nih.gov. A "genome" refers to the complete genetic information of an organism or virus, expressed in nucleic acid sequences. As used herein, a reference sequence or reference genome often is an assembled or partially assembled genomic sequence from an individual or multiple individuals. A reference genome may be an assembled or partially assembled genomic sequence from one or more human individuals. A reference genome may comprise sequences assigned to chromosomes.

In certain aspects, where a sample nucleic acid is from a pregnant female, a reference sequence sometimes is not from the fetus, the mother of the fetus or the father of the fetus, and is referred to herein as an "external reference." A maternal reference may be prepared and used in some aspects. When a reference from the pregnant female is prepared ("maternal reference sequence") based on an external reference, reads from DNA of the pregnant female that contains substantially no fetal DNA often are mapped to the external reference sequence and assembled. The external reference may be from DNA of an individual having substantially the same ethnicity as the pregnant female. A maternal reference sequence may not completely cover the maternal genomic DNA (e.g., it may cover about 50%, 60%, 70%, 80%, 90% or more of the maternal genomic DNA), and the maternal reference may not perfectly match the maternal genomic DNA sequence (e.g., the maternal reference sequence may include multiple mismatches).

Mappability may be assessed for a genomic region (e.g., portion, genomic portion, portion). Mappability is the ability to unambiguously align a nucleotide sequence read to a portion of a reference genome, typically up to a specified number of mismatches, including, for example, 0, 1, 2 or more mismatches. For a given genomic region, the expected mappability can be estimated using a sliding-window approach of a preset read length and averaging the resulting read-level mappability values. Genomic regions comprising stretches of unique nucleotide sequence sometimes have a high mappability value.

*Portions*

Mapped sequence reads (i.e. sequence tags) may be grouped together according to various parameters and assigned to particular portions (e.g., portions of a reference genome). Often, individual mapped sequence reads can be used to identify a portion (e.g., the presence, absence or amount of a portion) present in a sample. The amount of a portion may be indicative of the amount of a larger sequence (e.g. a chromosome) in the sample. The term "portion" can also be referred to herein as a "genomic section", "bin", "region", "partition", "portion of a reference genome", "portion of a chromosome" or "genomic portion." A portion may be an entire chromosome, a segment of a chromosome, a segment of a reference genome, a segment spanning multiple chromosome, multiple chromosome segments, and/or combinations thereof. A portion may be predefined based on specific parameters. A portion may be arbitrarily defined based on partitioning of a genome (e.g., partitioned by size, GC content, sequencing coverage variability, contiguous regions, contiguous regions of an arbitrarily defined size, and the like).

A portion may be delineated based on one or more parameters which include, for example, length or a particular feature or features of the sequence. Portions can be selected, filtered and/or removed from consideration using any suitable criteria know in the art or described herein. A portion may be based on a particular length of genomic sequence. A method may include analysis of multiple mapped sequence reads to a plurality of portions. Portions can be approximately the same length or portions can be different lengths. In Portions may be of about equal length. Portions of different lengths may be adjusted or weighted. A portion may be about 10 kilobases (kb) to about 20 kb, about 10 kb to about 100 kb, about 20 kb to about 80 kb, about 30 kb to about 70 kb, about 40 kb to about 60 kb. A portion may be about 10 kb, 20 kb, 30 kb, 40 kb, 50 kb or about 60 kb in length. A portion is not limited to contiguous runs of sequence. Thus, portions can be made up of contiguous and/or non-contiguous sequences. A portion is not limited to a single chromosome. A portion may include all or part of one chromosome or all or part of two or more chromosomes. Portions may span one, two, or more entire chromosomes. In addition, portions may span jointed or disjointed regions of multiple chromosomes.

Portions can be particular chromosome segments in a chromosome of interest, such as, for example, a chromosome where a copy number variation is assessed (e.g. an aneuploidy of chromosomes 13, 18 and/or 21 or a sex chromosome). A portion can also be a pathogenic genome (e.g. bacterial, fungal or viral) or fragment thereof. Portions can be genes, gene fragments, regulatory sequences, introns, exons, and the like.

A genome (e.g. human genome) may be partitioned into portions based on information content of particular regions. Partitioning a genome may eliminate similar regions (e.g., identical or homologous regions or sequences) across the genome and only keep unique regions. Regions removed during partitioning may be within a single chromosome or may span multiple chromosomes. A partitioned genome may be trimmed down and optimized for faster alignment, often allowing for focus on uniquely identifiable sequences.

Partitioning may down weight similar regions. A process for down weighting a portion is discussed in further detail below.

Partitioning of a genome into regions transcending chromosomes may be based on information gain produced in the context of classification. For example, information content may be quantified using a p-value profile measuring the significance of particular genomic locations for distinguishing between groups of confirmed normal and abnormal subjects (e.g. euploid and trisomy subjects, respectively). Partitioning of a genome into regions transcending chromosomes may be based on any other criterion, such as, for example, speed/convenience while aligning tags, GC content (e.g., high or low GC content), uniformity of GC content, other measures of sequence content (e.g. fraction of individual nucleotides, fraction of pyrimidines or purines, fraction of natural vs. non-natural nucleic acids, fraction of methylated nucleotides, and CpG content), methylation state, duplex melting temperature, amenability to sequencing or PCR, uncertainty value assigned to individual portions of a reference genome, and/or a targeted search for particular features.

A "segment" of a chromosome generally is part of a chromosome, and typically is a different part of a chromosome than a portion. A segment of a chromosome sometimes is in a different region of a chromosome than a portion, sometimes does not share a polynucleotide with a portion, and sometimes includes a polynucleotide that is in a portion. A segment of a chromosome often contains a larger number of nucleotides than a portion (e.g., a segment sometimes includes a portion), and sometimes a segment of a chromosome contains a smaller number of nucleotides than a portion (e.g., a segment sometimes is within a portion).

*Filtering and/or Selecting Portions*

Portions sometimes are processed (e.g., normalized, filtered, selected, the like, or combinations thereof) according to one or more features, parameters, criteria and/or methods described herein or known in the art. Portions can be processed by any suitable method and according to any suitable parameter. Non-limiting examples of features and/or parameters that can be used to filter and/or select portions include counts, coverage, mappability, variability, a level of uncertainty, guanine-cytosine (GC) content, CCF fragment length and/or read length (e.g., a fragment length ratio (FLR), a fetal ratio statistic (FRS)), DNasel-sensitivity, methylation state, acetylation, histone distribution, chromatin structure, percent repeats, the like or combinations thereof. Portions can be filtered and/or selected according to any suitable feature or parameter that correlates with a feature or parameter listed or described herein. Portions can be filtered and/or selected according to features or parameters that are specific to a portion (e.g., as determined for a single portion according to multiple samples) and/or features or parameters that are specific to a sample (e.g., as determined for multiple portions within a sample). Portions may be filtered and/or removed according to relatively low mappability, relatively high variability, a high level of uncertainty, relatively long CCF fragment lengths (e.g., low FRS, low FLR), relatively large fraction of repetitive sequences, high GC content, low GC content, low counts, zero counts, high counts, the like, or combinations thereof. Portions (e.g., a subset of portions) may be selected according to suitable level of mappability, variability, level of uncertainty, fraction of repetitive sequences, count, GC content, the like, or combinations thereof. Portions (e.g., a subset of portions) may be selected according to relatively short CCF fragment lengths (e.g., high FRS, high FLR). Counts and/or reads mapped to portions are sometimes processed (e.g., normalized) prior to and/or after filtering or selecting portions (e.g., a subset of portions). Counts and/or reads mapped to portions may not be processed prior to and/or after filtering or selecting portions (e.g., a subset of portions).

Sequence reads from any suitable number of samples can be utilized to identify a subset of portions that meet one or more criteria, parameters and/or features described herein. Sequence reads from a group of samples from multiple pregnant females sometimes are utilized. One or more samples from each of the multiple pregnant females can be addressed (e.g., 1 to about 20 samples from each pregnant female (e.g., about 2, 3, 4, 5, 6, 7, 8, 9, 10, 11, 12, 13, 14, 15, 16, 17, 18 or 19 samples)), and a suitable number of pregnant females may be addressed (e.g., about 2 to about 10,000 pregnant females (e.g., about 10, 20, 30, 40, 50, 60, 70, 80, 90, 100, 150, 200, 250, 300, 350, 400, 500, 600, 700, 800, 900, 1000, 2000, 3000, 4000, 5000, 6000, 7000, 8000, 9000 pregnant females)). Sequence reads from the same test sample(s) from the same pregnant female may be mapped to portions in the reference genome and are used to generate the subset of portions.

It has been observed that circulating cell free nucleic acid fragments (CCF fragments) obtained from a pregnant female generally comprise nucleic acid fragments originating from fetal cells (i.e., fetal fragments) and nucleic acid fragments originating from maternal cells (i.e., maternal fragments). Sequence reads derived from CCF fragments originating from a fetus are referred to herein as "fetal reads." Sequence reads derived from CCF fragments originating from the genome of a pregnant female (e.g., a mother) bearing a fetus are referred to herein as "maternal reads." CCF fragments from which fetal reads are obtained are referred to herein as fetal templates and CCF fragments from which maternal reads are obtained are referred herein to as maternal templates.

It also has been observed that in CCF fragments, fetal fragments generally are relatively short (e.g., about 200 base pairs in length or less) and that maternal fragments include such relatively short fragments and relatively longer fragments. A subset of portions to which are mapped a significant amount of reads from relatively short fragments can be selected and/or identified. Without being limited by theory, it is expected that reads mapped to such portions are enriched for fetal reads, which can improve the accuracy of a fetal genetic analysis (e.g., detecting the presence or absence of a fetal copy number variation (e.g., fetal chromosome aneuploidy (e.g., T21, T18 and/or T13))).

A significant number of reads often are not considered, however, when a fetal genetic analysis is based on a subset of reads. Selection of a subset of reads mapped to a selected subset of portions, and removal of reads in non-selected portions, for a fetal genetic analysis can decrease the accuracy of the genetic analysis, due to increased variance for example. About 30% to about 70% (e.g., about 35%, 40%, 45%, 50%, 55%, 60%, or 65%) of sequencing reads obtained from a subject or sample map may be removed from consideration upon selection of a subset of portions for a fetal genetic analysis. About 30% to about 70% (e.g., about 35%, 40%, 45%, 50%, 55%, 60%, or 65%) of sequencing reads obtained from a subject or sample may map to a subset of portions utilized for a fetal genetic analysis.

Portions can be selected and/or filtered by any suitable method. Portions may be selected according to visual inspection of data, graphs, plots and/or charts. Portions may be selected and/or filtered (e.g., in part) by a system or a machine comprising one or more microprocessors and memory. Portions may be selected and/or filtered (e.g., in part) by a non-transitory computer-readable storage medium with an executable program stored thereon, where the program instructs a microprocessor to perform the selecting and/or filtering.

A subset of portions selected by methods described herein can be utilized for a fetal genetic analysis in different manners. Reads derived from a sample may be utilized in a mapping process using a pre-selected subset of portions described herein, and not using all or most of the portions in a reference genome. Those reads that map to the pre-selected subset of portions often are utilized in further steps of a fetal genetic analysis, and reads that do not map to the pre-selected subset of portions often are not utilized in further steps of a fetal genetic analysis (e.g., reads that do not map are removed or filtered).

Sequence reads derived from a sample may be mapped to all or most portions of a reference genome and a pre-selected subset of portions described herein are thereafter selected. Reads from a selected subset of portions often are utilized in further steps of a fetal genetic analysis. In the latter case, reads from portions not selected are often not utilized in further steps of a fetal genetic analysis (e.g., reads in the non-selected portions are removed or filtered).

*Counts*

Sequence reads that are mapped or partitioned based on a selected feature or variable may be quantified to determine the number of reads that are mapped to one or more portions (e.g., portion of a reference genome). The quantity of sequence reads that are mapped to a portion may be termed counts (e.g., a count). Often a count is associated with a portion. Counts for two or more portions (e.g., a set of portions) may be mathematically manipulated (e.g., averaged, added, normalized, the like or a combination thereof). A count may be determined from some or all of the sequence reads mapped to (i.e., associated with) a portion. A count may be determined from a pre-defined subset of mapped sequence reads. Pre-defined subsets of mapped sequence reads can be defined or selected utilizing any suitable feature or variable. Pre-defined subsets of mapped sequence reads may include from 1 to n sequence reads, where n represents a number equal to the sum of all sequence reads generated from a test subject or reference subject sample. A count may be a quantification of sequence reads not mapped to a portion.

A count may be derived from sequence reads that are processed or manipulated by a suitable method, operation or mathematical process known in the art. A count (e.g., counts) can be determined by a suitable method, operation or mathematical process. A count may be derived from sequence reads associated with a portion where some or all of the sequence reads are weighted, removed, filtered, normalized, adjusted, averaged, derived as a mean, added, or subtracted or processed by a combination thereof. A count may be derived from raw sequence reads and or filtered sequence reads. A count value may be determined by a mathematical process. A count value may be an average, mean or sum of sequence reads mapped to a portion. Often a count is a mean number of counts. A count may be associated with an uncertainty value.

Counts may be manipulated or transformed (e.g., normalized, combined, added, filtered, selected, averaged, derived as a mean, the like, or a combination thereof). Counts may be transformed to produce normalized counts. Counts can be processed (e.g., normalized) by a method known in the art and/or as described herein (e.g., portion-wise normalization, median count (median bin count, median portion count) normalization, normalization by GC content, linear and nonlinear least squares regression, LOESS (e.g., GC LOESS), LOWESS, PERUN, ChAI, principal component normalization, RM, GCRM, cQn and/or combinations thereof). Counts may be processed (e.g., normalized) by one or more of LOESS, median count (median bin count, median portion count) normalization, and principal component normalization. Counts may be processed (e.g., normalized) by LOESS followed by median count (median bin count, median portion count) normalization. Counts may be processed (e.g., normalized) by LOESS followed by median count (median bin count, median portion count) normalization followed by principal component normalization.

Counts (e.g., raw, filtered and/or normalized counts) can be processed and normalized to one or more levels. Levels and profiles are described in greater detail hereafter. Counts may be processed and/or normalized to a reference level. Reference levels are addressed later herein. Counts processed according to a level (e.g., processed counts) can be associated with an uncertainty value (e.g., a calculated variance, an error, standard deviation, Z-score, p-value, mean absolute deviation, etc.). An uncertainty value may define a range above and below a level. A value for deviation can be used in place of an uncertainty value, and non-limiting examples of measures of deviation include standard deviation, average absolute deviation, median absolute deviation, standard score (e.g., Z-score, normal score, standardized variable) and the like.

Counts are often obtained from a nucleic acid sample from a pregnant female bearing a fetus. Counts of nucleic acid sequence reads mapped to one or more portions often are counts representative of both the fetus and the mother of the fetus (e.g., a pregnant female subject). Some of the counts mapped to a portion may be from a fetal genome and some of the counts mapped to the same portion are from a maternal genome.

*Data Processing and Normalization*

Mapped sequence reads and/or unmapped sequence reads that have been counted are referred to herein as raw data, since the data represents unmanipulated counts (e.g., raw counts). Sequence read data in a data set can be processed further (e.g., mathematically and/or statistically manipulated) and/or displayed to facilitate providing an outcome. Data sets, including larger data sets, may benefit from pre-processing to facilitate further analysis. Pre-processing of data sets sometimes involves removal of redundant and/or uninformative portions or portions of a reference genome (e.g., portions of a reference genome with uninformative data, redundant mapped reads, portions with zero median counts, over represented or under represented sequences). Without being limited by theory, data processing and/or preprocessing may (i) remove noisy data, (ii) remove uninformative data, (iii) remove redundant data, (iv) reduce the complexity of larger data sets, and/or (v) facilitate transformation of the data from one form into one or more other forms. The terms "pre-processing" and "processing" when utilized with respect to data or data sets are collectively referred to herein as "processing". Processing may render data more amenable to further analysis, and can generate an outcome. One or more or all processing methods (e.g., normalization methods, portion filtering, mapping, validation, the like or combinations thereof) may be performed by a processor, a micro-processor, a computer, in conjunction with memory and/or by a microprocessor controlled apparatus.

The term "noisy data" as used herein refers to (a) data that has a significant variance between data points when analyzed or plotted, (b) data that has a significant standard deviation (e.g., greater than 3 standard deviations), (c) data that has a significant standard error of the mean, the like, and combinations of the foregoing. Noisy data sometimes occurs due to the quantity and/or quality of starting material (e.g., nucleic acid sample), and sometimes occurs as part of processes for preparing or replicating DNA used to generate sequence reads. Noise may result from certain sequences being over represented when prepared using PCR-based methods. Methods described herein can reduce or eliminate the contribution of noisy data, and therefore reduce the effect of noisy data on the provided outcome.

The terms "uninformative data", "uninformative portions of a reference genome", and "uninformative portions" as used herein refer to portions, or data derived therefrom, having a numerical value that is significantly different from a predetermined threshold value or falls outside a predetermined cutoff range of values. The terms "threshold" and "threshold value" herein refer to any number that is calculated using a qualifying data set and serves as a limit of diagnosis of a genetic variation (e.g. a copy number variation, an aneuploidy, a microduplication, a microdeletion, a chromosomal aberration, and the like). A threshold may exceeded by results obtained by methods described herein and a subject is diagnosed with a copy number variation (e.g. trisomy 21). A threshold value or range of values often may be calculated by mathematically and/or statistically manipulating sequence read data (e.g., from a reference and/or subject), and sequence read data manipulated to generate a threshold value or range of values may be sequence read data (e.g., from a reference and/or subject). An uncertainty value may be determined. An uncertainty value generally is a measure of variance or error and can be any suitable measure of variance or error. An uncertainty value may be a standard deviation, standard error, calculated variance, p-value, or mean absolute deviation (MAD). An uncertainty value may be calculated according to a formula described herein.

Any suitable procedure can be utilized for processing data sets described herein. Non-limiting examples of procedures suitable for use for processing data sets include filtering, normalizing, weighting, monitoring peak heights, monitoring peak areas, monitoring peak edges, determining area ratios, mathematical processing of data, statistical processing of data, application of statistical algorithms, analysis with fixed variables, analysis with optimized variables, plotting data to identify patterns or trends for additional processing, the like and combinations of the foregoing. Data sets may be processed based on various features (e.g., GC content, redundant mapped reads, centromere regions, telomere regions, the like and combinations thereof) and/or variables (e.g., fetal gender, maternal age, maternal ploidy, percent contribution of fetal nucleic acid, the like or combinations thereof). Processing data sets as described herein may reduce the complexity and/or dimensionality of large and/or complex data sets. A non-limiting example of a complex data set includes sequence read data generated from one or more test subjects and a plurality of reference subjects of different ages and ethnic backgrounds. Data sets may include from thousands to millions of sequence reads for each test and/or reference subject.

Data processing can be performed in any number of steps. For example, data may be processed using only a single processing procedure, and data may be processed using 1 or more, 5 or more, 10 or more or 20 or more processing steps (e.g., 1 or more processing steps, 2 or more processing steps, 3 or more processing steps, 4 or more processing steps, 5 or more processing steps, 6 or more processing steps, 7 or more processing steps, 8 or more processing steps, 9 or more processing steps, 10 or more processing steps, 11 or more processing steps, 12 or more processing steps, 13 or more processing steps, 14 or more processing steps, 15 or more processing steps, 16 or more processing steps, 17 or more processing steps, 18 or more processing steps, 19 or more processing steps, or 20 or more processing steps). Processing steps may be the same step repeated two or more times (e.g., filtering two or more times, normalizing two or more times), and processing steps may be two or more different processing steps (e.g., filtering, normalizing; normalizing, monitoring peak heights and edges; filtering, normalizing, normalizing to a reference, statistical manipulation to determine p-values, and the like), carried out simultaneously or sequentially. Any suitable number and/or combination of the same or different processing steps can be utilized to process sequence read data to facilitate providing an outcome. Processing data sets by the criteria described herein may reduce the complexity and/or dimensionality of a data set.

One or more processing steps may comprise one or more filtering steps. The term "filtering" as used herein refers to removing portions or portions of a reference genome from consideration. Portions of a reference genome can be selected for removal based on any suitable criteria, including but not limited to redundant data (e.g., redundant or overlapping mapped reads), non-informative data (e.g., portions of a reference genome with zero median counts), portions of a reference genome with over represented or under represented sequences, noisy data, the like, or combinations of the foregoing. A filtering process often involves removing one or more portions of a reference genome from consideration and subtracting the counts in the one or more portions of a reference genome selected for removal from the counted or summed counts for the portions of a reference genome, chromosome or chromosomes, or genome under consideration. Portions of a reference genome may be removed successively (e.g., one at a time to allow evaluation of the effect of removal of each individual portion), and all portions of a reference genome marked for removal may be removed at the same time. Portions of a reference genome characterized by a variance above or below a certain level may be removed, which sometimes is referred to herein as filtering "noisy" portions of a reference genome. A filtering process may comprises obtaining data points from a data set that deviate from the mean profile level of a portion, a chromosome, or segment of a chromosome by a predetermined multiple of the profile variance, and a filtering process may comprise removing data points from a data set that do not deviate from the mean profile level of a portion, a chromosome or segment of a chromosome by a predetermined multiple of the profile variance. A filtering process may be utilized to reduce the number of candidate portions of a reference genome analyzed for the presence or absence of a copy number variation. Reducing the number of candidate portions of a reference genome analyzed for the presence or absence of a copy number variation (e.g., micro-deletion, micro-duplication) often reduces the complexity and/or dimensionality of a data set, and sometimes increases the speed of searching for and/or identifying copy number variations and/or genetic aberrations by two or more orders of magnitude.

One or more processing steps may comprise one or more normalization steps. Normalization can be performed by a suitable method described herein or known in the art. Normalization may comprise adjusting values measured on different scales to a notionally common scale. Normalization comprises a sophisticated mathematical adjustment to bring probability distributions of adjusted values into alignment. Normalization may comprise aligning distributions to a normal distribution. Normalization may comprise mathematical adjustments that allow comparison of corresponding normalized values for different datasets in a way that eliminates the effects of certain gross influences (e.g., error and anomalies). Normalization may comprise scaling. Normalization sometimes comprises division of one or more data sets by a predetermined variable or formula. Normalization sometimes comprises subtraction of one or more data sets by a predetermined variable or formula. Non-limiting examples of normalization methods include portion-wise normalization, normalization by GC content, median count (median bin count, median portion count) normalization, linear and nonlinear least squares regression, LOESS, GC LOESS, LOWESS (locally weighted scatterplot smoothing), PERUN, ChAI, principal component normalization, repeat masking (RM), GC-normalization and repeat masking (GCRM), cQn and/or combinations thereof. The determination of a presence or absence of a copy number variation (e.g., an aneuploidy, a microduplication, a microdeletion) may utilize a normalization method (e.g., portion-wise normalization, normalization by GC content, median count (median bin count, median portion count) normalization, linear and nonlinear least squares regression, LOESS, GC LOESS, LOWESS (locally weighted scatterplot smoothing), PERUN, ChAI, principal component normalization, repeat masking (RM), GC-normalization and repeat masking (GCRM), cQn, a normalization method known in the art and/or a combination thereof). The determination of a presence or absence of a copy number variation (e.g., an aneuploidy, a microduplication, a microdeletion) may utilize one or more of LOESS, median count (median bin count, median portion count) normalization, and principal component normalization. The determination of a presence or absence of a copy number variation may utilize LOESS followed by median count (median bin count, median portion count) normalization. The determination of a presence or absence of a copy number variation may utilize LOESS followed by median count (median bin count, median portion count) normalization followed by principal component normalization. Aspects of certain normalization processes (e.g., ChAI normalization, principal component normalization, PERUN normalization) are described, for example, in patent application no. PCT/US2014/039389 filed on May 23, 2014 and published as WO 2014/190286 on November 27, 2014; and patent application no. PCT/US2014/058885 filed on October 2, 2014 and published as WO 2015/051163 on April 9, 2015.

Any suitable number of normalizations can be used. Data sets may be normalized 1 or more, 5 or more, 10 or more or even 20 or more times. Data sets can be normalized to values (e.g., normalizing value) representative of any suitable feature or variable (e.g., sample data, reference data, or both). Non-limiting examples of types of data normalizations that can be used include normalizing raw count data for one or more selected test or reference portions to the total number of counts mapped to the chromosome or the entire genome on which the selected portion or sections are mapped; normalizing raw count data for one or more selected portions to a median reference count for one or more portions or the chromosome on which a selected portion or segments is mapped; normalizing raw count data to previously normalized data or derivatives thereof; and normalizing previously normalized data to one or more other predetermined normalization variables. Normalizing a data set sometimes has the effect of isolating statistical error, depending on the feature or property selected as the predetermined normalization variable. Normalizing a data set sometimes also allows comparison of data characteristics of data having different scales, by bringing the data to a common scale (e.g., predetermined normalization variable). One or more normalizations to a statistically derived value may be utilized to minimize data differences and diminish the importance of outlying data. Normalizing portions, or portions of a reference genome, with respect to a normalizing value sometimes is referred to as "portion-wise normalization".

A processing step comprising normalization may include normalizing to a static window, and a processing step comprising normalization may include normalizing to a moving or sliding window. The term "window" as used herein refers to one or more portions chosen for analysis, and sometimes used as a reference for comparison (e.g., used for normalization and/or other mathematical or statistical manipulation). The term "normalizing to a static window" as used herein refers to a normalization process using one or more portions selected for comparison between a test subject and reference subject data set. The selected portions may be utilized to generate a profile. A static window generally includes a predetermined set of portions that do not change during manipulations and/or analysis. The terms "normalizing to a moving window" and "normalizing to a sliding window" as used herein refer to normalizations performed to portions localized to the genomic region (e.g., immediate genetic surrounding, adjacent portion or sections, and the like) of a selected test portion, where one or more selected test portions are normalized to portions immediately surrounding the selected test portion. The selected portions may be utilized to generate a profile. A sliding or moving window normalization often includes repeatedly moving or sliding to an adjacent test portion, and normalizing the newly selected test portion to portions immediately surrounding or adjacent to the newly selected test portion, where adjacent windows have one or more portions in common. A plurality of selected test portions and/or chromosomes may be analyzed by a sliding window process.

Normalizing to a sliding or moving window may generate one or more values, where each value represents normalization to a different set of reference portions selected from different regions of a genome (e.g., chromosome). The one or more values generated may cumulative sums (e.g., a numerical estimate of the integral of the normalized count profile over the selected portion, domain (e.g., part of chromosome), or chromosome). The values generated by the sliding or moving window process can be used to generate a profile and facilitate arriving at an outcome. Cumulative sums of one or more portions may be displayed as a function of genomic position. Moving or sliding window analysis sometimes is used to analyze a genome for the presence or absence of micro-deletions and/or micro-insertions. Displaying cumulative sums of one or more portions may be used to identify the presence or absence of regions of copy number variation (e.g., micro-deletions, micro-duplications). Moving or sliding window analysis may be used to identify genomic regions containing micro-deletions and, moving or sliding window analysis may be used to identify genomic regions containing micro-duplications.

Described in greater detail hereafter are certain examples of normalization processes that can be utilized, such as LOESS, PERUN, ChAI and principal component normalization methods, for example.

A processing step may comprise a weighting. The terms "weighted", "weighting" or "weight function" or grammatical derivatives or equivalents thereof, as used herein, refer to a mathematical manipulation of a portion or all of a data set sometimes utilized to alter the influence of certain data set features or variables with respect to other data set features or variables (e.g., increase or decrease the significance and/or contribution of data contained in one or more portions or portions of a reference genome, based on the quality or usefulness of the data in the selected portion or portions of a reference genome). A weighting function may be used to increase the influence of data with a relatively small measurement variance, and/or to decrease the influence of data with a relatively large measurement variance. For example, portions of a reference genome with under represented or low quality sequence data can be "down weighted" to minimize the influence on a data set, whereas selected portions of a reference genome can be "up weighted" to increase the influence on a data set. A non-limiting example of a weighting function is $[1 / (standard\ deviation)^2]$. A weighting step sometimes is performed in a manner substantially similar to a normalizing step. A data set may be divided by a predetermined variable (e.g., weighting variable). A predetermined variable (e.g., minimized target function, Phi) often is selected to weigh different parts of a data set differently (e.g., increase the influence of certain data types while decreasing the influence of other data types).

A processing step can comprise one or more mathematical and/or statistical manipulations. Any suitable mathematical and/or statistical manipulation, alone or in combination, may be used to analyze and/or manipulate a data set described herein. Any suitable number of mathematical and/or statistical manipulations can be used. A data set can be mathematically and/or statistically manipulated 1 or more, 5 or more, 10 or more or 20 or more times. Non-limiting examples of mathematical and statistical manipulations that can be used include addition, subtraction, multiplication, division, algebraic functions, least squares estimators, curve fitting, differential equations, rational polynomials, double polynomials, orthogonal polynomials, z-scores, p-values, chi values, phi values, analysis of peak levels, determination of peak edge locations, calculation of peak area ratios, analysis of median chromosomal level, calculation of mean absolute deviation, sum of squared residuals, mean, standard deviation, standard error, the like or combinations thereof. A mathematical and/or statistical manipulation can be performed on all or a portion of sequence read data, or processed products thereof. Non-limiting examples of data set variables or features that can be statistically manipulated include raw counts, filtered counts, normalized counts, peak heights, peak widths, peak areas, peak edges, lateral tolerances, P-values, median levels, mean levels, count distribution within a genomic region, relative representation of nucleic acid species, the like or combinations thereof.

A processing step may comprise the use of one or more statistical algorithms. Any suitable statistical algorithm, alone or in combination, may be used to analyze and/or manipulate a data set described herein. Any suitable number of statistical algorithms can be used. A data set may be analyzed using 1 or more, 5 or more, 10 or more or 20 or more statistical algorithms. Non-limiting examples of statistical algorithms suitable for use with methods described herein include decision trees, counternulls, multiple comparisons, omnibus test, Behrens-Fisher problem, bootstrapping, Fisher's method for combining independent tests of significance, null hypothesis, type I error, type II error, exact test, one-sample Z test, two-sample Z test, one-sample t-test, paired t-test, two-sample pooled t-test having equal variances, two-sample unpooled t-test having unequal variances, one-proportion z-test, two-proportion z-test pooled, two-proportion z-test unpooled, one-sample chi-square test, two-sample F test for equality of variances, confidence interval, credible interval, significance, meta analysis, simple linear regression, robust linear regression, the like or combinations of the foregoing. Non-limiting examples of data set variables or features that can be analyzed using statistical algorithms include raw counts, filtered counts, normalized counts, peak heights, peak widths, peak edges, lateral tolerances, P-values, median levels, mean levels, count distribution within a genomic region, relative representation of nucleic acid species, the like or combinations thereof.

A data set may be analyzed by utilizing multiple (e.g., 2 or more) statistical algorithms (e.g., least squares regression, principle component analysis, linear discriminant analysis, quadratic discriminant analysis, bagging, neural networks, support vector machine models, random forests, classification tree models, K-nearest neighbors, logistic regression and/or loss smoothing) and/or mathematical and/or statistical manipulations (e.g., referred to herein as manipulations). The use of multiple manipulations may generate an N-dimensional space that can be used to provide an outcome. Analysis of a data set by utilizing multiple manipulations may reduce the complexity and/or dimensionality of the data set. For example, the use of multiple manipulations on a reference data set can generate an N-dimensional space (e.g., probability plot) that can be used to represent the presence or absence of a copy number variation, depending on the status of the reference samples (e.g., positive or negative for a selected copy number variation). Analysis of test samples using a substantially similar set of manipulations can be used to generate an N-dimensional point for each of the test samples. The complexity and/or dimensionality of a test subject data set sometimes is reduced to a single value or N-dimensional point that can be readily compared to the N-dimensional space generated from the reference data. Test sample data that fall within the N-dimensional space populated by the reference subject data are indicative of a genetic status substantially similar to that of the reference subjects. Test sample data that fall outside of the N-dimensional space populated by the reference subject data are indicative of a genetic status substantially dissimilar to that of the reference subjects. References may be euploid or do not otherwise have a copy number variation or medical condition.

After data sets have been counted, optionally filtered and normalized, the processed data sets may be further manipulated by one or more filtering and/or normalizing procedures. A data set that has been further manipulated by one or more filtering and/or normalizing procedures may be used to generate a profile. The one or more filtering and/or normalizing procedures sometimes can reduce data set complexity and/or dimensionality. An outcome can be provided based on a data set of reduced complexity and/or dimensionality.

Portions may be filtered according to a measure of error (e.g., standard deviation, standard error, calculated variance, p-value, mean absolute error (MAE), average absolute deviation and/or mean absolute deviation (MAD). A measure of error may refersto count variability. Portions may be filtered according to count variability. Count variability may be a measure of error determined for counts mapped to a portion (i.e., portion) of a reference genome for multiple samples (e.g., multiple sample obtained from multiple subjects, e.g., 50 or more, 100 or more, 500 or more 1000 or more, 5000 or more or 10,000 or more subjects). Portions with a count variability above a pre-determined upper range may be filtered (e.g., excluded from consideration). In A pre-determined upper range may be a MAD value equal to or greater than about 50, about 52, about 54, about 56, about 58, about 60, about 62, about 64, about 66, about 68, about 70, about 72, about 74 or equal to or greater than about 76. In A count variability below a pre-determined lower range may be filtered (e.g., excluded from consideration). A pre-determined lower range may be a MAD value equal to or less than about 40, about 35, about 30, about 25, about 20, about 15, about 10, about 5, about 1, or equal to or less than about 0. portions with a count variability outside a pre-determined range may be filtered (e.g., excluded from consideration). A pre-determined range may be a MAD value greater than zero and less than about 76, less than about 74, less than about 73, less than about 72, less than about 71, less than about 70, less than about 69, less than about 68, less than about 67, less than about 66, less than about 65, less than about 64, less than about 62, less than about 60, less than about 58, less than about 56, less than about 54, less than about 52 or less than about 50. A pre-determined range may be a MAD value greater than zero and less than about 67.7. Portions with a count variability within a pre-determined range may be selected (e.g., used for determining the presence or absence of a copy number variation).

The count variability of portions may represent a distribution (e.g., a normal distribution). Portions may be selected within a quantile of the distribution. Portions within a quantile equal to or less than about 99.9%, 99.8%, 99.7%, 99.6%, 99.5%, 99.4%, 99.3%, 99.2%, 99.1%, 99.0%, 98.9%, 98.8%, 98.7%, 98.6%, 98.5%, 98.4%, 98.3%, 98.2%, 98.1%, 98.0%, 97%, 96%, 95%, 94%, 93%, 92%, 91%, 90%, 85%, 80%, or equal to or less than a quantile of about 75% for the distribution may be selected. Portions within a 99% quantile of the distribution of count variability may be selected. Portions with a MAD > 0 and a MAD < 67.725 a within the 99% quantile may be selected, resulting in the identification of a set of stable portions of a reference genome.

Non-limiting examples of portion filtering with respect to PERUN, for example, is provided herein and in International Patent Application no. PCT/US12/59123 (WO2013/052913). Portions may be filtered based on, or based on part on, a measure of error. A measure of error comprising absolute values of deviation, such as an R-factor, may be used for portion removal or weighting. An R-factor may be defined as the sum of the absolute deviations of the predicted count values from the actual measurements divided by the predicted count values from the actual measurements (e.g., Equation C on page 228 of patent application no. PCT/US2012/059123 filed on October 5, 2012 and published as WO2013/052913 on April 11, 2013). While a measure of error comprising absolute values of deviation may be used, a suitable measure of error may be alternatively employed. A measure of error not comprising absolute values of deviation, such as a dispersion based on squares, may be utilized. Portions may be filtered or weighted according to a measure of mappability (e.g., a mappability score). A portion sometimes is filtered or weighted according to a relatively low number of sequence reads mapped to the portion (e.g., 0, 1, 2, 3, 4, 5 reads mapped to the portion). A portion sometimes is filtered or weighted according to fraction or percent of repetitive sequences. Portions may be filtered or weighted according to one or more of (i) a measure of mappability, (ii) measure of error (e.g., R-factor) and (iii) fraction or percent of repetitive sequences. Portions can be filtered or weighted according to the type of analysis being performed. For example, for chromosome 13, 18 and/or 21 aneuploidy analysis, sex chromosomes may be filtered, and only autosomes, or a subset of autosomes, may be analyzed.

In particular, the following filtering process may be employed. The same set of portions (e.g., portions of a reference genome) within a given chromosome (e.g., chromosome 21) are selected and the number of reads in affected and unaffected samples are compared. The gap relates trisomy 21 and euploid samples and it involves a set of portions covering most of chromosome 21. The set of portions is the same between euploid and T21 samples. The distinction between a set of portions and a single section is not crucial, as a portion can be defined. The same genomic region is compared in different patients. This process can be utilized for a trisomy analysis, such as for T13 or T18 in addition to, or instead of, T21.

After data sets have been counted, optionally filtered and normalized, the processed data sets may be manipulated by weighting. One or more portions may be selected for weighting to reduce the influence of data (e.g., noisy data, uninformative data) contained in the selected portions, and one or more portions may be selected for weighting to enhance or augment the influence of data (e.g., data with small measured variance) contained in the selected portions. A data set may be weighted utilizing a single weighting function that decreases the influence of data with large variances and increases the influence of data with small variances. A weighting function sometimes is used to reduce the influence of data with large variances and augment the influence of data with small variances (e.g., $[1/(standard deviation)^2]$). A profile plot of processed data further manipulated by weighting may be generated to facilitate classification and/or providing an outcome. An outcome can be provided based on a profile plot of weighted data

Filtering or weighting of portions can be performed at one or more suitable points in an analysis. For example, portions may be filtered or weighted before or after sequence reads are mapped to portions of a reference genome. Portions may be filtered or weighted before or after an experimental bias for individual genome portions is determined. Portions may be filtered or weighted before or after genomic section levels are calculated.

After data sets have been counted, optionally filtered, normalized, and optionally weighted, the processed data sets may be manipulated by one or more mathematical and/or statistical (e.g., statistical functions or statistical algorithm) manipulations. Processed data sets may be further manipulated by calculating Z-scores for one or more selected portions, chromosomes, or portions of chromosomes. Processed data sets may be further manipulated by calculating P-values. Mathematical and/or statistical manipulations may include one or more assumptions pertaining to ploidy and/or fetal fraction. A profile plot of processed data further manipulated by one or more statistical and/or mathematical manipulations may be generated to facilitate classification and/or providing an outcome. An outcome can be provided based on a profile plot of statistically and/or mathematically manipulated data. An outcome provided based on a profile plot of statistically and/or mathematically manipulated data often includes one or more assumptions pertaining to ploidy and/or fetal fraction.

Multiple manipulations may be performed on processed data sets to generate an N-dimensional space and/or N-dimensional point, after data sets have been counted, optionally filtered and normalized. An outcome can be provided based on a profile plot of data sets analyzed in N-dimensions.

Data sets may be processed utilizing one or more peak level analysis, peak width analysis, peak edge location analysis, peak lateral tolerances, the like, derivations thereof, or combinations of the foregoing, as part of or after data sets have processed and/or manipulated. A profile plot of data processed utilizing one or more peak level analysis, peak width analysis, peak edge location analysis, peak lateral tolerances, the like, derivations thereof, or combinations of the foregoing may be generated to facilitate classification and/or providing an outcome. An outcome can be provided based on a profile plot of data that has been processed utilizing one or more peak level analysis, peak width analysis, peak edge location analysis, peak lateral tolerances, the like, derivations thereof, or combinations of the foregoing.

The use of one or more reference samples that are substantially free of a copy number variation in question may be used to generate a reference median count profile, which may result in a predetermined value representative of the absence of the copy number variation, and often deviates from a predetermined value in areas corresponding to the genomic location in which the copy number variation is located in the test subject, if the test subject possessed the copy number variation. In test subjects at risk for, or suffering from a medical condition associated with a copy number variation, the numerical value for the selected portion or sections is expected to vary significantly from the predetermined value for non-affected genomic locations. The use of one or more reference samples known to carry the copy number variation in question may be used to generate a reference median count profile, which may result in a predetermined value representative of the presence of the copy number variation, and often deviates from a predetermined value in areas corresponding to the genomic location in which a test subject does not carry the copy number variation. In test subjects not at risk for, or suffering from a medical condition associated with a copy number variation, the numerical value for the selected portion or sections is expected to vary significantly from the predetermined value for affected genomic locations.

Analysis and processing of data may include the use of one or more assumptions. A suitable number or type of assumptions can be utilized to analyze or process a data set. Non-limiting examples of assumptions that can be used for data processing and/or analysis include maternal ploidy, fetal contribution, prevalence of certain sequences in a reference population, ethnic background, prevalence of a selected medical condition in related family members, parallelism between raw count profiles from different patients and/or runs after GC-normalization and repeat masking (e.g., GCRM), identical matches represent PCR artifacts (e.g., identical base position), assumptions inherent in a fetal quantifier assay (e.g., FQA), assumptions regarding twins (e.g., if 2 twins and only 1 is affected the effective fetal fraction is only 50% of the total measured fetal fraction (similarly for triplets, quadruplets and the like)), fetal cell free DNA (e.g., cfDNA) uniformly covers the entire genome, the like and combinations thereof.

In those instances where the quality and/or depth of mapped sequence reads does not permit an outcome prediction of the presence or absence of a copy number variation at a desired confidence level (e.g., 95% or higher confidence level), based on the normalized count profiles, one or more additional mathematical manipulation algorithms and/or statistical prediction algorithms, can be utilized to generate additional numerical values useful for data analysis and/or providing an outcome. The term "normalized count profile" as used herein refers to a profile generated using normalized counts. Examples of methods that can be used to generate normalized counts and normalized count profiles are described herein. As noted, mapped sequence reads that have been counted can be normalized with respect to test sample counts or reference sample counts. A normalized count profile may be presented as a plot.

*LOESS Normalization*

LOESS is a regression modeling method known in the art that combines multiple regression models in a k-nearest-neighbor-based meta-model. LOESS is sometimes referred to as a locally weighted polynomial regression. GC LOESS may apply an LOESS model to the relationship between fragment count (e.g., sequence reads, counts) and GC composition for portions of a reference genome. Plotting a smooth curve through a set of data points using LOESS is sometimes called an LOESS curve, particularly when each smoothed value is given by a weighted quadratic least squares regression over the span of values of the y-axis scattergram criterion variable. For each point in a data set, the LOESS method fits a low-degree polynomial to a subset of the data, with explanatory variable values near the point whose response is being estimated. The polynomial is fitted using weighted least squares, giving more weight to points near the point whose response is being estimated and less weight to points further away. The value of the regression function for a point is then obtained by evaluating the local polynomial using the explanatory variable values for that data point. The LOESS fit is sometimes considered complete after regression function values have been computed for each of the data points. Many of the details of this method, such as the degree of the polynomial model and the weights, are flexible.

*PERUN Normalization*

A normalization methodology for reducing error associated with nucleic acid indicators is referred to herein as Parameterized Error Removal and Unbiased Normalization (PERUN) described herein and in international patent application no. PCT/US12/59123 (WO2013/052913). PERUN methodology can be applied to a variety of nucleic acid indicators (e.g., nucleic acid sequence reads) for the purpose of reducing effects of error that confound predictions based on such indicators.

For example, PERUN methodology can be applied to nucleic acid sequence reads from a sample and reduce the effects of error that can impair genomic section level determinations. Such an application is useful for using nucleic acid sequence reads to determine the presence or absence of a copy number variation in a subject manifested as a varying level of a nucleotide sequence (e.g., a portion, a genomic section level). Non-limiting examples of variations in portions are chromosome aneuploidies (e.g., trisomy 21, trisomy 18, trisomy 13) and presence or absence of a sex chromosome (e.g., XX in females versus XY in males). A trisomy of an autosome (e.g., a chromosome other than a sex chromosome) can be referred to as an affected autosome. Other non-limiting examples of variations in genomic section levels include microdeletions, microinsertions, duplications and mosaicism.

In certain applications, PERUN methodology can reduce experimental bias by normalizing nucleic acid reads mapped to particular portions of a reference genome, the latter of which are referred to as portions and sometimes as portions of a reference genome. In such applications, PERUN methodology generally normalizes counts of nucleic acid reads at particular portions of a reference genome across a number of samples in three dimensions. A detailed description of PERUN and applications thereof is provided in international patent application no. PCT/US12/59123 (WO2013/052913) and U.S. patent application publication no. US20130085681.

PERUN methodology may include calculating a genomic section level for portions of a reference genome from (a) sequence read counts mapped to a portion of a reference genome for a test sample, (b) experimental bias (e.g., GC bias) for the test sample, and (c) one or more fit parameters (e.g., estimates of fit) for a fitted relationship between (i) experimental bias for a portion of a reference genome to which sequence reads are mapped and (ii) counts of sequence reads mapped to the portion. Experimental bias for each of the portions of a reference genome can be determined across multiple samples according to a fitted relationship for each sample between (i) the counts of sequence reads mapped to each of the portions of a reference genome, and (ii) a mapping feature for each of the portions of a reference genome. This fitted relationship for each sample can be assembled for multiple samples in three dimensions. The assembly may be ordered according to the experimental bias, although PERUN methodology may be practiced without ordering the assembly according to the experimental bias. The fitted relationship for each sample and the fitted relationship for each portion of the reference genome can be fitted independently to a linear function or non-linear function by a suitable fitting process known in the art.

A relationship may be a geometric and/or graphical relationship. A relationship may be a mathematical relationship. A relationship may be plotted. A relationship may be a linear relationship. A relationship may be a non-linear relationship. A relationship may be a regression (e.g., a regression line). A regression can be a linear regression or a non-linear regression. A relationship can be expressed by a mathematical equation. Often a relationship is defined, in part, by one or more constants. A relationship can be generated by a method known in the art. A relationship in two dimensions may be generated for one or more samples, and a variable probative of error, or possibly probative of error, can be selected for one or more of the dimensions. A relationship can be generated, for example, using graphing software known in the art that plots a graph using values of two or more variables provided by a user. A relationship can be fitted using a method known in the art (e.g., graphing software). Certain relationships can be fitted by linear regression, and the linear regression can generate a slope value and intercept value. Certain relationships sometimes are not linear and can be fitted by a non-linear function, such as a parabolic, hyperbolic or exponential function (e.g., a quadratic function), for example.

In PERUN methodology, one or more of the fitted relationships may be linear. For an analysis of cell-free circulating nucleic acid from pregnant females, where the experimental bias is GC bias and the mapping feature is GC content, a fitted relationship for a sample between the (i) the counts of sequence reads mapped to each portion, and (ii) GC content for each of the portions of a reference genome, can be linear. For the latter fitted relationship, the slope pertains to GC bias, and a GC bias coefficient can be determined for each sample when the fitted relationships are assembled across multiple samples. In such aspects, the fitted relationship for multiple samples and a portion between (i) GC bias coefficient for the portion, and (ii) counts of sequence reads mapped to portion, also can be linear. An intercept and slope can be obtained from the latter fitted relationship. In such applications, the slope addresses sample-specific bias based on GC-content and the intercept addresses a portion-specific attenuation pattern common to all samples. PERUN methodology can significantly reduce such sample-specific bias and portion-specific attenuation when calculating genomic section levels for providing an outcome (e.g., presence or absence of copy number variation; determination of fetal sex).

PERUN normalization may make use of fitting to a linear function and is described by Equation I, Equation II or a derivation thereof.

Equation I:

$$M = LI + GS \qquad\qquad (I)$$

Equation II:

$$L = (M - GS)/I \qquad\qquad (II)$$

$L$ may be a PERUN normalized level or profile. Also $L$ may be the desired output from the PERUN normalization procedure. $L$ may be portion specific. $L$ may be determined according to multiple portions of a reference genome and represents a PERUN normalized level of a genome, chromosome, portions or segment thereof. The level $L$ is often used for further analyses (e.g., to determine Z-values, maternal deletions/duplications, fetal microdeletions/ microduplications, fetal gender, sex aneuploidies, and so on). The method of normalizing according to Equation II is named Parameterized Error Removal and Unbiased Normalization (PERUN).

$G$ may be a GC bias coefficient measured using a linear model, LOESS, or any equivalent approach. $G$ may be a slope. The GC bias coefficient G may be evaluated as the slope of the regression for counts $M$ (e.g., raw counts) for portion $i$ and the GC content of portion $i$ determined from a reference genome. $G$ may represent secondary information, extracted from M and determined according to a relationship. $G$ may represent a relationship for a set of portion-specific counts and a set of portion-specific GC content values for a sample (e.g., a test sample). Portion-specific GC content may be derived from a reference genome. Portion-specific GC content may be derived from observed or measured GC content (e.g., measured from the sample). A GC bias coefficient often is determined for each sample in a group of samples and generally is determined for a test sample. A GC bias coefficient often is sample specific. A GC bias coefficient may be a constant. A GC bias coefficient, once derived for a sample, may not change.

I may be an intercept and S may be a slope derived from a linear relationship. In The relationship from which I and S are derived may be different than the relationship from which G is derived. The relationship from which I and S are derived may be fixed for a given experimental setup. In I and S may be derived from a linear relationship according to counts (e.g., raw counts) and a GC bias coefficient according to multiple samples. I and S may be derived independently of the test sample. I and S may be derived from multiple samples. I and S often are portion specific. I and S may be determined with the assumption that L = 1 for all portions of a reference genome in euploid samples. A linear relationship may be determined for euploid samples and $I$ and $S$ values specific for a selected portion (assuming $L$ = 1) may be determined. The same procedure may be applied to all portions of a reference genome in a human genome and a set of intercepts $I$ and slopes $S$ may be determined for every portion.

A cross-validation approach may be applied. Cross-validation, sometimes is referred to as rotation estimation. A cross-validation approach may be applied to assess how accurately a predictive model (e.g., such as PERUN) will perform in practice using a test sample. One round of cross-validation may comprise partitioning a sample of data into complementary subsets, performing a cross validation analysis on one subset (e.g., sometimes referred to as a training set), and validating the analysis using another subset (e.g., sometimes called a validation set or test set). Multiple rounds of cross-validation may be performed using different partitions and/or different subsets). Non-limiting examples of cross-validation approaches include leave-one-out, sliding edges, K-fold, 2-fold, repeat random sub-sampling, the like or combinations thereof. A cross-validation randomly may select a work set containing 90% of a set of samples comprising known euploid fetuses and uses that subset to train a model. The random selection may be repeated 100 times, yielding a set of 100 slopes and 100 intercepts for every portion.

The value of $M$ may bea measured value derived from a test sample. $M$ may be measured raw counts for a portion. Where the values $I$ and $S$ are available for a portion, measurement $M$ may be determined from a test sample and may be used to determine the PERUN normalized level L for a genome, chromosome, segment or portion thereof according to Equation II.

Thus, application of PERUN methodology to sequence reads across multiple samples in parallel can significantly reduce error caused by (i) sample-specific experimental bias (e.g., GC bias) and (ii) portion-specific attenuation common to samples. Other methods in which each of these two sources of error are addressed separately or serially often are not able to reduce these as effectively as PERUN methodology. Without being limited by theory, it is expected that PERUN methodology reduces error more effectively in part because its generally additive processes do not magnify spread as much as generally multiplicative processes utilized in other normalization approaches (e.g., GC-LOESS).

Additional normalization and statistical techniques may be utilized in combination with PERUN methodology. An additional process can be applied before, after and/or during employment of PERUN methodology. Non-limiting examples of processes that can be used in combination with PERUN methodology are described hereafter.

A secondary normalization or adjustment of a genomic section level for GC content may be utilized in conjunction with PERUN methodology. A suitable GC content adjustment or normalization procedure can be utilized (e.g., GC-LOESS, GCRM). A particular sample may be identified for application of an additional GC normalization process. For example, application of PERUN methodology can determine GC bias for each sample, and a sample associated with a GC bias above a certain threshold can be selected for an additional GC normalization process. In such aspects, a predetermined threshold level can be used to select such samples for additional GC normalization.

A portion filtering or weighting process may be utilized in conjunction with PERUN methodology. A suitable portion filtering or weighting process can be utilized, non-limiting examples are described herein, in international patent application no. PCT/US12/59123 (WO2013/052913) and U.S. patent application publication no. US20130085681. A normalization technique that reduces error associated with maternal insertions, duplications and/or deletions (e.g., maternal and/or fetal copy number variations), may be utilized in conjunction with PERUN methodology.

Genomic section levels calculated by PERUN methodology can be utilized directly for providing an outcome. Genomic section levels may be utilized directly to provide an outcome for samples in which fetal fraction is about 2% to about 6% or greater (e.g., fetal fraction of about 4% or greater). Genomic section levels calculated by PERUN methodology sometimes are further processed for the provision of an outcome. Calculated genomic section levels may be standardized. The sum, mean or median of calculated genomic section levels for a test portion (e.g., chromosome 21) can be divided by the sum, mean or median of calculated genomic section levels for portions other than the test portion (e.g., autosomes other than chromosome 21), to generate an experimental genomic section level. An experimental genomic section level or a raw genomic section level can be used as part of a standardization analysis, such as calculation of a Z-score. A Z-score can be generated for a sample by subtracting an expected genomic section level from an experimental genomic section level or raw genomic section level and the resulting value may be divided by a standard deviation for the samples. Resulting Z-scores may be distributed for different samples and analyzed, or can be related to other variables, such as fetal fraction and others, and analyzed, to provide an outcome.

As noted herein, PERUN methodology is not limited to normalization according to GC bias and GC content per se, and can be used to reduce error associated with other sources of error. A non-limiting example of a source of non-GC content bias is mappability. When normalization parameters other than GC bias and content are addressed, one or more of the fitted relationships may be non-linear (e.g., hyperbolic, exponential). Where experimental bias is determined from a non-linear relationship, for example, an experimental bias curvature estimation may be analyzed.

PERUN methodology can be applied to a variety of nucleic acid indicators. Non-limiting examples of nucleic acid indicators are nucleic acid sequence reads and nucleic acid levels at a particular location on a microarray. Non-limiting examples of sequence reads include those obtained from cell-free circulating DNA, cell-free circulating RNA, cellular DNA and cellular RNA. PERUN methodology can be applied to sequence reads mapped to suitable reference sequences, such as genomic reference DNA, cellular reference RNA (e.g., transcriptome), and portions thereof (e.g., part(s) of a genomic complement of DNA or RNA transcriptome, part(s) of a chromosome).

Thus, in cellular nucleic acid (e.g., DNA or RNA) may serve as a nucleic acid indicator. Cellular nucleic acid reads mapped to reference genome portions can be normalized using PERUN methodology. Cellular nucleic acid bound to a particular protein sometimes are referred to chromatin immunoprecipitation (ChIP) processes. ChIP-enriched nucleic acid is a nucleic acid in association with cellular protein, such as DNA or RNA for example. Reads of ChIP-enriched nucleic acid can be obtained using technology known in the art. Reads of ChIP-enriched nucleic acid can be mapped to one or more portions of a reference genome, and results can be normalized using PERUN methodology for providing an outcome.

Cellular RNA may serve as nucleic acid indicators. Cellular RNA reads can be mapped to reference RNA portions and normalized using PERUN methodology for providing an outcome. Known sequences for cellular RNA, referred to as a transcriptome, or a segment thereof, can be used as a reference to which RNA reads from a sample can be mapped. Reads of sample RNA can be obtained using technology known in the art. Results of RNA reads mapped to a reference can be normalized using PERUN methodology for providing an outcome.

Microarray nucleic acid levels may serve as nucleic acid indicators. Nucleic acid levels across samples for a particular address, or hybridizing nucleic acid, on an array can be analyzed using PERUN methodology, thereby normalizing nucleic acid indicators provided by microarray analysis. In this manner, a particular address or hybridizing nucleic acid on a microarray is analogous to a portion for mapped nucleic acid sequence reads, and PERUN methodology can be used to normalize microarray data to provide an improved outcome.

*ChAl Normalization*

Another normalization methodology that can be used to reduce error associated with nucleic acid indicators is referred to herein as ChAl and often makes use of a principal component analysis. A principal component analysis may include (a) filtering, according to a read density distribution, portions of a reference genome, thereby providing a read density profile for a test sample comprising read densities of filtered portions, where the read densities comprise sequence reads of circulating cell-free nucleic acid from a test sample from a pregnant female, and the read density distribution is determined for read densities of portions for multiple samples, (b) adjusting the read density profile for the test sample according to one or more principal components, which principal components are obtained from a set of known euploid samples by a principal component analysis, thereby providing a test sample profile comprising adjusted read densities, and (c) comparing the test sample profile to a reference profile, thereby providing a comparison. A principal component analysis may include (d) determining the presence or absence of a copy number variation for the test sample according to the comparison. Certain aspects of ChAl normalization is described, for example, in patent application no. PCT/US2014/058885 filed on October 2, 2014 and published as WO 2015/051163 on April 9, 2015.

*Filtering Portions*

One or more portions (e.g., portions of a genome) may be removed from consideration by a filtering process. One or more portions may be filtered (e.g., subjected to a filtering process) thereby providing filtered portions. A filtering process may remove certain portions and retains portions (e.g., a subset of portions). Following a filtering process, retained portions are often referred to herein as filtered portions. Portions of a reference genome may be filtered. Portions of a reference genome that are removed by a filtering process may not be included in a determination of the presence or absence of a copy number variation (e.g., a chromosome aneuploidy, microduplication, microdeletion). Portions associated with read densities (e.g., where a read density is for a portion) may be removed by a filtering process and read densities associated with removed portions are not included in a determination of the presence or absence of a copy number variation (e.g., a chromosome aneuploidy, microduplication, microdeletion). A read density profile may comprise and/or consist of read densities of filtered portions. Portions can be selected, filtered, and/or removed from consideration using any suitable criteria and/or method known in the art or described herein. Non-limiting examples of criteria used for filtering portions include redundant data (e.g., redundant or overlapping mapped reads), non-informative data (e.g., portions of a reference genome with zero mapped counts), portions of a reference genome with over represented or under represented sequences, GC content, noisy data, mappability, counts, count variability, read density, variability of read density, a measure of uncertainty, a repeatability measure, the like, or combinations of the foregoing. Portions are sometimes filtered according to a distribution of counts and/or a distribution of read densities. Portions are filtered according to a distribution of counts and/or read densities where the counts and/or read densities may be obtained from one or more reference samples. One or more reference samples is sometimes referred to herein as a training set. Portions may be filtered according to a distribution of counts and/or read densities where the counts and/or read densities may be obtained from one or more test samples. Portions may be filtered according to a measure of uncertainty for a read density distribution. Portions that demonstrate a large deviation in read densities may be removed by a filtering process. For example, a distribution of read densities (e.g., a distribution of average mean, or median read densities) can be determined, where each read density in the distribution maps to the same portion. A measure of uncertainty (e.g., a MAD) can be determined by comparing a distribution of read densities for multiple samples where each portion of a genome is associated with measure of uncertainty. According to the foregoing example, portions can be filtered according to a measure of uncertainty (e.g., a standard deviation (SD), a MAD) associated with each portion and a predetermined threshold. A predetermined threshold is indicated by the dashed vertical lines enclosing a range of acceptable MAD values. In certain instances, portions comprising MAD values within the acceptable range are retained and portions comprising MAD values outside of the acceptable range are removed from consideration by a filtering process. According to the foregoing example, portions comprising read densities values (e.g., median, average or mean read densities) outside a pre-determined measure of uncertainty may often be removed from consideration by a filtering process. Portions comprising read densities values (e.g., median, average or mean read densities) outside an inter-quartile range of a distribution may be removed from consideration by a filtering process. Portions comprising read densities values outside more than 2 times, 3 times, 4 times or 5 times an inter-quartile range of a distribution may be removed from consideration by a filtering process. Portions comprising read densities values outside more than 2 sigma, 3 sigma, 4 sigma, 5 sigma, 6 sigma, 7 sigma or 8 sigma (e.g., where sigma is a range defined by a standard deviation) may be removed from consideration by a filtering process.

A system may comprise a filtering module. A filtering module often accepts, retrieves and/or stores portions (e.g., portions of pre-determined sizes and/or overlap, portion locations within a reference genome) and read densities associated with portions, often from another suitable module. Selected portions (e.g., filtered portions) may be provided by a filtering module. A filtering module may be required to provide filtered portions and/or to remove portions from consideration. A filtering module may remove read densities from consideration where read densities are associated with removed portions. A filtering module often provides selected portions (e.g., filtered portions) to another suitable module.

*Bias Estimates*

Sequencing technologies can be vulnerable to multiple sources of bias. Sometimes sequencing bias is a local bias (e.g., a local genome bias). Local bias often is manifested at the level of a sequence read. A local genome bias can be any suitable local bias. Non-limiting examples of a local bias include sequence bias (e.g., GC bias, AT bias, and the like), bias correlated with DNase I sensitivity, entropy, repetitive sequence bias, chromatin structure bias, polymerase error-rate bias, palindrome bias, inverted repeat bias, PCR related bias, the like or combinations thereof. The source of a local bias may not be determined or known.

A local genome bias estimate may be determined. A local genome bias estimate is sometimes referred to herein as a local genome bias estimation. A local genome bias estimate can be determined for a reference genome, a segment or a portion thereof. A local genome bias estimate may be determined for one or more sequence reads (e.g., some or all sequence reads of a sample). A local genome bias estimate is often determined for a sequence read according to a local genome bias estimation for a corresponding location and/or position of a reference (e.g., a reference genome). A local genome bias estimate may comprise a quantitative measure of bias of a sequence (e.g., a sequence read, a sequence of a reference genome). A local genome bias estimation can be determined by a suitable method or mathematical process. A local genome bias estimate may be determined by a suitable distribution and/or a suitable distribution function (e.g., a PDF). A local genome bias estimate may comprise a quantitative representation of a PDF. A local genome bias estimate (e.g., a probability density estimation (PDE), a kernel density estimation) may be determined by a probability density function (e.g., a PDF, e.g., a kernel density function) of a local bias content. A density estimation may comprise a kernel density estimation. A local genome bias estimate is sometimes expressed as an average, mean, or median of a distribution. Sometimes a local genome bias estimate is expressed as a sum or an integral (e.g., an area under a curve (AUC) of a suitable distribution.

A PDF (e.g., a kernel density function, e.g., an Epanechnikov kernel density function) often comprises a bandwidth variable (e.g., a bandwidth). A bandwidth variable often defines the size and/or length of a window from which a probability density estimate (PDE) is derived when using a PDF. A window from which a PDE is derived often comprises a defined length of polynucleotides. A window from which a PDE may be derived is a portion. A portion (e.g., a portion size, a portion length) is often determined according to a bandwidth variable. A bandwidth variable determines the length or size of the window used to determine a local genome bias estimate; a length of a polynucleotide segment (e.g., a contiguous segment of nucleotide bases) from which a local genome bias estimate is determined. A PDE (e.g., read density, local genome bias estimate (e.g., a GC density)) can be determined using any suitable bandwidth, non-limiting examples of which include a bandwidth of about 5 bases to about 100,000 bases, about 5 bases to about 50,000 bases, about 5 bases to about 25,000 bases, about 5 bases to about 10,000 bases, about 5 bases to about 5,000 bases, about 5 bases to about 2,500 bases, about 5 bases to about 1000 bases, about 5 bases to about 500 bases, about 5 bases to about 250 bases, about 20 bases to about 250 bases, or the like. A local genome bias estimate (e.g., a GC density) may be determined using a bandwidth of about 400 bases or less, about 350 bases or less, about 300 bases or less, about 250 bases or less, about 225 bases or less, about 200 bases or less, about 175 bases or less, about 150 bases or less, about 125 bases or less, about 100 bases or less, about 75 bases or less, about 50 bases or less or about 25 bases or less. A local genome bias estimate (e.g., a GC density) may be determined using a bandwidth determined according to an average, mean, median, or maximum read length of sequence reads obtained for a given subject and/or sample. Sometimes a local genome bias estimate (e.g., a GC density) is determined using a bandwidth about equal to an average, mean, median, or maximum read length of sequence reads obtained for a given subject and/or sample. A local genome bias estimate (e.g., a GC density) may be determined using a bandwidth of about 250, 240, 230, 220, 210, 200, 190, 180, 160, 150, 140, 130, 120, 110, 100, 90, 80, 70, 60, 50, 40, 30, 20 or about 10 bases.

A local genome bias estimate can be determined at a single base resolution, although local genome bias estimates (e.g., local GC content) can be determined at a lower resolution. A local genome bias estimate may be determined for a local bias content. A local genome bias estimate (e.g., as determined using a PDF) often is determined using a window. A local genome bias estimate may comprise use of a window comprising a pre-selected number of bases. Sometimes a window comprises a segment of contiguous bases. Sometimes a window comprises one or more portions of non-contiguous bases. Sometimes a window comprises one or more portions (e.g., portions of a genome). A window size or length is often determined by a bandwidth and according to a PDF. A window may be about 10 or more, 8 or more, 7 or more, 6 or more, 5 or more, 4 or more, 3 or more, or about 2 or more times the length of a bandwidth. A window is sometimes twice the length of a selected bandwidth when a PDF (e.g., a kernel density function) is used to determine a density estimate. A window may comprise any suitable number of bases. A window may comprise about 5 bases to about 100,000 bases, about 5 bases to about 50,000 bases, about 5 bases to about 25,000 bases, about 5 bases to about 10,000 bases, about 5 bases to about 5,000 bases, about 5 bases to about 2,500 bases, about 5 bases to about 1000 bases, about 5 bases to about 500 bases, about 5 bases to about 250 bases, or about 20 bases to about 250 bases. A genome, or segments thereof, may be partitioned into a plurality of windows. Windows encompassing regions of a genome may or may not overlap. Windows may be positioned at equal distances from each other. Windows may be positioned at different distances from each other. A genome, or segment thereof, may be partitioned into a plurality of sliding windows, where a window is slid incrementally across a genome, or segment thereof, where each window at each increment comprises a local genome bias estimate (e.g., a local GC density). A window can be slid across a genome at any suitable increment, according to any numerical pattern or according to any athematic defined sequence. For a local genome bias estimate determination, a window may be slid across a genome, or a segment thereof, at an increment of about 10,000 bp or more about 5,000 bp or more, about 2,500 bp or more, about 1,000 bp or more, about 750 bp or more, about 500 bp or more, about 400 bases or more, about 250 bp or more, about 100 bp or more, about 50 bp or more, or about 25 bp or more. For a local genome bias estimate determination, a window may be slid across a genome, or a segment thereof, at an increment of about 25, 24, 23, 22, 21, 20, 19, 18, 17, 16, 15, 14, 13, 12, 11, 10, 9, 8, 7, 6, 5, 4, 3, 2, or about 1 bp. For example, for a local genome bias estimate determination, a window may comprise about 400 bp (e.g., a bandwidth of 200 bp) and may be slid across a genome in increments of 1 bp. A local genome bias estimate may be determined for each base in a genome, or segment thereof, using a kernel density function and a bandwidth of about 200 bp.

A local genome bias estimate may be a local GC content and/or a representation of local GC content. The term "local" as used herein (e.g., as used to describe a local bias, local bias estimate, local bias content, local genome bias, local GC content, and the like) refers to a polynucleotide segment of 10,000 bp or less. The term "local" may refer to a polynucleotide segment of 5000 bp or less, 4000 bp or less, 3000 bp or less, 2000 bp or less, 1000 bp or less, 500 bp or less, 250 bp or less, 200 bp or less, 175 bp or less, 150 bp or less, 100 bp or less, 75 bp or less, or 50 bp or less. A local GC content is often a representation (e.g., a mathematical, a quantitative representation) of GC content for a local segment of a genome, sequence read, sequence read assembly (e.g., a contig, a profile, and the like). For example, a local GC content can be a local GC bias estimate or a GC density.

One or more GC densities are often determined for polynucleotides of a reference or sample (e.g., a test sample). A GC density may be a representation (e.g., a mathematical, a quantitative representation) of local GC content (e.g., for a polynucleotide segment of 5000 bp or less). A GC density may be a local genome bias estimate. A GC density can be determined using a suitable process described herein and/or known in the art. A GC density can be determined using a suitable PDF (e.g., a kernel density function (e.g., an Epanechnikov kernel density function). A GC density may be a PDE (e.g., a kernel density estimation). A GC density may be defined by the presence or absence of one or more guanine (G) and/or cytosine (C) nucleotides. Inversely, a GC density may be defined by the presence or absence of one or more a adenine (A) and/or thymidine (T) nucleotides. GC densities for local GC content may be normalized according to GC densities determined for an entire genome, or segment thereof (e.g., autosomes, set of chromosomes, single chromosome, a gene). One or more GC densities can be determined for polynucleotides of a sample (e.g., a test sample) or a reference sample. A GC density often is determined for a reference genome. A GC density may be determined for a sequence read according to a reference genome. A GC density of a read is often determined according to a GC density determined for a corresponding location and/or position of a reference genome to which a read is mapped. A GC density determined for a location on a reference genome may be assigned and/or provided for a read, where the read, or a segment thereof, maps to the same location on the reference genome. Any suitable method can be used to determine a location of a mapped read on a reference genome for the purpose of generating a GC density for a read. A median position of a mapped read may determine a location on a reference genome from which a GC density for the read is determined. For example, where the median position of a read maps to Chromosome 12 at base number x of a reference genome, the GC density of the read is often provided as the GC density determined by a kernel density estimation for a position located on Chromosome 12 at or near base number x of the reference genome. A GC density may be determined for some or all base positions of a read according to a reference genome. Sometimes a GC density of a read comprises an average, sum, median or integral of two or more GC densities determined for a plurality of base positions on a reference genome.

A local genome bias estimation (e.g., a GC density) is quantitated and/or may be provided a value. A local genome bias estimation (e.g., a GC density) is sometimes expressed as an average, mean, and/or median. A local genome bias estimation (e.g., a GC density) is sometimes expressed as a maximum peak height of a PDE. Sometimes a local genome bias estimation (e.g., a GC density) is expressed as a sum or an integral (e.g., an area under a curve (AUC)) of a suitable PDE. A GC density may comprise a kernel weight. A GC density of a read may comprise a value about equal to an average, mean, sum, median, maximum peak height or integral of a kernel weight.

*Bias Frequencies*

Bias frequencies are sometimes determined according to one or more local genome bias estimates (e.g., GC densities). A bias frequency is sometimes a count or sum of the number of occurrences of a local genome bias estimate for a sample, reference (e.g., a reference genome, a reference sequence) or part thereof. A bias frequency is sometimes a count or sum of the number of occurrences of a local genome bias estimate (e.g., each local genome bias estimate) for a sample, reference, or part thereof. A bias frequency may be a GC density frequency. A GC density frequency is often determined according to one or more GC densities. For example, a GC density frequency may represent the number of times a GC density of value x is represented over an entire genome, or a segment thereof. A bias frequency is often a distribution of local genome bias estimates, where the number of occurrences of each local genome bias estimate is represented as a bias frequency. Bias frequencies are sometimes mathematically manipulated and/or normalized. Bias frequencies can be mathematically manipulated and/or normalized by a suitable method. Bias frequencies may be normalized according to a representation (e.g., a fraction, a percentage) of each local genome bias estimate for a sample, reference or part thereof (e.g., autosomes, a subset of chromosomes, a single chromosome, or reads thereof). Bias frequencies can be determined for some or all local genome bias estimates of a sample or reference. Bias frequencies may be determined for local genome bias estimates for some or all sequence reads of a test sample.

A system may comprise a bias density module 6. A bias density module can accept, retrieve and/or store mapped sequence reads 5 and reference sequences 2 in any suitable format and generate local genome bias estimates, local genome bias distributions, bias frequencies, GC densities, GC density distributions and/or GC density frequencies (collectively represented by box 7). A bias density module may transfer data and/or information (e.g., 7) to another suitable module (e.g., a relationship module 8).

*Bias Relationships*

Oone or more relationships may be generated between local genome bias estimates and bias frequencies. The term "relationship" as use herein refers to a mathematical and/or a graphical relationship between two or more variables or values. A relationship can be generated by a suitable mathematical and/or graphical process. Non-limiting examples of a relationship include a mathematical and/or graphical representation of a function, a correlation, a distribution, a linear or non-linear equation, a line, a regression, a fitted regression, the like or a combination thereof. Sometimes a relationship comprises a fitted relationship. A fitted relationship may comprise a fitted regression. Sometimes a relationship comprises two or more variables or values that are weighted. A relationship may comprise a fitted regression where one or more variables or values of the relationship a weighted. Sometimes a regression is fitted in a weighted fashion. Sometimes a regression is fitted without weighting. Generating a relationship may comprise plotting or graphing.

A suitable relationship may be determined between local genome bias estimates and bias frequencies. Generating a relationship between (i) local genome bias estimates and (ii) bias frequencies for a sample may provide a sample bias relationship. Generating a relationship between (i) local genome bias estimates and (ii) bias frequencies for a reference may provide a reference bias relationship. A relationship may be generated between GC densities and GC density frequencies. Generating a relationship between (i) GC densities and (ii) GC density frequencies for a sample may provide a sample GC density relationship. Generating a relationship between (i) GC densities and (ii) GC density frequencies for a reference may provide a reference GC density relationship. Where local genome bias estimates are GC densities, a sample bias relationship is a sample GC density relationship and a reference bias relationship may be a reference GC density relationship. GC densities of a reference GC density relationship and/or a sample GC density relationship are often representations (e.g., mathematical or quantitative representation) of local GC content. A relationship between local genome bias estimates and bias frequencies may comprise a distribution. A relationship between local genome bias estimates and bias frequencies may comprise a fitted relationship (e.g., a fitted regression). A relationship between local genome bias estimates and bias frequencies may comprise a fitted linear or non-linear regression (e.g., a polynomial regression). A relationship between local genome bias estimates and bias frequencies may comprise a weighted relationship where local genome bias estimates and/or bias frequencies may be weighted by a suitable process. A weighted fitted relationship (e.g., a weighted fitting) may be obtained by a process comprising a quantile regression, parameterized distributions or an empirical distribution with interpolation. A relationship between local genome bias estimates and bias frequencies for a test sample, a reference or part thereof, may comprise a polynomial regression where local genome bias estimates may be weighted. A weighed fitted model comprises weighting values of a distribution. Values of a distribution can be weighted by a suitable process. Values located near tails of a distribution may be provided less weight than values closer to the median of the distribution. For example, for a distribution between local genome bias estimates (e.g., GC densities) and bias frequencies (e.g., GC density frequencies), a weight is determined according to the bias frequency for a given local genome bias estimate, where local genome bias estimates comprising bias frequencies closer to the mean of a distribution are provided greater weight than local genome bias estimates comprising bias frequencies further from the mean.

A system may comprise a relationship module 8. A relationship module can generate relationships as well as functions, coefficients, constants and variables that define a relationship. A relationship module can accept, store and/or retrieve data and/or information (e.g., 7) from a suitable module (e.g., a bias density module 6) and generate a relationship.

A relationship module often generates and compares distributions of local genome bias estimates. A relationship module can compare data sets and sometimes generate regressions and/or fitted relationships. A relationship module may compare one or more distributions (e.g., distributions of local genome bias estimates of samples and/or references) and may provide weighting factors and/or weighting assignments 9 for counts of sequence reads to another suitable module (e.g., a bias correction module). Sometimes a relationship module provides normalized counts of sequence reads directly to a distribution module 21 where the counts are normalized according to a relationship and/or a comparison.

*Generating a comparison and use thereof*

A process for reducing local bias in sequence reads may comprise normalizing counts of sequence reads. Counts of sequence reads are often normalized according to a comparison of a test sample to a reference. For example, sometimes counts of sequence reads are normalized by comparing local genome bias estimates of sequence reads of a test sample to local genome bias estimates of a reference (e.g., a reference genome, or part thereof). Counts of sequence reads may be normalized by comparing bias frequencies of local genome bias estimates of a test sample to bias frequencies of local genome bias estimates of a reference. Counts of sequence reads may be normalized by comparing a sample bias relationship and a reference bias relationship, thereby generating a comparison.

Counts of sequence reads are often normalized according to a comparison of two or more relationships. Two or more relationships may be compared thereby providing a comparison that is used for reducing local bias in sequence reads (e.g., normalizing counts). Two or more relationships can be compared by a suitable method. A comparison may comprise adding, subtracting, multiplying and/or dividing a first relationship from a second relationship. Comparing two or more relationships may comprise a use of a suitable linear regression and/or a non-linear regression. Comparing two or more relationships may comprise a suitable polynomial regression (e.g., a 3rd order polynomial regression). A comparison may comprise adding, subtracting, multiplying and/or dividing a first regression from a second regression. Two or more relationships may be compared by a process comprising an inferential framework of multiple regressions. Two or more relationships may be compared by a process comprising a suitable multivariate analysis. Two or more relationships may be compared by a process comprising a basis function (e.g., a blending function, e.g., polynomial bases, Fourier bases, or the like), splines, a radial basis function and/or wavelets.

A distribution of local genome bias estimates comprising bias frequencies for a test sample and a reference may be compared by a process comprising a polynomial regression where local genome bias estimates are weighted. A polynomial regression may be generated between (i) ratios, each of which ratios comprises bias frequencies of local genome bias estimates of a reference and bias frequencies of local genome bias estimates of a sample and (ii) local genome bias estimates. A polynomial regression may be generated between (i) a ratio of bias frequencies of local genome bias estimates of a reference to bias frequencies of local genome bias estimates of a sample and (ii) local genome bias estimates. A comparison of a distribution of local genome bias estimates for reads of a test sample and a reference may comprise determining a log ratio (e.g., a log2 ratio) of bias frequencies of local genome bias estimates for the reference and the sample. A comparison of a distribution of local genome bias estimates may comprise dividing a log ratio (e.g., a log2 ratio) of bias frequencies of local genome bias estimates for the reference by a log ratio (e.g., a log2 ratio) of bias frequencies of local genome bias estimates for the sample.

Normalizing counts according to a comparison typically adjusts some counts and not others. Normalizing counts sometimes adjusts all counts and sometimes does not adjust any counts of sequence reads. A count for a sequence read sometimes is normalized by a process that comprises determining a weighting factor and sometimes the process does not include directly generating and utilizing a weighting factor. Normalizing counts according to a comparison sometimes comprises determining a weighting factor for each count of a sequence read. A weighting factor is often specific to a sequence read and is applied to a count of a specific sequence read. A weighting factor is often determined according to a comparison of two or more bias relationships (e.g., a sample bias relationship compared to a reference bias relationship). A normalized count is often determined by adjusting a count value according to a weighting factor. Adjusting a count according to a weighting factor sometimes includes adding, subtracting, multiplying and/or dividing a count for a sequence read by a weighting factor. A weighting factor and/or a normalized count sometimes are determined from a regression (e.g., a regression line). A normalized count is sometimes obtained directly from a regression line (e.g., a fitted regression line) resulting from a comparison between bias frequencies of local genome bias estimates of a reference (e.g., a reference genome) and a test sample. Each count of a read of a sample may be provided a normalized count value according to a comparison of (i) bias frequencies of a local genome bias estimates of reads compared to (ii) bias frequencies of a local genome bias estimates of a reference. Counts of sequence reads obtained for a sample may be normalized and bias in the sequence reads is reduced.

Sometimes a system comprises a bias correction module 10. Functions of a bias correction module may be performed by a relationship modeling module 8. A bias correction module can accept, retrieve, and/or store mapped sequence reads and weighting factors (e.g., 9) from a suitable module (e.g., a relationship module 8, a compression module 4). A bias correction module may provide a count to mapped reads. A bias correction module may apply weighting assignments and/or bias correction factors to counts of sequence reads thereby providing normalized and/or adjusted counts. A bias correction module often provides normalized counts to another suitable module (e.g., a distribution module 21).

Normalizing counts may comprise factoring one or more features in addition to GC density, and normalizing counts of the sequence reads. Normalizing counts may comprise factoring one or more different local genome bias estimates, and normalizing counts of the sequence reads. Counts of sequence reads may be weighted according to a weighting determined according to one or more features (e.g., one or more biases). Counts may be normalized according to one or more combined weights. Sometimes factoring one or more features and/or normalizing counts according to one or more combined weights may be by a process comprising use of a multivariate model. Any suitable multivariate model can be used to normalize counts. Non-limiting examples of a multivariate model include a multivariate linear regression, multivariate quantile regression, a multivariate interpolation of empirical data, a non-linear multivariate model, the like, or a combination thereof.

A system may comprise a multivariate correction module 13. A multivariate correction module can perform functions of a bias density module 6, relationship module 8 and/or a bias correction module 10 multiple times thereby adjusting counts for multiple biases. A multivariate correction module may comprise one or more bias density modules 6, relationship modules 8 and/or bias correction modules 10. Sometimes a multivariate correction module provides normalized counts 11 to another suitable module (e.g., a distribution module 21).

*Weighted portions*

Portions may be weighted. One or more portions may be weighted thereby providing weighted portions. Weighting portions sometimes removes portion dependencies. Portions can be weighted by a suitable process. One or more portions may weighted by an eigen function (e.g., an eigenfunction). An eigen function may comprise replacing portions with orthogonal eigen-portions. A system may comprise a portion weighting module 42. A weighting module may accept, retrieve and/or store read densities, read density profiles, and/or adjusted read density profiles. Weighted portions may be provided by a portion weighting module. A weighting module may be required to weight portions. A weighting module can weight portions by one or more weighting methods known in the art or described herein. A weighting module often provides weighted portions to another suitable module (e.g., a scoring module 46, a PCA statistics module 33, a profile generation module 26 and the like).

*Principal component analysis*

A read density profile (e.g., a read density profile of a test sample) may be adjusted according to a principal component analysis (PCA). A read density profile of one or more reference samples and/or a read density profile of a test subject can be adjusted according to a PCA. Removing bias from a read density profile by a PCA related process is sometimes referred to herein as adjusting a profile. A PCA can be performed by a suitable PCA method, or a variation thereof. Non-limiting examples of a PCA method include a canonical correlation analysis (CCA), a Karhunen-Loeve transform (KLT), a Hotelling transform, a proper orthogonal decomposition (POD), a singular value decomposition (SVD) of X, an eigenvalue decomposition (EVD) of XTX, a factor analysis, an Eckart-Young theorem, a Schmidt-Mirsky theorem, empirical orthogonal functions (EOF), an empirical eigenfunction decomposition, an empirical component analysis, quasiharmonic modes, a spectral decomposition, an empirical modal analysis, the like, variations or combinations thereof. A PCA often identifies one or more biases in a read density profile. A bias identified by a PCA is sometimes referred to herein as a principal component. One or more biases may be removed by adjusting a read density profile according to one or more principal component using a suitable method. A read density profile can be adjusted by adding, subtracting, multiplying and/or dividing one or more principal components from a read density profile. One or more biases may be removed from a read density profile by subtracting one or more principal components from a read density profile. Although bias in a read density profile is often identified and/or quantitated by a PCA of a profile, principal components are often subtracted from a profile at the level of read densities. A PCA often identifies one or more principal components. A PCA may identify a 1st, 2nd, 3rd, 4th, 5th, 6th, 7th, 8th, 9th, and a 10th or more principal components. 1, 2, 3, 4, 5, 6, 7, 8, 9, 10 or more principal components may be used to adjust a profile. Often, principal components are used to adjust a profile in the order of there appearance in a PCA. For example, where three principal components are subtracted from a read density profile, a 1st, 2nd and 3rd principal component are used. Sometimes a bias identified by a principal component comprises a feature of a profile that is not used to adjust a profile. For example, a PCA may identify a copy number variation (e.g., an aneuploidy, microduplication, microdeletion, deletion, translocation, insertion) and/or a gender difference as a principal component. Thus, one or more principal components are not used to adjust a profile. For example, sometimes a 1st, 2nd and 4th principal component are used to adjust a profile where a 3rd principal component is not used to adjust a profile. A principal component can be obtained from a PCA using any suitable sample or reference. Components may be obtained from a test sample (e.g., a test subject). Principal components may be obtained from one or more references (e.g., reference samples, reference sequences, a reference set). In certain instances, a PCA is performed on a median read density profile obtained from a training set comprising multiple samples resulting in the identification of a $1^{st}$ principal component and a second principal component. Principal components may be obtained from a set of subjects known to be devoid of a copy number variation in question. Principal components may be obtained from a set of known euploids. Principal component are often identified according to a PCA performed using one or more read density profiles of a reference (e.g., a training set). One or more principal components obtained from a reference are often subtracted from a read density profile of a test subject thereby providing an adjusted profile.

A system may comprise a PCA statistics module 33. A PCA statistics module can accept and/or retrieve read density profiles from another suitable module (e.g., a profile generation module 26). A PCA is often performed by a PCA statistics module. A PCA statistics module often accepts, retrieves and/or stores read density profiles and processes read density profiles from a reference set 32, training set 30 and/or from one or more test subjects 28. A PCA statistics module can generate and/or provide principal components and/or adjust read density profiles according to one or more principal components. Adjusted read density profiles (e.g., 40, 38) are often provided by a PCA statistics module. A PCA statistics module can provide and/or transfer adjusted read density profiles (e.g., 38, 40) to another suitable module (e.g., a portion weighting module 42, a scoring module 46). A PCA statistics module may provide a gender call 36. A gender call is sometimes a determination of fetal gender determined according to a PCA and/or according to one or more principal components. A PCA statistics module may comprise some, all or a modification of the R code shown below. An R code for computing principal components generally starts with cleaning the data (e.g., subtracting median, filtering portions, and trimming extreme values):

```
#Clean the data outliers for PCA
dclean <- (dat - m)[mask,]
for (j in 1:ncol(dclean))
{
q <- quantile(dclean[,j],c(.25,.75))
qmin <- q[1] - 4*(q[2]-q[1])
qmax <- q[2] + 4*(q[2]-q[1])
dclean[dclean[,j] < qmin,j] <- qmin
dclean[dclean[,j] > qmax,j] <- qmax
}
```

Then the principal components are computed:

```
#Compute principal components
pc <- prcomp(dclean)$x
```

Finally, each sample's PCA-adjusted profile can be computed with:

```
#Compute residuals
mm <- model.matrix(~pc[,1:numpc])
for (j in 1:ncol(dclean))
dclean[,j] <- dclean[,j] - predict(lm(dclean[,j]~mm))
```

*Comparing Profiles*

Determining an outcome may comprise a comparison. In A read density profile, or a portion thereof, may be utilized to provide an outcome. Determining an outcome (e.g., a determination of the presence or absence of a copy number variation) may comprise a comparison of two or more read density profiles. Comparing read density profiles often comprises comparing read density profiles generated for a selected segment of a genome. For example, a test profile is often compared to a reference profile where the test and reference profiles were determined for a segment of a genome (e.g., a reference genome) that is substantially the same segment. Comparing read density profiles sometimes comprises comparing two or more subsets of portions of a read density profile. A subset of portions of a read density profile may represent a segment of a genome (e.g., a chromosome, or segment thereof). A read density profile can comprise any amount of subsets of portions. Sometimes a read density profile comprises two or more, three or more, four or more, or five or more subsets. A read density profile may comprise two subsets of portions where each portion represents segments of a reference genome that are adjacent. A test profile may be compared to a reference profile where the test profile and reference profile both comprise a first subset of portions and a second subset of portions where the first and second subsets represent different segments of a genome. Some subsets of portions of a read density profile may comprise copy number variations and other subsets of portions are sometimes substantially free of copy number variations. Sometimes all subsets of portions of a profile (e.g., a test profile) are substantially free of a copy number variation. Sometimes all subsets of portions of a profile (e.g., a test profile) comprise a copy number variation. A test profile may comprise a first subset of portions that comprise a genetic variation and a second subset of portions that are substantially free of a copy number variation.

Methods described herein may comprise performing a comparison (e.g., comparing a test profile to a reference profile). Two or more data sets, two or more relationships and/or two or more profiles can be compared by a suitable method. Non-limiting examples of statistical methods suitable for comparing data sets, relationships and/or profiles include Behrens-Fisher approach, bootstrapping, Fisher's method for combining independent tests of significance, Neyman-Pearson testing, confirmatory data analysis, exploratory data analysis, exact test, F-test, Z-test, T-test, calculating and/or comparing a measure of uncertainty, a null hypothesis, counternulls and the like, a chi-square test, omnibus test, calculating and/or comparing level of significance (e.g., statistical significance), a meta analysis, a multivariate analysis, a regression, simple linear regression, robust linear regression, the like or combinations of the foregoing. Comparing two or more data sets, relationships and/or profiles may comprise determining and/or comparing a measure of uncertainty. A "measure of uncertainty" as used herein refers to a measure of significance (e.g., statistical significance), a measure of error, a measure of variance, a measure of confidence, the like or a combination thereof. A measure of uncertainty can be a value (e.g., a threshold) or a range of values (e.g., an interval, a confidence interval, a Bayesian confidence interval, a threshold range). Non-limiting examples of a measure of uncertainty include p-values, a suitable measure of deviation (e.g., standard deviation, sigma, absolute deviation, mean absolute deviation, the like), a suitable measure of error (e.g., standard error, mean squared error, root mean squared error, the like), a suitable measure of variance, a suitable standard score (e.g., standard deviations, cumulative percentages, percentile equivalents, Z-scores, T-scores, R-scores, standard nine (stanine), percent in stanine, the like), the like or combinations thereof. Determining the level of significance may comprise determining a measure of uncertainty (e.g., a p-value). Two or more data sets, relationships and/or profiles may be analyzed and/or compared by utilizing multiple (e.g., 2 or more) statistical methods (e.g., least squares regression, principle component analysis, linear discriminant analysis, quadratic discriminant analysis, bagging, neural networks, support vector machine models, random forests, classification tree models, K-nearest neighbors, logistic regression and/or loss smoothing) and/or any suitable mathematical and/or statistical manipulations (e.g., referred to herein as manipulations).

Comparing two or more read density profiles may comprise determining and/or comparing a measure of uncertainty for two or more read density profiles. Read density profiles and/or associated measures of uncertainty are sometimes compared to facilitate interpretation of mathematical and/or statistical manipulations of a data set and/or to provide an outcome. A read density profile generated for a test subject sometimes is compared to a read density profile generated for one or more references (e.g., reference samples, reference subjects, and the like). An outcome may be provided by comparing a read density profile from a test subject to a read density profile from a reference for a chromosome, portions or segments thereof, where a reference read density profile is obtained from a set of reference subjects known not to possess a copy number variation (e.g., a reference). An outcome may be provided by comparing a read density profile from a test subject to a read density profile from a reference for a chromosome, portions or segments thereof, where a reference read density profile is obtained from a set of reference subjects known to possess a specific copy number variation (e.g., a chromosome aneuploidy, a trisomy, a microduplication, a microdeletion).

A read density profile of a test subject may be compared to a predetermined value representative of the absence of a copy number variation, and sometimes deviates from a predetermined value at one or more genomic locations (e.g., portions) corresponding to a genomic location in which a copy number variation is located. For example, in test subjects (e.g., subjects at risk for, or suffering from a medical condition associated with a copy number variation), read density profiles are expected to differ significantly from read density profiles of a reference (e.g., a reference sequence, reference subject, reference set) for selected portions when a test subject comprises a copy number variation in question. Read density profiles of a test subject are often substantially the same as read density profiles of a reference (e.g., a reference sequence, reference subject, reference set) for selected portions when a test subject does not comprise a copy number variation in question. Read density profiles are often compared to a predetermined threshold and/or threshold range. The term "threshold" as used herein refers to any number that is calculated using a qualifying data set and serves as a limit of diagnosis of a copy number variation (e.g., a copy number variation, an aneuploidy, a chromosomal aberration, a microduplication, a microdeletion, and the like). A threshold may be exceeded by results obtained by methods described herein and a subject is diagnosed with a copy number variation (e.g., a trisomy). A threshold value or range of values often may be calculated by mathematically and/or statistically manipulating sequence read data (e.g., from a reference and/or subject). A predetermined threshold or threshold range of values indicative of the presence or absence of a copy number variation can vary while still providing an outcome useful for determining the presence or absence of a copy number variation. A read density profile comprising normalized read densities and/or normalized counts may be generated to facilitate classification and/or providing an outcome. An outcome can be provided based on a plot of a read density profile comprising normalized counts (e.g., using a plot of such a read density profile).

A system may comprise a scoring module 46. A scoring module can accept, retrieve and/or store read density profiles (e.g., adjusted, normalized read density profiles) from another suitable module (e.g., a profile generation module 26, a PCA statistics module 33, a portion weighting module 42, and the like). A scoring module can accept, retrieve, store and/or compare two or more read density profiles (e.g., test profiles, reference profiles, training sets, test subjects). A scoring module can often provide a score (e.g., a plot, profile statistics, a comparison (e.g., a difference between two or more profiles), a Z-score, a measure of uncertainty, a call zone, a sample call 50 (e.g., a determination of the presence or absence of a copy number variation), and/or an outcome). A scoring module can provide a score to an end user and/or to another suitable module (e.g., a display, printer, the like). A scoring module may comprises some, all or a modification of the R code shown below which comprises an R function for computing Chi-square statistics for a specific test (e.g., High-chr21 counts).

The three parameters are:

    x = sample read data (portion x sample)
    m = median values for portions
    y = test vector (Ex. False for all portions except True for chr21)

```
getChisqP <- function(x,m,y)
                {
                ahigh <- apply(x[!y,],2,function(x) sum((x>m[!y])))
                alow <- sum((!y))-ahigh
                bhigh <- apply(x[y,],2,function(x) sum((x>m[y])))
                blow <- sum(y)-bhigh
                p <- sapply(1:length(ahigh), function(i) {
                p <- chisq.test(matrix(c(ahigh[i],alow[i],bhigh[i],blow[i]),2))$p.value/2
                if (ahigh[i]/alow[i] > bhigh[i]/blow[i]) p <- max(p,1-p)
                else p <- min(p,1-p); p})
                return(p)
```

*Hybrid Regression Normalization*

A hybrid normalization method may be used. A hybrid normalization method may reduce bias (e.g., GC bias). A hybrid normalization, may comprise (i) an analysis of a relationship of two variables (e.g., counts and GC content) and (ii) selection and application of a normalization method according to the analysis. A hybrid normalization may comprise (i) a regression (e.g., a regression analysis) and (ii) selection and application of a normalization method according to the regression. Counts obtained for a first sample (e.g., a first set of samples) may be normalized by a different method than counts obtained from another sample (e.g., a second set of samples). Counts obtained for a first sample (e.g., a first set of samples) may be normalized by a first normalization method and counts obtained from a second sample (e.g., a second set of samples) may be normalized by a second normalization method. For example, a first normalization method may comprise use of a linear regression and a second normalization method may comprise use of a non-linear regression (e.g., a LOESS, GC-LOESS, LOWESS regression, LOESS smoothing).

A hybrid normalization method may be used to normalize sequence reads mapped to portions of a genome or chromosome (e.g., counts, mapped counts, mapped reads). Raw counts may be normalized and adjusted, weighted, filtered or previously normalized counts may be normalized by a hybrid normalization method. Genomic section levels or Z-scores may be normalized. Counts mapped to selected portions of a genome or chromosome may be normalized by a hybrid normalization approach. Counts can refer to a suitable measure of sequence reads mapped to portions of a genome, non-limiting examples of which include raw counts (e.g., unprocessed counts), normalized counts (e.g., normalized by PERUN, ChAI or a suitable method), portion levels (e.g., average levels, mean levels, median levels, or the like), Z-scores, the like, or combinations thereof. The counts can be raw counts or processed counts from one or more samples (e.g., a test sample, a sample from a pregnant female). Counts may be obtained from one or more samples obtained from one or more subjects.

A normalization method (e.g., the type of normalization method) may be selected according to a regression (e.g., a regression analysis) and/or a correlation coefficient. A regression analysis refers to a statistical technique for estimating a relationship among variables (e.g., counts and GC content). A regression may be generated according to counts and a measure of GC content for each portion of multiple portions of a reference genome. A suitable measure of GC content can be used, non-limiting examples of which include a measure of guanine, cytosine, adenine, thymine, purine (GC), or pyrimidine (AT or ATU) content, melting temperature ($T_m$) (e.g., denaturation temperature, annealing temperature, hybridization temperature), a measure of free energy, the like or combinations thereof. A measure of guanine (G), cytosine (C), adenine (A), thymine (T), purine (GC), or pyrimidine (AT or ATU) content can be expressed as a ratio or a percentage. Any suitable ratio or percentage may be used, non-limiting examples of which include GC/AT, GC/total nucleotide, GC/A, GC/T, AT/total nucleotide, AT/GC, AT/G, AT/C, G/A, C/A, G/T, G/A, G/AT, C/T, the like or combinations thereof. A measure of GC content may be a ratio or percentage of GC to total nucleotide content. A measure of GC content may be a ratio or percentage of GC to total nucleotide content for sequence reads mapped to a portion of reference genome. The GC content may be determined according to and/or from sequence reads mapped to each portion of a reference genome and the sequence reads are obtained from a sample (e.g., a sample obtained from a pregnant female). A measure of GC content may not be determined according to and/or from sequence reads. A measure of GC content may be determined for one or more samples obtained from one or more subjects.

Generating a regression may comprise generating a regression analysis or a correlation analysis. A suitable regression can be used, non-limiting examples of which include a regression analysis, (e.g., a linear regression analysis), a goodness of fit analysis, a Pearson's correlation analysis, a rank correlation, a fraction of variance unexplained, Nash-Sutcliffe model efficiency analysis, regression model validation, proportional reduction in loss, root mean square deviation, the like or a combination thereof. A regression line may be generated. Generating a regression may comprise generating a linear regression. Generating a regression may comprise generating a non-linear regression (e.g., an LOESS regression, an LOWESS regression).

A regression may determine the presence or absence of a correlation (e.g., a linear correlation), for example between counts and a measure of GC content. A regression (e.g., a linear regression) may be generated and a correlation coefficient is determined. A suitable correlation coefficient may be determined, non-limiting examples of which include a coefficient of determination, an $R^2$ value, a Pearson's correlation coefficient, or the like.

Goodness of fit may be determined for a regression (e.g., a regression analysis, a linear regression). Goodness of fit sometimes may be determined by visual or mathematical analysis. An assessment sometimes includes determining whether the goodness of fit is greater for a non-linear regression or for a linear regression. A correlation coefficient may be a measure of a goodness of fit. An assessment of a goodness of fit for a regression may be determined according to a correlation coefficient and/or a correlation coefficient cutoff value. An assessment of a goodness of fit may comprise comparing a correlation coefficient to a correlation coefficient cutoff value. An assessment of a goodness of fit for a regression may be indicative of a linear regression. For example, a goodness of fit may be greater for a linear regression than for a non-linear regression and the assessment of the goodness of fit is indicative of a linear regression. An assessment may be indicative of a linear regression and a linear regression is used to normalized the counts. An assessment of a goodness of fit for a regression may be indicative of a non-linear regression. For example, a goodness of fit may be greater for a non-linear regression than for a linear regression and the assessment of the goodness of fit is indicative of a non-linear regression. An assessment may be indicative of a non-linear regression and a non-linear regression is used to normalized the counts.

An assessment of a goodness of fit may be indicative of a linear regression when a correlation coefficient is equal to or greater than a correlation coefficient cutoff. An assessment of a goodness of fit may be indicative of a non-linear regression when a correlation coefficient is less than a correlation coefficient cutoff. A correlation coefficient cutoff may be pre-determined. A correlation coefficient cut-off may be about 0.5 or greater, about 0.55 or greater, about 0.6 or greater, about 0.65 or greater, about 0.7 or greater, about 0.75 or greater, about 0.8 or greater or about 0.85 or greater.

For example, a normalization method comprising a linear regression may be used when a correlation coefficient is equal to or greater than about 0.6. Counts of a sample (e.g., counts per portion of a reference genome, counts per portion) may be normalized according to a linear regression when a correlation coefficient is equal to or greater than a correlation coefficient cut-off of 0.6, otherwise the counts are normalized according to a non-linear regression (e.g., when the coefficient is less than a correlation coefficient cut-off of 0.6). A normalization process may comprise generating a linear regression or non-linear regression for the (i) the counts and (ii) the GC content, for each portion of multiple portions of a reference genome. A normalization method comprising a non-linear regression (e.g., a LOWESS, a LOESS) may be used when a correlation coefficient is less than a correlation coefficient cut-off of 0.6. A normalization method comprising a non-linear regression (e.g., a LOWESS) may be used when a correlation coefficient (e.g., a correlation coefficient) is less than a correlation coefficient cut-off of about 0.7, less than about 0.65, less than about 0.6, less than about 0.55 or less than about 0.5. For example, a normalization method comprising a non-linear regression (e.g., a LOWESS, a LOESS) may be used when a correlation coefficient is less than a correlation coefficient cut-off of about 0.6.

A specific type of regression may be selected (e.g., a linear or non-linear regression) and, after the regression is generated, counts may be normalized by subtracting the regression from the counts. A regression from the counts may provide normalized counts with reduced bias (e.g., GC bias). A linear regression may be subtracted from the counts. A non-linear regression (e.g., a LOESS, GC-LOESS, LOWESS regression) may be subtracted from the counts. Any suitable method can be used to subtract a regression line from the counts. For example, if counts x are derived from portion *i* (e.g., a portion *i*) comprising a GC content of 0.5 and a regression line determines counts y at a GC content of 0.5, then x-y = normalized counts for portion *i*. Counts may be normalized prior to and/or after subtracting a regression. Counts normalized by a hybrid normalization approach may be used to generate genomic section levels, Z-cores, levels and/or profiles of a genome or a segment thereof. Counts normalized by a hybrid normalization approach may be analyzed by methods described herein to determine the presence or absence of a copy number variation (e.g., in a fetus).

A hybrid normalization method may comprise filtering or weighting one or more portions before or after normalization. A suitable method of filtering portions, including methods of filtering portions (e.g., portions of a reference genome) described herein can be used. Portions (e.g., portions of a reference genome) may be filtered prior to applying a hybrid normalization method. Only counts of sequencing reads mapped to selected portions (e.g., portions selected according to count variability) may be normalized by a hybrid normalization. Counts of sequencing reads mapped to filtered portions of a reference genome (e.g., portions filtered according to count variability) may be removed prior to utilizing a hybrid normalization method. A hybrid normalization method may comprise selecting or filtering portions (e.g., portions of a reference genome) according to a suitable method (e.g., a method described herein). A hybrid normalization method may comprise selecting or filtering portions (e.g., portions of a reference genome) according to an uncertainty value for counts mapped to each of the portions for multiple test samples. A hybrid normalization method may comprise selecting or filtering portions (e.g., portions of a reference genome) according to count variability. A hybrid normalization method may comprise selecting or filtering portions (e.g., portions of a reference genome) according to GC content, repetitive elements, repetitive sequences, introns, exons, the like or a combination thereof.

For example, multiple samples from multiple pregnant female subjects may be analyzed and a subset of portions (e.g., portions of a reference genome) are selected according to count variability. A linear regression may be used to determine a correlation coefficient for (i) counts and (ii) GC content, for each of the selected portions for a sample obtained from a pregnant female subject. A correlation coefficient may be determined that is greater than a pre-determined correlation cutoff value (e.g., of about 0.6), an assessment of the goodness of fit may be indicative of a linear regression and the counts may be normalized by subtracting the linear regression from the counts. A correlation coefficient may be determined that is less than a pre-determined correlation cutoff value (e.g., of about 0.6), an assessment of the goodness of fit may be indicative of a non-linear regression, an LOESS regression may be generated and the counts my be normalized by subtracting the LOESS regression from the counts.

*Profiles*

A processing step may comprise generating one or more profiles (e.g., profile plot) from various aspects of a data set or derivation thereof (e.g., product of one or more mathematical and/or statistical data processing steps known in the art and/or described herein).

The term "profile" as used herein refers to a product of a mathematical and/or statistical manipulation of data that can facilitate identification of patterns and/or correlations in large quantities of data. A "profile" often includes values resulting from one or more manipulations of data or data sets, based on one or more criteria. A profile often includes multiple data points. Any suitable number of data points may be included in a profile depending on the nature and/or complexity of a data set. Profiles may include 2 or more data points, 3 or more data points, 5 or more data points, 10 or more data points, 24 or more data points, 25 or more data points, 50 or more data points, 100 or more data points, 500 or more data points, 1000 or more data points, 5000 or more data points, 10,000 or more data points, or 100,000 or more data points.

A profile may be representative of the entirety of a data set, and a profile may be representative of a part or subset of a data set. That is, a profile sometimes includes or is generated from data points representative of data that has not been filtered to remove any data, and sometimes a profile includes or is generated from data points representative of data that has been filtered to remove unwanted data. A data point in a profile may represent the results of data manipulation for a portion. A data point in a profile may include results of data manipulation for groups of portions. Groups of portions may be adjacent to one another, groups of portions may be from different parts of a chromosome or genome.

Data points in a profile derived from a data set can be representative of any suitable data categorization. Non-limiting examples of categories into which data can be grouped to generate profile data points include: portions based on size, portions based on sequence features (e.g., GC content, AT content, position on a chromosome (e.g., short arm, long arm, centromere, telomere), and the like), levels of expression, chromosome, the like or combinations thereof. A profile may be generated from data points obtained from another profile (e.g., normalized data profile renormalized to a different normalizing value to generate a renormalized data profile). A profile generated from data points obtained from another profile may reduces the number of data points and/or complexity of the data set. Reducing the number of data points and/or complexity of a data set often facilitates interpretation of data and/or facilitates providing an outcome.

A profile (e.g., a genomic profile, a chromosome profile, a profile of a segment of a chromosome) often is a collection of normalized or non-normalized counts for two or more portions. A profile often includes at least one level (e.g., a genomic section level), and often comprises two or more levels (e.g., a profile often has multiple levels). A level generally is for a set of portions having about the same counts or normalized counts. Levels are described in greater detail herein. A profile may comprise one or more portions, which portions can be weighted, removed, filtered, normalized, adjusted, averaged, derived as a mean, added, subtracted, processed or transformed by any combination thereof. A profile often comprises normalized counts mapped to portions defining two or more levels, where the counts are further normalized according to one of the levels by a suitable method. Often counts of a profile (e.g., a profile level) are associated with an uncertainty value.

A profile comprising one or more levels is sometimes padded (e.g., hole padding). Padding (e.g., hole padding) refers to a process of identifying and adjusting levels in a profile that are due to maternal microdeletions or maternal duplications (e.g., copy number variations). Levels may be padded that are due to fetal microduplications or fetal micro-deletions. Microduplications or microdeletions in a profile may artificially raise or lower the overall level of a profile (e.g., a profile of a chromosome) leading to false positive or false negative determinations of a chromosome aneuploidy (e.g., a trisomy). Levels in a profile that are due to microduplications and/or deletions may be identified and adjusted (e.g., padded and/or removed) by a process sometimes referred to as padding or hole padding. A profile may comprise one or more first levels that are significantly different than a second level within the profile, each of the one or more first levels comprise a maternal copy number variation, fetal copy number variation, or a maternal copy number variation and a fetal copy number variation and one or more of the first levels are adjusted.

A profile comprising one or more levels can include a first level and a second level. A first level may be different (e.g., significantly different) than a second level. A first level may comprise a first set of portions, a second level comprises a second set of portions and the first set of portions is not a subset of the second set of portions. A first set of portions may be different than a second set of portions from which a first and second level are determined. A profile may have multiple first levels that are different (e.g., significantly different, e.g., have a significantly different value) than a second level within the profile. A profile may comprise one or more first levels that are significantly different than a second level within the profile and one or more of the first levels are adjusted. A profile may comprise one or more first levels that are significantly different than a second level within the profile, each of the one or more first levels comprise a maternal copy number variation, fetal copy number variation, or a maternal copy number variation and a fetal copy number variation and one or more of the first levels are adjusted. A first level within a profile may be removed from the profile or adjusted (e.g., padded). A profile can comprise multiple levels that include one or more first levels significantly different than one or more second levels and often the majority of levels in a profile are second levels, which second levels are about equal to one another. Greater than 50%, greater than 60%, greater than 70%, greater than 80%, greater than 90% or greater than 95% of the levels in a profile may be second levels.

A profile sometimes is displayed as a plot. For example, one or more levels representing counts (e.g., normalized counts) of portions can be plotted and visualized. Non-limiting examples of profile plots that can be generated include raw count (e.g., raw count profile or raw profile), normalized count, portion-weighted, z-score, p-value, area ratio versus fitted ploidy, median level versus ratio between fitted and measured fetal fraction, principle components, the like, or combinations thereof. Profile plots may allow visualization of the manipulated data. A profile plot may be utilized to provide an outcome (e.g., area ratio versus fitted ploidy, median level versus ratio between fitted and measured fetal fraction, principle components). The terms "raw count profile plot" or "raw profile plot" as used herein refer to a plot of counts in each portion in a region normalized to total counts in a region (e.g., genome, portion, chromosome, chromosome portions of a reference genome or a segment of a chromosome). A profile may be generated using a static window process, and a profile may be generated using a sliding window process.

A profile generated for a test subject sometimes is compared to a profile generated for one or more reference subjects, to facilitate interpretation of mathematical and/or statistical manipulations of a data set and/or to provide an outcome. A profile may be generated based on one or more starting assumptions (e.g., maternal contribution of nucleic acid (e.g., maternal fraction), fetal contribution of nucleic acid (e.g., fetal fraction), ploidy of reference sample, the like or combinations thereof). A test profile may often center around a predetermined value representative of the absence of a copy number variation, and may often deviate from a predetermined value in areas corresponding to the genomic location in which the copy number variation is located in the test subject, if the test subject possessed the copy number variation. In test subjects at risk for, or suffering from a medical condition associated with a copy number variation, the numerical value for a selected portion is expected to vary significantly from the predetermined value for non-affected genomic locations. Depending on starting assumptions (e.g., fixed ploidy or optimized ploidy, fixed fetal fraction or optimized fetal fraction or combinations thereof) the predetermined threshold or cutoff value or threshold range of values indicative of the presence or absence of a copy number variation can vary while still providing an outcome useful for determining the presence or absence of a copy number variation. A profile may be indicative of and/or representative of a phenotype.

By way of a non-limiting example, normalized sample and/or reference count profiles can be obtained from raw sequence read data by (a) calculating reference median counts for selected chromosomes, portions or segments thereof from a set of references known not to carry a copy number variation, (b) removal of uninformative portions from the reference sample raw counts (e.g., filtering); (c) normalizing the reference counts for all remaining portions of a reference genome to the total residual number of counts (e.g., sum of remaining counts after removal of uninformative portions of a reference genome) for the reference sample selected chromosome or selected genomic location, thereby generating a normalized reference subject profile; (d) removing the corresponding portions from the test subject sample; and (e) normalizing the remaining test subject counts for one or more selected genomic locations to the sum of the residual reference median counts for the chromosome or chromosomes containing the selected genomic locations, thereby generating a normalized test subject profile. An additional normalizing step with respect to the entire genome, reduced by the filtered portions in (b), may be included between (c) and (d).

A data set profile can be generated by one or more manipulations of counted mapped sequence read data. Some aspects include the following. Sequence reads are mapped and the number of counts (i.e. sequence tags) mapping to each genomic portion are determined (e.g., counted). A raw count profile is generated from the mapped sequence reads that are counted. An outcome may be provided by comparing a raw count profile from a test subject to a reference median count profile for chromosomes, portions or segments thereof from a set of reference subjects known not to possess a copy number variation.

Sequence read data may be optionally filtered to remove noisy data or uninformative portions. After filtering, the remaining counts typically are summed to generate a filtered data set. A filtered count profile may be generated from a filtered data set.

After sequence read data have been counted and optionally filtered, data sets can be normalized to generate levels or profiles. A data set can be normalized by normalizing one or more selected portions to a suitable normalizing reference value. A normalizing reference value may be representative of the total counts for the chromosome or chromosomes from which portions are selected. A normalizing reference value may be representative of one or more corresponding portions, portions of chromosomes or chromosomes from a reference data set prepared from a set of reference subjects known not to possess a copy number variation. A normalizing reference value may be representative of one or more corresponding portions, portions of chromosomes or chromosomes from a test subject data set prepared from a test subject being analyzed for the presence or absence of a copy number variation. The normalizing process may be performed utilizing a static window approach, and the normalizing process may be performed utilizing a moving or sliding window approach. A profile comprising normalized counts may be generated to facilitate classification and/or providing an outcome. An outcome can be provided based on a plot of a profile comprising normalized counts (e.g., using a plot of such a profile).

*Levels*

A value (e.g., a number, a quantitative value) may be ascribed to a level. A level can be determined by a suitable method, operation or mathematical process (e.g., a processed level). A level often is, or is derived from, counts (e.g., normalized counts) for a set of portions. A level of a portion may be substantially equal to the total number of counts mapped to a portion (e.g., counts, normalized counts). Often a level is determined from counts that are processed, transformed or manipulated by a suitable method, operation or mathematical process known in the art. A level may be derived from counts that are processed and non-limiting examples of processed counts include weighted, removed, filtered, normalized, adjusted, averaged, derived as a mean (e.g., mean level), added, subtracted, transformed counts or combination thereof. A level may comprise counts that are normalized (e.g., normalized counts of portions). A level can be for counts normalized by a suitable process, non-limiting examples of which include portion-wise normalization, normalization by GC content, median count normalization, linear and nonlinear least squares regression, LOESS (e.g., GC LOESS), LOWESS, PERUN, ChAI, principal component normalization, RM, GCRM, cQn, the like and/or combinations thereof. A level can comprise normalized counts or relative amounts of counts. A level may be for counts or normalized counts of two or more portions that are averaged and the level is referred to as an average level. A level may be for a set of portions having a mean count or mean of normalized counts which is referred to as a mean level. A level may be derived for portions that comprise raw and/or filtered counts. A level may be based on counts that are raw. A level may be associated with an uncertainty value (e.g., a standard deviation, a MAD). A level may be represented by a Z-score or p-value.

A level for one or more portions is synonymous with a "genomic section level" herein. The term "level" as used herein is sometimes synonymous with the term "elevation". The term "level" may be synonymous with "sequence read count representation" and/or "chromosome representation." A determination of the meaning of the term "level" can be determined from the context in which it is used. For example, the term "level", when used in the context of genomic sections, profiles, reads and/or counts often means an elevation. The term "level", when used in the context of a substance or composition (e.g., level of RNA, plexing level) often refers to an amount. The term "level", when used in the context of uncertainty (e.g., level of error, level of confidence, level of deviation, level of uncertainty) often refers to an amount.

Normalized or non-normalized counts for two or more levels (e.g., two or more levels in a profile) can sometimes be mathematically manipulated (e.g., added, multiplied, averaged, normalized, the like or combination thereof) according to levels. For example, normalized or non-normalized counts for two or more levels can be normalized according to one, some or all of the levels in a profile. Normalized or non-normalized counts of all levels in a profile may be normalized according to one level in the profile. Normalized or non-normalized counts of a fist level in a profile may be normalized according to normalized or non-normalized counts of a second level in the profile.

Non-limiting examples of a level (e.g., a first level, a second level) are a level for a set of portions comprising processed counts, a level for a set of portions comprising a mean, median or average of counts, a level for a set of portions comprising normalized counts, the like or any combination thereof. A first level and a second level in a profile may be derived from counts of portions mapped to the same chromosome. A first level and a second level in a profile may be derived from counts of portions mapped to different chromosomes.

A level may be determined from normalized or non-normalized counts mapped to one or more portions. A level may be determined from normalized or non-normalized counts mapped to two or more portions, where the normalized counts for each portion often are about the same. There can be variation in counts (e.g., normalized counts) in a set of portions for a level. In a set of portions for a level there can be one or more portions having counts that are significantly different than in other portions of the set (e.g., peaks and/or dips). Any suitable number of normalized or non-normalized counts associated with any suitable number of portions can define a level.

One or more levels may be determined from normalized or non-normalized counts of all or some of the portions of a genome. Often a level can be determined from all or some of the normalized or non-normalized counts of a chromosome, or segment thereof. Two or more counts derived from two or more portions (e.g., a set of portions) may determine a level. Two or more counts (e.g., counts from two or more portions) may determine a level. Counts from 2 to about 100,000 portions may determine a level. Counts from 2 to about 50,000, 2 to about 40,000, 2 to about 30,000, 2 to about 20,000, 2 to about 10,000, 2 to about 5000, 2 to about 2500, 2 to about 1250, 2 to about 1000, 2 to about 500, 2 to about 250, 2 to about 100 or 2 to about 60 portions may determine a level. Counts from about 10 to about 50 portions may determine a level. Counts from about 20 to about 40 or more portions may determine a level. A level may comprise counts from about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60 or more portions. A level may correspond to a set of portions (e.g., a set of portions of a reference genome, a set of portions of a chromosome or a set of portions of a segment of a chromosome).

A level may be determined for normalized or non-normalized counts of portions that are contiguous. Portions (e.g., a set of portions) that are contiguous may represent neighboring segments of a genome or neighboring segments of a chromosome or gene. For example, two or more contiguous portions, when aligned by merging the portions end to end, can represent a sequence assembly of a DNA sequence longer than each portion. For example two or more contiguous portions can represent of an intact genome, chromosome, gene, intron, exon or segment thereof. A level may be determined from a collection (e.g., a set) of contiguous portions and/or non-contiguous portions.

*Decision Analysis*

A determination of an outcome (e.g., making a call) or a determination of the presence or absence of a chromosome aneuploidy, microduplication or microdeletion may be made according to a decision analysis. Certain decision analysis features are described in International Patent Application Publication No. WO 2014/190286. For example, a decision analysis sometimes comprises applying one or more methods that produce one or more results, an evaluation of the results, and a series of decisions based on the results, evaluations and/or the possible consequences of the decisions and terminating at some juncture of the process where a final decision is made. A decision analysis may be a decision tree. A decision analysis, may comprise coordinated use of one or more processes (e.g., process steps, e.g., algorithms). A decision analysis can be performed by person, a system, apparatus, software (e.g., a module), a computer, a processor (e.g., a microprocessor), the like or a combination thereof. A decision analysis may comprise a method of determining the presence or absence of a chromosome aneuploidy, microduplication or microdeletion in a fetus with reduced false negative and reduced false positive determinations, compared to an instance in which no decision analysis is utilized (e.g., a determination is made directly from normalized counts). A decision analysis may comprise determining the presence or absence of a condition associated with one or more microduplications or microdeletions. For example, a decision analysis may comprise determining the presence or absence of one or more copy number variations associated with DiGeorge syndrome for a test sample from a subject. A decision analysis may comprise determining the presence or absence of DiGeorge syndrome for a test sample from a subject.

A decision analysis may comprise generating a profile for a genome or a segment of a genome (e.g., a chromosome or part thereof). A profile can be generated by any suitable method, known or described herein, and often includes obtaining counts of sequence reads mapped to portions of a reference genome, normalizing counts, normalizing levels, padding, the like or combinations thereof. Obtaining counts of sequence reads mapped to a reference genome can include obtaining a sample (e.g., from a pregnant female subject), sequencing nucleic acids from a sample (e.g., circulating cell-free nucleic acids), obtaining sequence reads, mapping sequence reads to portions of a reference genome, the like and combinations thereof. Generating a profile may comprise normalizing counts mapped to portions of a reference genome, thereby providing calculated genomic section levels.

A decision analysis may comprise segmenting. Segmenting may modify and/or transform a profile thereby providing one or more decomposition renderings of a profile. A profile subjected to a segmenting process often is a profile of normalized counts mapped to portions (e.g., bins) in a reference genome or portion thereof (e.g., autosomes and sex chromosomes). As addressed herein, raw counts mapped to the portions can be normalized by one or more suitable normalization processes (e.g. PERUN, LOESS, GC-LOESS, principal component normalization (ChAI) or combination thereof) to generate a profile that is segmented as part of a decision analysis. A decomposition rendering of a profile is often a transformation of a profile. A decomposition rendering of a profile is sometimes a transformation of a profile into a representation of a genome, chromosome or segment thereof.

A segmenting process utilized for the segmenting may locate and identify one or more levels within a profile that are different (e.g., substantially or significantly different) than one or more other levels within a profile. A level identified in a profile according to a segmenting process that is different than another level in the profile, and has edges that are different than another level in the profile, is referred to herein as a wavelet, and more generally as a level for a discrete segment. A segmenting process can generate, from a profile of normalized counts or levels, a decomposition rendering in which one or more discrete segments or wavelets can be identified. A discrete segment generally covers fewer portions (e.g., bins) than what is segmented (e.g., chromosome, chromosomes, autosomes).

Segmenting may locate and identify edges of discrete segments and wavelets within a profile. One or both edges of one or more discrete segments and wavelets may be identified. For example, a segmentation process can identify the location (e.g., genomic coordinates, e.g., portion location) of the right and/or the left edges of a discrete segment or wavelet in a profile. A discrete segment or wavelet often comprises two edges. For example, a discrete segment or wavelet can include a left edge and a right edge. Depending upon the representation or view, a left edge may be a 5'-edge and a right edge may be a 3'-edge of a nucleic acid segment in a profile. A left edge may be a 3'-edge and a right edge may bne be a 5'-edge of a nucleic acid segment in a profile. Often the edges of a profile are known prior to segmentation and therefore, the edges of a profile may determine which edge of a level is a 5'-edge and which edge is 3'-edge. One or both edges of a profile and/or discrete segment (e.g., wavelet) may be an edge of a chromosome.

The edges of a discrete segment or wavelet may be determined according to a decomposition rendering generated for a reference sample (e.g., a reference profile). A null edge height distribution may be determined according to a decomposition rendering of a reference profile (e.g., a profile of a chromosome or segment thereof). The edges of a discrete segment or wavelet in a profile may be identified when the level of the discrete segment or wavelet is outside a null edge height distribution. The edges of a discrete segment or wavelet in a profile may be identified according a Z-score calculated according to a decomposition rendering for a reference profile.

Sometimes segmenting generates two or more discrete segments or wavelets (e.g., two or more fragmented levels, two or more fragmented segments) in a profile. A decomposition rendering derived from a segmenting process may be over-segmented or fragmented and may comprise multiple discrete segments or wavelets. Sometimes discrete segments or wavelets generated by segmenting are substantially different and sometimes discrete segments or wavelets generated by segmenting are substantially similar. Substantially similar discrete segments or wavelets (e.g., substantially similar levels) often refers to two or more adjacent discrete segments or wavelets in a segmented profile each having a genomic section level (e.g., a level) that differs by less than a predetermined level of uncertainty. Substantially similar discrete segments or wavelets may be adjacent to each other and may not be separated by an intervening segment or wavelet. Substantially similar discrete segments or wavelets may be separated by one or more smaller segments or wavelets. Substantially similar discrete segments or wavelets may be separated by about 1 to about 20, about 1 to about 15, about 1 to about 10 or about 1 to about 5 portions (e.g., bins) where one or more of the intervening portions have a level significantly different that the level of each of the substantially similar discrete segments or wavelets. The level of substantially similar discrete segments or wavelets may differ by less than about 3 times, less than about 2 times, less than about 1 times or less than about 0.5 times a level of uncertainty. Substantially similar discrete segments or wavelets may comprise a median genomic section level that differs by less than 3 MAD (e.g., less than 3 sigma), less than 2 MAD, less than 1 MAD or less than about 0.5 MAD, where a MAD is calculated from a median genomic section level of each of the segments or wavelets. Substantially different discrete segments or wavelets may not be adjacent or are separated by 10 or more, 15 or more or 20 or more portions. Substantially different discrete segments or wavelets generally have substantially different levels. Substantially different discrete segments or wavelets may comprise levels that differ by more than about 2.5 times, more than about 3 times, more than about 4 times, more than about 5 times, more than about 6 times a level of uncertainty. Substantially different discrete segments or wavelets may comprise a median genomic section level that differs by more than 2.5 MAD (e.g., more than 2.5 sigma), more than 3 MAD, more than 4 MAD, more than about 5 MAD or more than about 6 MAD, where a MAD is calculated from a median genomic section level of each of the discrete segments or wavelets.

A segmentation process may comprise determining (e.g., calculating) a level (e.g., a quantitative value, e.g., a mean or median level), a level of uncertainty (e.g., an uncertainty value), Z-score, Z-value, p-value, the like or combinations thereof for one or more discrete segments or wavelets (e.g., levels) in a profile or segment thereof. In A level (e.g., a quantitative value, e.g., a mean or median level), a level of uncertainty (e.g., an uncertainty value), Z-score, Z-value, p-value, the like or combinations thereof may be determined (e.g., calculated) for a discrete segment or wavelet.

Segmenting may be accomplished by a process that comprises one process or multiple sub-processes, non-limiting examples of which include a decomposition generating process (e.g., a wavelet decomposition generating process), thresholding, leveling, smoothing, the like or combination thereof. Thresholding, leveling, smoothing and the like can be performed in conjunction with a decomposition generating process, and/or a wavelet decomposition rendering process.

*Outcome*

Methods described herein can provide a determination of the presence or absence of a genetic variation (e.g., fetal aneuploidy) for a sample, thereby providing an outcome (e.g., thereby providing an outcome determinative of the presence or absence of a genetic variation (e.g., fetal aneuploidy)). A genetic variation often includes a gain, a loss and/or alteration (e.g., duplication, deletion, fusion, insertion, mutation, reorganization, substitution or aberrant methylation) of genetic information (e.g., chromosomes, segments of chromosomes, polymorphic regions, translocated regions, altered nucleotide sequence, the like or combinations of the foregoing) that results in a detectable change in the genome or genetic information of a test subject with respect to a reference. Presence or absence of a genetic variation can be determined by transforming, analyzing and/or manipulating sequence reads that have been mapped to portions (e.g., counts, counts of genomic portions of a reference genome). Determining an outcome may comprise analyzing nucleic acid from a pregnant female. An outcome may be determined according to counts (e.g., normalized counts, read densities, read density profiles) obtained from a pregnant female where the counts are from nucleic acid obtained from the pregnant female.

Methods described herein sometimes determine presence or absence of a fetal aneuploidy (e.g., full chromosome aneuploidy, partial chromosome aneuploidy or segmental chromosomal aberration (e.g., mosaicism, deletion and/or insertion)) for a test sample from a pregnant female bearing a fetus. Methods described herein may detect euploidy or lack of euploidy (non-euploidy) for a sample from a pregnant female bearing a fetus. Methods described herein sometimes detect trisomy for one or more chromosomes (e.g., chromosome 13, chromosome 18, chromosome 21 or combination thereof) or segment thereof.

Presence or absence of a genetic variation (e.g., a fetal aneuploidy) may be determined by a method described herein, by a method known in the art or by a combination thereof. Presence or absence of a genetic variation generally is determined from counts of sequence reads mapped to portions of a reference genome.

Read densities from a reference sometimes are for a nucleic acid sample from the same pregnant female from which a test sample is obtained. Read densities from a reference may be for a nucleic acid sample from one or more pregnant females different than the female from which a test sample was obtained. Read densities and/or read density profiles from a first set of portions form a test subject may be compared to read densities and/or read density profiles from a second set of portions, where the second set of portions may be different than the first set of portions. Read densities and/or read density profiles from a first set of portions form a test subject may be compared to read densities and/or read density profiles from a second set of portions, where the second set of portion may be from the test subject or from a reference subject that is not the test subject. In a non-limiting example, where a first set of portions is in chromosome 21 or segment thereof, a second set of portions often is in another chromosome (e.g., chromosome 1, chromosome 13, chromosome 14, chromosome 18, chromosome 19, segment thereof or combination of the foregoing). A reference often is located in a chromosome or segment thereof that is typically euploid. For example, chromosome 1 and chromosome 19 often are euploid in fetuses owing to a high rate of early fetal mortality associated with chromosome 1 and chromosome 19 aneuploidies. A measure of uncertainty between the read densities and/or read density profiles from a test subject and a reference can be generated and/or compared. Presence or absence of a genetic variation (e.g., fetal aneuploidy) sometimes is determined without comparing read densities and/or read density profiles from a test subject to a reference.

A reference may comprise read densities and/or a read profile for the same set of portions as for a test subject, where the read densities for the reference are from one or more reference samples (e.g., often multiple reference samples from multiple reference subjects). A reference sample often is from one or more pregnant females different than a female from which a test sample is obtained.

A measure of uncertainty for read densities and/or read profiles of a test subject and/or reference can be generated. A measure of uncertainty may be determined for read densities and/or read profiles of a test subject. A measure of uncertainty may be determined for read densities and/or read profiles of a reference subject. A measure of uncertainty may be determined from an entire read density profile or a subset of portions within a read density profile.

Reference samples may be euploid for a selected segment of a genome, and a measure of uncertainty between a test profile and a reference profile is assessed for the selected segment. A determination of the presence or absence of a genetic variation may be according to the number of deviations (e.g., measures of deviations, MAD) between a test profile and a reference profile for a selected segment of a genome (e.g., a chromosome, or segment thereof). The presence of a genetic variation may be determined when the number of deviations between a test profile and a reference profile is greater than about 1, greater than about 1.5, greater than about 2, greater than about 2.5, greater than about 2.6, greater than about 2.7, greater than about 2.8, greater than about 2.9, greater than about 3, greater than about 3.1, greater than about 3.2, greater than about 3.3, greater than about 3.4, greater than about 3.5, greater than about 4, greater than about 5, or greater than about 6. For example, sometimes a test profile and a reference profile differ by more than 3 measures of deviation (e.g., 3 sigma, 3 MAD) and the presence of a genetic variation is determined. A test profile obtained from a pregnant female may be larger than a reference profile by more than 3 measures of deviation (e.g., 3 sigma, 3 MAD) and the presence of a fetal chromosome aneuploidy (e.g., a fetal trisomy) may be determined. A deviation of greater than three between a test profile and a reference profile often is indicative of a non-euploid test subject (e.g., presence of a genetic variation) for a selected segment of a genome. A test profile significantly greater than a reference profile for a selected segment of a genome, which reference is euploid for the selected segment, sometimes is determinative of a trisomy. A read density profile obtained from a pregnant female may be less than a reference profile for a selected segment, by more than 3 measures of deviation (e.g., 3 sigma, 3 MAD) and the presence of a fetal chromosome aneuploidy (e.g., a fetal monosomy) is determined. Test profiles significantly below a reference profile, which reference profile is indicative of euploidy, sometimes are determinative of a monosomy.

The absence of a genetic variation may be determined when the number of deviations between a test profile and reference profile for a selected segment of a genome is less than about 3.5, less than about 3.4, less than about 3.3, less than about 3.2, less than about 3.1 ,less than about 3.0,less than about 2.9,less than about 2.8,less than about 2.7,less than about 2.6, less than about 2.5, less than about 2.0, less than about 1.5, or less than about 1.0. For example, sometimes a test profile differs from a reference profile by less than 3 measures of deviation (e.g., 3 sigma, 3 MAD) and the absence of a genetic variation is determined. A test profile obtained from a pregnant female may differ from a reference profile by less than 3 measures of deviation (e.g., 3 sigma, 3 MAD) and the absence of a fetal chromosome aneuploidy (e.g., a fetal euploid) is determined. (E.g., deviation of less than three between test profiles and reference profiles (e.g., 3-sigma for standard deviation) often may be indicative of a segment of a genome that is euploid (e.g., absence of a genetic variation). A measure of deviation between test profiles for a test sample and reference profiles for one or more reference subjects can be plotted and visualized (e.g., z-score plot).

Any other suitable reference can be factored with test profiles for determining presence or absence of a genetic variation (or determination of euploid or non-euploid) for a test region (e.g., a segment of a genome that is tested) of a test sample. A fetal fraction determination may be factored with counts of sequence reads (e.g., read densities) to determine the presence or absence of a genetic variation. For example, read densities and/or read density profiles can be normalized according to fetal fraction prior to a comparison and/or determining an outcome. A suitable process for quantifying fetal fraction can be utilized, non-limiting examples of which include a mass spectrometric process, sequencing process or combination thereof.

A determination of the presence or absence of a genetic variation (e.g., a fetal aneuploidy) may be determined according to a call zone. A call may be made (e.g., a call determining the presence or absence of a genetic variation, e.g., an outcome) when a value (e.g., a read density profile and/or a measure of uncertainty) or collection of values falls within a pre-defined range (e.g., a zone, a call zone). A call zone may be defined according to a collection of values (e.g., read density profiles and/or measures of uncertainty) that are obtained from the same patient sample. A call zone may be defined according to a collection of values that are derived from the same chromosome or segment thereof. A call zone based on a genetic variation determination may be defined according a measure of uncertainty (e.g., high level of confidence, e.g., low measure of uncertainty) and/or a fetal fraction.

A call zone may be defined according to a determination of a genetic variation and a fetal fraction of about 2.0% or greater, about 2.5% or greater, about 3% or greater, about 3.25% or greater, about 3.5% or greater, about 3.75% or greater, or about 4.0 % or greater. For example, a call may be made that a fetus comprises a trisomy 21 based on a comparison of a test profile and a reference profile where a test sample, from which the test profile was derived, comprises a fetal fraction determination of 2% or greater or 4% or greater for a test sample obtained from a pregnant female bearing a fetus. For example, a call may be made that a fetus is euploid based on a comparison of a test profile and a reference profile where a test sample, from which the test profile was derived, comprises a fetal fraction determination of 2% or greater or 4% or greater for a test sample obtained from a pregnant female bearing a fetus. A call zone may be defined by a confidence level of about 99% or greater, about 99.1% or greater, about 99.2% or greater, about 99.3% or greater, about 99.4% or greater, about 99.5% or greater, about 99.6% or greater, about 99.7% or greater, about 99.8% or greater or about 99.9% or greater. A call may be made without using a call zone. A call may be made using a call zone and additional data or information. A call may be made based on a comparison without the use of a call zone. A call may be made based on visual inspection of a profile (e.g., visual inspection of read densities).

A no-call zone may be where a call is not made. A no-call zone may be defined by a value or collection of values that indicate low accuracy, high risk, high error, low level of confidence, high measure of uncertainty, the like or a combination thereof. A no-call zone may be defined, in part, by a fetal fraction of about 5% or less, about 4% or less, about 3% or less, about. 2.5% or less, about 2.0% or less, about 1.5% or less or about 1.0% or less. '

A genetic variation sometimes is associated with medical condition. An outcome determinative of a genetic variation is sometimes an outcome determinative of the presence or absence of a condition (e.g., a medical condition), disease, syndrome or abnormality, or includes, detection of a condition, disease, syndrome or abnormality (e.g., non-limiting examples listed in Table 1). A diagnosis may comprise assessment of an outcome. An outcome determinative of the presence or absence of a condition (e.g., a medical condition), disease, syndrome or abnormality by methods described herein can sometimes be independently verified by further testing (e.g., by karyotyping and/or amniocentesis). Analysis and processing of data can provide one or more outcomes. The term "outcome" as used herein can refer to a result of data processing that facilitates determining the presence or absence of a genetic variation (e.g., an aneuploidy, a copy number variation). The term "outcome" as used herein may refer to a conclusion that predicts and/or determines the presence or absence of a genetic variation (e.g., an aneuploidy, a copy number variation). The term "outcome" as used herein may refer to a conclusion that predicts and/or determines a risk or probability of the presence or absence of a genetic variation (e.g., an aneuploidy, a copy number variation) in a subject (e.g., a fetus). A diagnosis sometimes comprises use of an outcome. For example, a health practitioner may analyze an outcome and provide a diagnosis bases on, or based in part on, the outcome. Determination, detection or diagnosis of a condition, syndrome or abnormality (e.g., listed in Table 1) may comprise use of an outcome determinative of the presence or absence of a genetic variation. An outcome based on counted mapped sequence reads or transformations thereof may be determinative of the presence or absence of a genetic variation. An outcome generated utilizing one or more methods (e.g., data processing methods) described herein may be determinative of the presence or absence of one or more conditions, syndromes or abnormalities listed in Table 1. A diagnosis may comprise a determination of a presence or absence of a condition, syndrome or abnormality. Often a diagnosis comprises a determination of a genetic variation as the nature and/or cause of a condition, syndrome or abnormality. An outcome may not be a diagnosis. An outcome often comprises one or more numerical values generated using a processing method described herein in the context of one or more considerations of probability. A consideration of risk or probability can include, but is not limited to: a measure of uncertainty, a confidence level, sensitivity, specificity, standard deviation, coefficient of variation (CV) and/or confidence level, Z-scores, Chi values, Phi values, ploidy values, fitted fetal fraction, area ratios, median level, the like or combinations thereof. A consideration of probability can facilitate determining whether a subject is at risk of having, or has, a genetic variation, and an outcome determinative of a presence or absence of a genetic disorder often includes such a consideration.

An outcome sometimes is a phenotype. An outcome sometimes is a phenotype with an associated level of confidence (e.g., a measure of uncertainty, e.g., a fetus is positive for trisomy 21 with a confidence level of 99%, a test subject is negative for a cancer associated with a genetic variation at a confidence level of 95%). Different methods of generating outcome values sometimes can produce different types of results. Generally, there are four types of possible scores or calls that can be made based on outcome values generated using methods described herein: true positive, false positive, true negative and false negative. The terms "score", "scores", "call" and "calls" as used herein refer to calculating the probability that a particular genetic variation is present or absent in a subject/sample. The value of a score may be used to determine, for example, a variation, difference, or ratio of mapped sequence reads that may correspond to a genetic variation. For example, calculating a positive score for a selected genetic variation or portion from a data set, with respect to a reference genome can lead to an identification of the presence or absence of a genetic variation, which genetic variation sometimes is associated with a medical condition (e.g., cancer, preeclampsia, trisomy, monosomy, and the like). An outcome may comprise a read density, a read density profile and/or a plot (e.g., a profile plot). In those aspects in which an outcome comprises a profile, a suitable profile or combination of profiles can be used for an outcome. Non-limiting examples of profiles that can be used for an outcome include z-score profiles, p-value profiles, chi value profiles, phi value profiles, the like, and combinations thereof

An outcome generated for determining the presence or absence of a genetic variation sometimes includes a null result (e.g., a data point between two clusters, a numerical value with a standard deviation that encompasses values for both the presence and absence of a genetic variation, a data set with a profile plot that is not similar to profile plots for subjects having or free from the genetic variation being investigated). An outcome indicative of a null result still may be a determinative result, and the determination can include the need for additional information and/or a repeat of the data generation and/or analysis for determining the presence or absence of a genetic variation.

An outcome may be generated after performing one or more processing steps described herein. An outcome may be generated as a result of one of the processing steps described herein, and an outcome may be generated after each statistical and/or mathematical manipulation of a data set is performed. An outcome pertaining to the determination of the presence or absence of a genetic variation can be expressed in a suitable form, which form comprises without limitation, a probability (e.g., odds ratio, p-value), likelihood, value in or out of a cluster, value over or under a threshold value, value within a range (e.g., a threshold range), value with a measure of variance or confidence, or risk factor, associated with the presence or absence of a genetic variation for a subject or sample. Comparison between samples may allow confirmation of sample identity (e.g., may allow identification of repeated samples and/or samples that have been mixed up (e.g., mislabeled, combined, and the like)).

An outcome may comprise a value above or below a predetermined threshold or cutoff value and/or a measure of uncertainty or a confidence level associated with the value. A predetermined threshold or cutoff value may be an expected level or an expected level range. An outcome also can describe an assumption used in data processing. An outcome may comprises a value that falls within or outside a predetermined range of values (e.g., a threshold range) and the associated uncertainty or confidence level for that value being inside or outside the range. An outcome may comprise a value that is equal to a predetermined value (e.g., equal to 1, equal to zero), or is equal to a value within a predetermined value range, and its associated uncertainty or confidence level for that value being equal or within or outside a range. An outcome sometimes is graphically represented as a plot (e.g., profile plot).

As noted above, an outcome can be characterized as a true positive, true negative, false positive or false negative. The term "true positive" as used herein refers to a subject correctly diagnosed as having a genetic variation. The term "false positive" as used herein refers to a subject wrongly identified as having a genetic variation. The term "true negative" as used herein refers to a subject correctly identified as not having a genetic variation. The term "false negative" as used herein refers to a subject wrongly identified as not having a genetic variation. Two measures of performance for any given method can be calculated based on the ratios of these occurrences: (i) a sensitivity value, which generally is the fraction of predicted positives that are correctly identified as being positives; and (ii) a specificity value, which generally is the fraction of predicted negatives correctly identified as being negative.

One or more of sensitivity, specificity and/or confidence level may be expressed as a percentage. The percentage, independently for each variable, may be greater than about 90% (e.g., about 90, 91, 92, 93, 94, 95, 96, 97, 98 or 99%, or greater than 99% (e.g., about 99.5%, or greater, about 99.9% or greater, about 99.95% or greater, about 99.99% or greater)). Coefficient of variation (CV) may be expressed as a percentage, and sometimes the percentage is about 10% or less (e.g., about 10, 9, 8, 7, 6, 5, 4, 3, 2 or 1%, or less than 1% (e.g., about 0.5% or less, about 0.1% or less, about 0.05% or less, about 0.01% or less)). A probability (e.g., that a particular outcome is not due to chance) may be expressed as a Z-score, a p-value, or the results of a t-test. A measured variance, confidence interval, sensitivity, specificity and the like (e.g., referred to collectively as confidence parameters) for an outcome may be be generated using one or more data processing manipulations described herein. Specific examples of generating outcomes and associated confidence levels are described in the Examples section and in international patent application no. PCT/US12/59123 (WO2013/052913).

The term "sensitivity" as used herein refers to the number of true positives divided by the number of true positives plus the number of false negatives, where sensitivity (sens) may be within the range of $0 \leq sens \leq 1$. The term "specificity" as used herein refers to the number of true negatives divided by the number of true negatives plus the number of false positives, where sensitivity (spec) may be within the range of $0 \leq spec \leq 1$A method that has sensitivity and specificity equal to one, or 100%, or near one (e.g., between about 90% to about 99%) sometimes may be selected. A method having a sensitivity equaling 1, or 100% may be selected, and a method having a sensitivity near 1 may be selected (e.g., a sensitivity of about 90%, a sensitivity of about 91%, a sensitivity of about 92%, a sensitivity of about 93%, a sensitivity of about 94%, a sensitivity of about 95%, a sensitivity of about 96%, a sensitivity of about 97%, a sensitivity of about 98%, or a sensitivity of about 99%). A method having a specificity equaling 1, or 100% may be selected, and a method having a specificity near 1 may be selected (e.g., a specificity of about 90%, a specificity of about 91%, a specificity of about 92%, a specificity of about 93%, a specificity of about 94%, a specificity of about 95%, a specificity of about 96%, a specificity of about 97%, a specificity of about 98%, or a specificity of about 99%).

Presence or absence of a genetic variation (e.g., chromosome aneuploidy) may be determined for a fetus. Presence or absence of a fetal genetic variation (e.g., fetal chromosome aneuploidy) may be determined.

Presence or absence of a genetic variation (e.g., chromosome aneuploidy) may be determined for a sample. Presence or absence of a genetic variation in sample nucleic acid (e.g., chromosome aneuploidy) may be determined. A variation detected or not detected may reside in sample nucleic acid from one source but not in sample nucleic acid from another source. Non-limiting examples of sources include placental nucleic acid, fetal nucleic acid, maternal nucleic acid, cancer cell nucleic acid, non-cancer cell nucleic acid, the like and combinations thereof. In non-limiting examples, a particular genetic variation detected or not detected (i) resides in placental nucleic acid but not in fetal nucleic acid and not in maternal nucleic acid; (ii) resides in fetal nucleic acid but not maternal nucleic acid; or (iii) resides in maternal nucleic acid but not fetal nucleic acid.

The presence or absence of a genetic variation and/or associated medical condition (e.g., an outcome) is often provided by an outcome module. The presence or absence of a genetic variation (e.g., an aneuploidy, a fetal aneuploidy, a copy number variation) may be identified by an outcome module or by a machine comprising an outcome module. An outcome module can be specialized for determining a specific genetic variation (e.g., a trisomy, a trisomy 21, a trisomy 18). For example, an outcome module that identifies a trisomy 21 can be different than and/or distinct from an outcome module that identifies a trisomy 18. An outcome module or a machine comprising an outcome module may be required to identify a genetic variation or an outcome determinative of a genetic variation (e.g., an aneuploidy, a copy number variation). An outcome may be transferred from an outcome module to a display module where an outcome is provided by the display module.

A genetic variation or an outcome determinative of a genetic variation identified by methods described herein can be independently verified by further testing (e.g., by targeted sequencing of maternal and/or fetal nucleic acid). An outcome typically is provided to a health care professional (e.g., laboratory technician or manager; physician or assistant). An outcome may be provided on a suitable visual medium (e.g., a peripheral or component of a machine, e.g., a printer or display). An outcome determinative of the presence or absence of a genetic variation may be provided to a healthcare professional in the form of a report, and the report may comprise a display of an outcome value and an associated confidence parameter. Generally, an outcome can be displayed in a suitable format that facilitates determination of the presence or absence of a genetic variation and/or medical condition. Non-limiting examples of formats suitable for use for reporting and/or displaying data sets or reporting an outcome include digital data, a graph, a 2D graph, a 3D graph, and 4D graph, a picture (e.g., a jpg, bitmap (e.g., bmp), pdf, tiff, gif, raw, png, the like or suitable format), a pictograph, a chart, a table, a bar graph, a pie graph, a diagram, a flow chart, a scatter plot, a map, a histogram, a density chart, a function graph, a circuit diagram, a block diagram, a bubble map, a constellation diagram, a contour diagram, a cartogram, spider chart, Venn diagram, nomogram, and the like, and combination of the foregoing.

Generating an outcome may be viewed as a transformation of nucleic acid sequence read data, or the like, into a representation of a subject's cellular nucleic acid. For example, analyzing sequence reads of nucleic acid from a subject and generating a chromosome profile and/or outcome can be viewed as a transformation of relatively small sequence read fragments to a representation of relatively large chromosome structure. An outcome may result from a. transformation of sequence reads from a subject (e.g., a pregnant female), into a representation of an existing structure (e.g., a genome, a chromosome or segment thereof) present in the subject (e.g., a maternal and/or fetal nucleic acid). An outcome may comprise a transformation of sequence reads from a first subject (e.g., a pregnant female), into a composite representation of structures (e.g., a genome, a chromosome or segment thereof), and a second transformation of the composite representation that yields a representation of a structure present in a first subject (e.g., a pregnant female) and/or a second subject (e.g., a fetus).

*Use of Outcomes*

A health care professional, or other qualified individual, receiving a report comprising one or more outcomes determinative of the presence or absence of a genetic variation can use the displayed data in the report to make a call regarding the status of the test subject or patient. The healthcare professional may make a recommendation based on the provided outcome. A health care professional or qualified individual may provide a test subject or patient with a call or score with regards to the presence or absence of the genetic variation based on the outcome value or values and associated confidence parameters provided in a report. A score or call may be made manually by a healthcare professional or qualified individual, using visual observation of the provided report. A score or call may be made by an automated routine, sometimes embedded in software, and reviewed by a healthcare professional or qualified individual for accuracy prior to providing information to a test subject or patient. The term "receiving a report" as used herein refers to obtaining, by a communication means, a written and/or graphical representation comprising an outcome, which upon review allows a healthcare professional or other qualified individual to make a determination as to the presence or absence of a genetic variation in a test subject or patient. The report may be generated by a computer or by human data entry, and can be communicated using electronic means (e.g., over the internet, via computer, via fax, from one network location to another location at the same or different physical sites), or by a other method of sending or receiving data (e.g., mail service, courier service and the like). The outcome may be transmitted to a health care professional in a suitable medium, including, without limitation, in verbal, document, or file form. The file may be, for example, but not limited to, an auditory file, a computer readable file, a paper file, a laboratory file or a medical record file.

The term "providing an outcome" and grammatical equivalents thereof, as used herein also can refer to a method for obtaining such information, including, without limitation, obtaining the information from a laboratory (e.g., a laboratory file). A laboratory file can be generated by a laboratory that carried out one or more assays or one or more data processing steps to determine the presence or absence of the medical condition. The laboratory may be in the same location or different location (e.g., in another country) as the personnel identifying the presence or absence of the medical condition from the laboratory file. For example, the laboratory file can be generated in one location and transmitted to another location in which the information therein will be transmitted to the pregnant female subject. The laboratory file may be in tangible form or electronic form (e.g., computer readable form).

An outcome may be provided to a health care professional, physician or qualified individual from a laboratory and the health care professional, physician or qualified individual can make a diagnosis based on the outcome. An outcome may be provided to a health care professional, physician or qualified individual from a laboratory and the health care professional, physician or qualified individual can make a diagnosis based, in part, on the outcome along with additional data and/or information and other outcomes.

A healthcare professional or qualified individual, can provide a suitable recommendation based on the outcome or outcomes provided in the report. Non-limiting examples of recommendations that can be provided based on the provided outcome report includes, surgery, radiation therapy, chemotherapy, genetic counseling, after birth treatment solutions (e.g., life planning, long term assisted care, medicaments, symptomatic treatments), pregnancy termination, organ transplant, blood transfusion, the like or combinations of the foregoing. The recommendation may be dependent on the outcome based classification provided (e.g., Down's syndrome, Turner syndrome, medical conditions associated with genetic variations in T13, medical conditions associated with genetic variations in T18).

Laboratory personnel (e.g., a laboratory manager) can analyze values (e.g., test profiles, reference profiles, level of deviation) underlying a determination of the presence or absence of a genetic variation (or determination of euploid or non-euploid for a test region). For calls pertaining to presence or absence of a genetic variation that are close or questionable, laboratory personnel can re-order the same test, and/or order a different test (e.g., karyotyping and/or amniocentesis in the case of fetal aneuploidy determinations), that makes use of the same or different sample nucleic acid from a test subject.

*Machines, Software and Interfaces*

Certain processes and methods described herein (e.g., quantifying, mapping, normalizing, range setting, adjusting, categorizing, counting and/or determining sequence reads, counts, levels (e.g., levels) and/or profiles) often cannot be performed without a computer, microprocessor, software, module or other machine. Methods described herein typically are computer-implemented methods, and one or more portions of a method sometimes are performed by one or more processors (e.g., microprocessors), computers, or microprocessor controlled machines. Aspects pertaining to methods described in this document generally are applicable to the same or related processes implemented by instructions in systems, machines and computer program products described herein. Aspects pertaining to methods described in this document generally can be applicable to the same or related processes implemented by a non-transitory computer-readable storage medium with an executable program stored thereon, where the program instructs a microprocessor to perform the method, or a part thereof. Processes and methods described herein (e.g., quantifying, counting and/or determining sequence reads, counts, levels and/or profiles) may be performed by automated methods. One or more steps and a method described herein may be carried out by a microprocessor and/or computer, and/or carried out in conjunction with memory. An automated method may be embodied in software, modules, microprocessors, peripherals and/or a machine comprising the like, that determine sequence reads, counts, mapping, mapped sequence tags, levels, profiles, normalizations, comparisons, range setting, categorization, adjustments, plotting, outcomes, transformations and identifications. As used herein, software refers to computer readable program instructions that, when executed by a microprocessor, perform computer operations, as described herein.

Sequence reads, counts, levels, and profiles derived from a test subject (e.g., a patient, a pregnant female) and/or from a reference subject can be further analyzed and processed to determine the presence or absence of a copy number variation. Sequence reads, counts, levels and/or profiles sometimes are referred to as "data" or "data sets". Data or data sets may be characterized by one or more features or variables (e.g., sequence based [e.g., GC content, specific nucleotide sequence, the like], function specific [e.g., expressed genes, cancer genes, the like], location based [genome specific, chromosome specific, portion or portion-specific], the like and combinations thereof). Data or data sets may be organized into a matrix having two or more dimensions based on one or more features or variables. Data organized into matrices can be organized using any suitable features or variables. A non-limiting example of data in a matrix includes data that is organized by maternal age, maternal ploidy, and fetal contribution. Data sets characterized by one or more features or variables sometimes may be processed after counting.

Machines, software and interfaces may be used to conduct methods described herein. Using machines, software and interfaces, a user may enter, request, query or determine options for using particular information, programs or processes (e.g., mapping sequence reads, processing mapped data and/or providing an outcome), which can involve implementing statistical analysis algorithms, statistical significance algorithms, statistical algorithms, iterative steps, validation algorithms, and graphical representations, for example. A data set may be entered by a user as input information, a user may download one or more data sets by a suitable hardware media (e.g., flash drive), and/or a user may send a data set from one system to another for subsequent processing and/or providing an outcome (e.g., send sequence read data from a sequencer to a computer system for sequence read mapping; send mapped sequence data to a computer system for processing and yielding an outcome and/or report).

A system typically comprises one or more machines. Each machine comprises one or more of memory, one or more microprocessors, and instructions. Where a system includes two or more machines, some or all of the machines may be located at the same location, some or all of the machines may be located at different locations, all of the machines may be located at one location and/or all of the machines may be located at different locations. Where a system includes two or more machines, some or all of the machines may be located at the same location as a user, some or all of the machines may be located at a location different than a user, all of the machines may be located at the same location as the user, and/or all of the machine may be located at one or more locations different than the user.

A system sometimes comprises a computing machine and a sequencing apparatus or machine, where the sequencing apparatus or machine is configured to receive physical nucleic acid and generate sequence reads, and the computing apparatus is configured to process the reads from the sequencing apparatus or machine. The computing machine sometimes is configured to determine the presence or absence of a genetic variation (e.g., copy number variation; fetal chromosome aneuploidy) from the sequence reads.

A user may, for example, place a query to software which then may acquire a data set via internet access, and a programmable microprocessor may be prompted to acquire a suitable data set based on given parameters. A programmable microprocessor also may prompt a user to select one or more data set options selected by the microprocessor based on given parameters. A programmable microprocessor may prompt a user to select one or more data set options selected by the microprocessor based on information found via the internet, other internal or external information, or the like. Options may be chosen for selecting one or more data feature selections, one or more statistical algorithms, one or more statistical analysis algorithms, one or more statistical significance algorithms, iterative steps, one or more validation algorithms, and one or more graphical representations of methods, machines, apparatuses, computer programs or a non-transitory computer-readable storage medium with an executable program stored thereon.

Systems addressed herein may comprise general components of computer systems, such as, for example, network servers, laptop systems, desktop systems, handheld systems, personal digital assistants, computing kiosks, and the like. A computer system may comprise one or more input means such as a keyboard, touch screen, mouse, voice recognition or other means to allow the user to enter data into the system. A system may further comprise one or more outputs, including, but not limited to, a display screen (e.g., CRT or LCD), speaker, FAX machine, printer (e.g., laser, ink jet, impact, black and white or color printer), or other output useful for providing visual, auditory and/or hardcopy output of information (e.g., outcome and/or report).

In a system, input and output means may be connected to a central processing unit which may comprise among other components, a microprocessor for executing program instructions and memory for storing program code and data. Processes may be implemented as a single user system located in a single geographical site. Processes may be implemented as a multi-user system. In the case of a multi-user implementation, multiple central processing units may be connected by means of a network. The network may be local, encompassing a single department in one portion of a building, an entire building, span multiple buildings, span a region, span an entire country or be worldwide. The network may be private, being owned and controlled by a provider, or it may be implemented as an internet based service where the user accesses a web page to enter and retrieve information. Accordingly, a system may include one or more machines, which may be local or remote with respect to a user. More than one machine in one location or multiple locations may be accessed by a user, and data may be mapped and/or processed in series and/or in parallel. Thus, a suitable configuration and control may be utilized for mapping and/or processing data using multiple machines, such as in local network, remote network and/or "cloud" computing platforms.

A system may include a communications interface. A communications interface allows for transfer of software and data between a computer system and one or more external devices. Non-limiting examples of communications interfaces include a modem, a network interface (such as an Ethernet card), a communications port, a PCMCIA slot and card, and the like. Software and data transferred via a communications interface generally are in the form of signals, which can be electronic, electromagnetic, optical and/or other signals capable of being received by a communications interface. Signals often are provided to a communications interface via a channel. A channel often carries signals and can be implemented using wire or cable, fiber optics, a phone line, a cellular phone link, an RF link and/or other communications channels. Thus, in an example, a communications interface may be used to receive signal information that can be detected by a signal detection module.

Data may be input by a suitable device and/or method, including, but not limited to, manual input devices or direct data entry devices (DDEs). Non-limiting examples of manual devices include keyboards, concept keyboards, touch sensitive screens, light pens, mouse, tracker balls, joysticks, graphic tablets, scanners, digital cameras, video digitizers and voice recognition devices. Non-limiting examples of DDEs include bar code readers, magnetic strip codes, smart cards, magnetic ink character recognition, optical character recognition, optical mark recognition, and turnaround documents.

Output from a sequencing apparatus or machine may serve as data that can be input via an input device. Mapped sequence reads may serve as data that can be input via an input device. Nucleic acid fragment size (e.g., length) may serve as data that can be input via an input device. Output from a nucleic acid capture process (e.g., genomic region origin data) may serve as data that can be input via an input device. A combination of nucleic acid fragment size (e.g., length) and output from a nucleic acid capture process (e.g., genomic region origin data) may serve as data that can be input via an input device. Simulated data may be generated by an in silico process and the simulated data serves as data that can be input via an input device. The term "in silico" refers to research and experiments performed using a computer. In silico processes include, but are not limited to, mapping sequence reads and processing mapped sequence reads according to processes described herein.

A system may include software useful for performing a process described herein, and software can include one or more modules for performing such processes (e.g., sequencing module, logic processing module, data display organization module). The term "software" refers to computer readable program instructions that, when executed by a computer, perform computer operations. Instructions executable by the one or more microprocessors sometimes are provided as executable code, that when executed, can cause one or more microprocessors to implement a method described herein. A module described herein can exist as software, and instructions (e.g., processes, routines, subroutines) embodied in the software can be implemented or performed by a microprocessor. For example, a module (e.g.,

a software module) can be a part of a program that performs a particular process or task. The term "module" refers to a self-contained functional unit that can be used in a larger machine or software system. A module can comprise a set of instructions for carrying out a function of the module. A module can transform data and/or information. Data and/or information can be in a suitable form. For example, data and/or information can be digital or analogue. Data and/or information sometimes may be packets, bytes, characters, or bits. Data and/or information may be any gathered, assembled or usable data or information. Non-limiting examples of data and/or information include a suitable media, pictures, video, sound (e.g. frequencies, audible or non-audible), numbers, constants, a value, objects, time, functions, instructions, maps, references, sequences, reads, mapped reads, levels, ranges, thresholds, signals, displays, representations, or transformations thereof. A module can accept or receive data and/or information, transform the data and/or information into a second form, and provide or transfer the second form to a machine, peripheral, component or another module. A module can perform one or more of the following non-limiting functions: mapping sequence reads, providing counts, assembling portions, providing or determining a level, providing a count profile, normalizing (e.g., normalizing reads, normalizing counts, and the like), providing a normalized count profile or levels of normalized counts, comparing two or more levels, providing uncertainty values, providing or determining expected levels and expected ranges(e.g., expected level ranges, threshold ranges and threshold levels), providing adjustments to levels (e.g., adjusting a first level, adjusting a second level, adjusting a profile of a chromosome or a segment thereof, and/or padding), providing identification (e.g., identifying a copy number variation, genetic variation or aneuploidy), categorizing, plotting, and/or determining an outcome, for example. A microprocessor may carry out the instructions in a module. One or more microprocessors may be required to carry out instructions in a module or group of modules. A module can provide data and/or information to another module, machine or source and can receive data and/or information from another module, machine or source.

A computer program product sometimes is embodied on a tangible computer-readable medium, and sometimes is tangibly embodied on a non-transitory computer-readable medium. A module sometimes is stored on a computer readable medium (e.g., disk, drive) or in memory (e.g., random access memory). A module and microprocessor capable of implementing instructions from a module can be located in a machine or in a different machine. A module and/or microprocessor capable of implementing an instruction for a module can be located in the same location as a user (e.g., local network) or in a different location from a user (e.g., remote network, cloud system). In aspects in which a method is carried out in conjunction with two or more modules, the modules may be located in the same machine, one or more modules may be located in different machine in the same physical location, and one or more modules may be located in different machines in different physical locations.

A machine, may comprise at least one microprocessor for carrying out the instructions in a module. Counts of sequence reads mapped to portions of a reference genome sometimes are accessed by a microprocessor that executes instructions configured to carry out a method described herein. Counts that are accessed by a microprocessor can be within memory of a system, and the counts can be accessed and placed into the memory of the system after they are obtained. A machine may include a microprocessor (e.g., one or more microprocessors) which microprocessor can perform and/or implement one or more instructions (e.g., processes, routines and/or subroutines) from a module. A machine may include multiple microprocessors, such as microprocessors coordinated and working in parallel. A machine may operate with one or more external microprocessors (e.g., an internal or external network, server, storage device and/or storage network (e.g., a cloud)). A machine may comprise a module. A machine may comprise one or more modules. A machine comprising a module often can receive and transfer one or more of data and/or information to and from other modules. A machine may comprise peripherals and/or components. A machine may comprise one or more peripherals or components that can transfer data and/or information to and from other modules, peripherals and/or components. A machine may interact with a peripheral and/or component that provides data and/or information. Peripherals and components may assist a machine in carrying out a function or interact directly with a module. Non-limiting examples of peripherals and/or components include a suitable computer peripheral, I/O or storage method or device including but not limited to scanners, printers, displays (e.g., monitors, LED, LCT or CRTs), cameras, microphones, pads (e.g., ipads, tablets), touch screens, smart phones, mobile phones, USB I/O devices, USB mass storage devices, keyboards, a computer mouse, digital pens, modems, hard drives, jump drives, flash drives, a microprocessor, a server, CDs, DVDs, graphic cards, specialized I/O devices (e.g., sequencers, photo cells, photo multiplier tubes, optical readers, sensors, etc.), one or more flow cells, fluid handling components, network interface controllers, ROM, RAM, wireless transfer methods and devices (Bluetooth, WiFi, and the like,), the world wide web (www), the internet, a computer and/or another module.

Software often is provided on a program product containing program instructions recorded on a computer readable medium, including, but not limited to, magnetic media including floppy disks, hard disks, and magnetic tape; and optical media including CD-ROM discs, DVD discs, magneto-optical discs, flash drives, RAM, floppy discs, the like, and other such media on which the program instructions can be recorded. In online implementation, a server and web site maintained by an organization can be configured to provide software downloads to remote users, or remote users may access a remote system maintained by an organization to remotely access software. Software may obtain or receive input information. Software may include a module that specifically obtains or receives data (e.g., a data receiving module that receives sequence read data and/or mapped read data) and may include a module that specifically processes the data (e.g., a processing module that processes received data (e.g., filters, normalizes, provides an outcome and/or report). The terms "obtaining" and "receiving" input information refers to receiving data (e.g., sequence reads, mapped reads) by computer communication means from a local, or remote site, human data entry, or any other method of receiving data. The input information may be generated in the same location at which it is received, or it may be generated in a different location and transmitted to the receiving location. Input information may be modified before it is processed (e.g., placed into a format amenable to processing (e.g., tabulated)).

Als described herein are computer program products, such as, for example, a computer program product comprising a computer usable medium having a computer readable program code described therein, the computer readable program code adapted to be executed to implement a method comprising: (a) generating a count of nucleic acid sequence reads for a genome segment, which sequence reads are reads of nucleic acid from a test sample from a subject having the genome, thereby providing a count $A$ for the segment; (b) generating a count of nucleic acid sequence reads for the genome or a subset of the genome, thereby providing a count $B$ for the genome or subset of the genome, where the count $B$ is a count of sequence reads not aligned to a reference genome; and (c) determining a count representation for the segment as a ratio of the count $A$ to the count $B$.

Software may include one or more algorithms. An algorithm may be used for processing data and/or providing an outcome or report according to a finite sequence of instructions. An algorithm often is a list of defined instructions for completing a task. Starting from an initial state, the instructions may describe a computation that proceeds through a defined series of successive states, eventually terminating in a final ending state. The transition from one state to the next is not necessarily deterministic (e.g., some algorithms incorporate randomness). By way of example, and without limitation, an algorithm can be a search algorithm, sorting algorithm, merge algorithm, numerical algorithm, graph algorithm, string algorithm, modeling algorithm, computational genometric algorithm, combinatorial algorithm, machine learning algorithm, cryptography algorithm, data compression algorithm, parsing algorithm and the like. An algorithm can include one algorithm or two or more algorithms working in combination. An algorithm can be of any suitable complexity class and/or parameterized complexity. An algorithm can be used for calculation and/or data processing, and may be used in a deterministic or probabilistic/predictive approach. An algorithm can be implemented in a computing environment by use of a suitable programming language, non-limiting examples of which are C, C++, Java, Perl, Python, Fortran, and the like. An algorithm may be configured or modified to include margin of errors, statistical analysis, statistical significance, and/or comparison to other information or data sets (e.g., applicable when using a neural net or clustering algorithm).

Several algorithms may be implemented for use in software. These algorithms may be trained with raw data. For each new raw data sample, the trained algorithms may produce a representative processed data set or outcome. A processed data set sometimes is of reduced complexity compared to the parent data set that was processed. Based on a processed set, the performance of a trained algorithm may be assessed based on sensitivity and specificity. An algorithm with the highest sensitivity and/or specificity may be identified and utilized.

Simulated (or simulation) data may aid data processing, for example, by training an algorithm or testing an algorithm. Simulated data may include hypothetical various samplings of different groupings of sequence reads. Simulated data may be based on what might be expected from a real population or may be skewed to test an algorithm and/or to assign a correct classification. Simulated data also is referred to herein as "virtual" data. Simulations may be performed by a computer program. One possible step in using a simulated data set is to evaluate the confidence of an identified result, e.g., how well a random sampling matches or best represents the original data. One approach is to calculate a probability value (p-value), which estimates the probability of a random sample having better score than the selected samples. An empirical model may be assessed, in which it is assumed that at least one sample matches a reference sample (with or without resolved variations). Another distribution, such as a Poisson distribution for example, may be used to define the probability distribution.

A system may include one or more microprocessors. A microprocessor can be connected to a communication bus. A computer system may include a main memory, often random access memory (RAM), and can also include a secondary memory. Memory may comprise a non-transitory computer-readable storage medium. Secondary memory can include, for example, a hard disk drive and/or a removable storage drive, representing a floppy disk drive, a magnetic tape drive, an optical disk drive, memory card and the like. A removable storage drive often reads from and/or writes to a removable storage unit. Non-limiting examples of removable storage units include a floppy disk, magnetic tape, optical disk, and the like, which can be read by and written to by, for example, a removable storage drive. A removable storage unit can include a computer-usable storage medium having stored therein computer software and/or data.

A microprocessor may implement software in a system. A microprocessor may be programmed to automatically perform a task described herein that a user could perform. Accordingly, a microprocessor, or algorithm conducted by such a microprocessor, can require little to no supervision or input from a user (e.g., software may be programmed to implement a function automatically). The complexity of a process may be so large that a single person or group of persons could not perform the process in a timeframe short enough for determining the presence or absence of a copy number variation.

Secondary memory may include other similar means for allowing computer programs or other instructions to be loaded into a computer system. For example, a system can include a removable storage unit and an interface device. Non-limiting examples of such systems include a program cartridge and cartridge interface (such as that found in video game devices), a removable memory chip (such as an EPROM, or PROM) and associated socket, and other removable storage units and interfaces that allow software and data to be transferred from the removable storage unit to a computer system.

One entity may generate counts of sequence reads, map the sequence reads to portions, count the mapped reads, and utilize the counted mapped reads in a method, system, machine, apparatus or computer program product described herein,. Counts of sequence reads mapped to portions sometimes may be transferred by one entity to a second entity for use by the second entity in a method, system, machine, apparatus or computer program product described herein.

One entity may generate sequence reads and a second entity may map those sequence reads to portions in a reference genome. The second entity sometimes counts the mapped reads and utilizes the counted mapped reads in a method, system, machine or computer program product described herein. The second entity may transfer the mapped reads to a third entity, and the third entity counts the mapped reads and utilizes the mapped reads in a method, system, machine or computer program product described herein. The second entity may count the mapped reads and transfers the counted mapped reads to a third entity, and the third entity may utilize the counted mapped reads in a method, system, machine or computer program product described herein. Involving a third entity, the third entity sometimes may be the same as the first entity. That is, the first entity sometimes transfers sequence reads to a second entity, which second entity can map sequence reads to portions in a reference genome and/or count the mapped reads, and the second entity can transfer the mapped and/or counted reads to a third entity. A third entity sometimes can utilize the mapped and/or counted reads in a method, system, machine or computer program product described herein, where the third entity sometimes is the same as the first entity, and sometimes the third entity is different from the first or second entity.

One entity may obtain blood from a pregnant female, optionally isolates nucleic acid from the blood (e.g., from the plasma or serum), and may transfer the blood or nucleic acid to a second entity that may generate sequence reads from the nucleic acid.

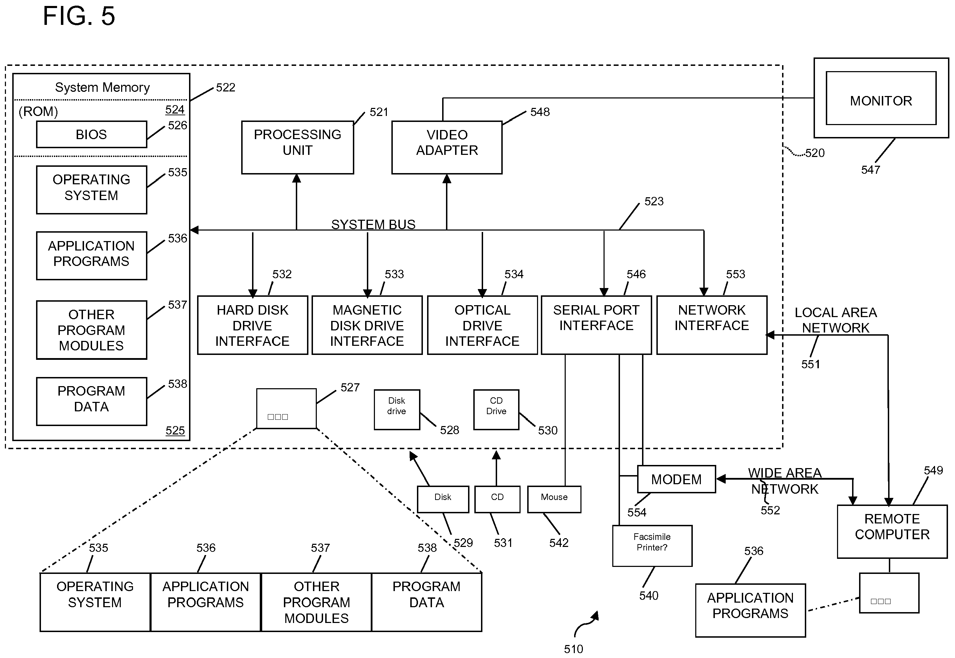

FIG. 5 illustrates a non-limiting example of a computing environment 510 in which various systems, methods, algorithms, and data structures described herein may be implemented. The computing environment 510 is only one example of a suitable computing environment and is not intended to suggest any limitation as to the scope of use or functionality of the systems, methods, and data structures described herein. Neither should computing environment 510 be interpreted as having any dependency or requirement relating to any one or combination of components illustrated in computing environment 510. A subset of systems, methods, and data structures shown in FIG. 5 may be utilized. Systems, methods, and data structures described herein are operational with numerous other general purpose or special purpose computing system environments or configurations. Examples of known computing systems, environments, and/or configurations that may be suitable include, but are not limited to, personal computers, server computers, thin clients, thick clients, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The operating environment 510 of FIG. 5 includes a general purpose computing device in the form of a computer 520, including a processing unit 521, a system memory 522, and a system bus 523 that operatively couples various system components including the system memory 522 to the processing unit 521. There may be only one or there may be more than one processing unit 521, such that the processor of computer 520 includes a single central-processing unit (CPU), or a plurality of processing units, commonly referred to as a parallel processing environment. The computer 520 may be a conventional computer, a distributed computer, or any other type of computer.

The system bus 523 may be any of several types of bus structures including a memory bus or memory controller, a peripheral bus, and a local bus using any of a variety of bus architectures. The system memory may also be referred to as simply the memory, and includes read only memory (ROM) 524 and random access memory (RAM). A basic input/output system (BIOS) 526, containing the basic routines that help to transfer information between elements within the computer 520, such as during start-up, is stored in ROM 524. The computer 520 may further include a hard disk drive interface 527 for reading from and writing to a hard disk, not shown, a magnetic disk drive 528 for reading from or writing to a removable magnetic disk 529, and an optical disk drive 530 for reading from or writing to a removable optical disk 531 such as a CD ROM or other optical media.

The hard disk drive 527, magnetic disk drive 528, and optical disk drive 530 are connected to the system bus 523 by a hard disk drive interface 532, a magnetic disk drive interface 533, and an optical disk drive interface 534, respectively. The drives and their associated computer-readable media provide nonvolatile storage of computer-readable instructions, data structures, program modules and other data for the computer 520. Any type of computer-readable media that can store data that is accessible by a computer, such as magnetic cassettes, flash memory cards, digital video disks, Bernoulli cartridges, random access memories (RAMs), read only memories (ROMs), and the like, may be used in the operating environment.

A number of program modules may be stored on the hard disk, magnetic disk 529, optical disk 531, ROM 524, or RAM, including an operating system 535, one or more application programs 536, other program modules 537, and program data 538. A user may enter commands and information into the personal computer 520 through input devices such as a keyboard 540 and pointing device 542. Other input devices (not shown) may include a microphone, joystick, game pad, satellite dish, scanner, or the like. These and other input devices are often connected to the processing unit 521 through a serial port interface 546 that is coupled to the system bus, but may be connected by other interfaces, such as a parallel port, game port, or a universal serial bus (USB). A monitor 547 or other type of display device is also connected to the system bus 523 via an interface, such as a video adapter 548. In addition to the monitor, computers typically include other peripheral output devices (not shown), such as speakers and printers.

The computer 520 may operate in a networked environment using logical connections to one or more remote computers, such as remote computer 549. These logical connections may be achieved by a communication device coupled to or a part of the computer 520, or in other manners. The remote computer 549 may be another computer, a server, a router, a network PC, a client, a peer device or other common network node, and typically includes many or all of the elements described above relative to the computer 520, although only a memory storage device 550 has been illustrated in FIG. 5. The logical connections depicted in FIG. 5 include a local-area network (LAN) 551 and a wide-area network (WAN) 552. Such networking environments are commonplace in office networks, enterprise-wide computer networks, intranets and the Internet, which all are types of networks.

When used in a LAN-networking environment, the computer 520 is connected to the local network 551 through a network interface or adapter 553, which is one type of communications device. When used in a WAN-networking environment, the computer 520 often includes a modem 554, a type of communications device, or any other type of communications device for establishing communications over the wide area network 552. The modem 554, which may be internal or external, is connected to the system bus 523 via the serial port interface 546. In a networked environment, program modules depicted relative to the personal computer 520, or portions thereof, may be stored in the remote memory storage device. It is appreciated that the network connections shown are non-limiting examples and other communications devices for establishing a communications link between computers may be used.

*Transformations*

As noted above, data sometimes is transformed from one form into another form. The terms "transformed", "transformation", and grammatical derivations or equivalents thereof, as used herein refer to an alteration of data from a physical starting material (e.g., test subject and/or reference subject sample nucleic acid) into a digital representation of the physical starting material (e.g., sequence read data), and may include a further transformation into one or more numerical values or graphical representations of the digital representation that can be utilized to provide an outcome (e.g., fetal fraction determination or estimation for a test sample). The one or more numerical values and/or graphical representations of digitally represented data may be utilized to represent the appearance of a test subject's physical genome (e.g., virtually represent or visually represent the presence or absence of a genomic insertion, duplication or deletion; represent the presence or absence of a variation in the physical amount of a sequence associated with medical conditions). A virtual representation sometimes is further transformed into one or more numerical values or graphical representations of the digital representation of the starting material. These methods can transform physical starting material into a numerical value or graphical representation, or a representation of the physical appearance of a test subject's genome.

Transformation of a data set may facilitate providing an outcome by reducing data complexity and/or data dimensionality. Data set complexity sometimes is reduced during the process of transforming a physical starting material into a virtual representation of the starting material (e.g., sequence reads representative of physical starting material). A suitable feature or variable can be utilized to reduce data set complexity and/or dimensionality. Non-limiting examples of features that can be chosen for use as a target feature for data processing include GC content, fetal gender prediction, fragment size (e.g., length of CCF fragments, reads or a suitable representation thereof (e.g., FRS)), fragment sequence, identification of chromosomal aneuploidy, identification of particular genes or proteins, identification of cancer, diseases, inherited genes/traits, chromosomal abnormalities, a biological category, a chemical category, a biochemical category, a category of genes or proteins, a gene ontology, a protein ontology, co-regulated genes, cell signaling genes, cell cycle genes, proteins pertaining to the foregoing genes, gene variants, protein variants, co-regulated genes, co-regulated proteins, amino acid sequence, nucleotide sequence, protein structure data and the like, and combinations of the foregoing. Non-limiting examples of data set complexity and/or dimensionality reduction include; reduction of a plurality of sequence reads to profile plots, reduction of a plurality of sequence reads to numerical values (e.g., normalized values, Z-scores, p-values); reduction of multiple analysis methods to probability plots or single points; principle component analysis of derived quantities; and the like or combinations thereof.

*Genetic Variations and Medical Conditions*

The presence or absence of a genetic variance can be determined using a method, machine or apparatus described herein. The presence or absence of one or more genetic variations may be determined according to an outcome provided by methods, machines and apparatuses described herein. A genetic variation generally is a particular genetic phenotype present in certain individuals, and often a genetic variation is present in a statistically significant sub-population of individuals. A genetic variation may be a chromosome abnormality (e.g., aneuploidy, duplication of one or more chromosomes, loss of one or more chromosomes), partial chromosome abnormality or mosaicism (e.g., loss or gain of one or more segments of a chromosome), translocations, inversions, each of which is described in greater detail herein. Non-limiting examples of genetic variations include one or more deletions (e.g., microdeletions), duplications (e.g., micro-duplications), insertions, mutations, polymorphisms (e.g., single-nucleotide polymorphisms), fusions, repeats (e.g., short tandem repeats), distinct methylation sites, distinct methylation patterns, the like and combinations thereof. An insertion, repeat, deletion, duplication, mutation or polymorphism can be of any length, and may be about 1 base or base pair (bp) to about 250 megabases (Mb) in length. An insertion, repeat, deletion, duplication, mutation or polymorphism may be about 1 base or base pair (bp) to about 50,000 kilobases (kb) in length (e.g., about 10 bp, 50 bp, 100 bp, 500 bp, 1 kb, 5 kb, 10kb, 50 kb, 100 kb, 500 kb, 1000 kb, 5000 kb or 10,000 kb in length).

A genetic variation is sometime a deletion. A deletion may be a mutation (e.g., a genetic aberration) in which a part of a chromosome or a sequence of DNA is missing. A deletion is often the loss of genetic material. Any number of nucleotides can be deleted. A deletion can comprise the deletion of one or more entire chromosomes, a segment of a chromosome, an allele, a gene, an intron, an exon, any non-coding region, any coding region, a segment thereof or combination thereof. A deletion can comprise a microdeletion. A deletion can comprise the deletion of a single base.

A genetic variation is sometimes a genetic duplication. A duplication may be a mutation (e.g., a genetic aberration) in which a part of a chromosome or a sequence of DNA is copied and inserted back into the genome. In A genetic duplication (e.g., duplication) may be any duplication of a region of DNA. A duplication may be a nucleic acid sequence that is repeated, often in tandem, within a genome or chromosome. A duplication can comprise a copy of one or more entire chromosomes, a segment of a chromosome, an allele, a gene, an intron, an exon, any non-coding region, any coding region, segment thereof or combination thereof. A duplication can comprise a microduplication. A duplication sometimes comprises one or more copies of a duplicated nucleic acid. A duplication sometimes is characterized as a genetic region repeated one or more times (e.g., repeated 1, 2, 3, 4, 5, 6, 7, 8, 9 or 10 times). Duplications can range from small regions (thousands of base pairs) to whole chromosomes in some instances. Duplications frequently occur as the result of an error in homologous recombination or due to a retrotransposon event. Duplications have been associated with certain types of proliferative diseases. Duplications can be characterized using genomic microarrays or comparative genetic hybridization (CGH).

A genetic variation is sometimes an insertion. An insertion is sometimes the addition of one or more nucleotide base pairs into a nucleic acid sequence. An insertion is sometimes a microinsertion. An insertion may comprise the addition of a segment of a chromosome into a genome, chromosome, or segment thereof. An insertion may comprise the addition of an allele, a gene, an intron, an exon, any non-coding region, any coding region, segment thereof or combination thereof into a genome or segment thereof. An insertion may comprise the addition (e.g., insertion) of nucleic acid of unknown origin into a genome, chromosome, or segment thereof. An insertion may comprise the addition (e.g., insertion) of a single base.

As used herein a "copy number variation" generally is a class or type of genetic variation or chromosomal aberration. A copy number variation can be a deletion (e.g., micro-deletion), duplication (e.g., a micro-duplication) or insertion (e.g., a micro-insertion). Often, the prefix "micro" as used herein sometimes is a segment of nucleic acid less than 5 Mb in length. A copy number variation can include one or more deletions (e.g., micro-deletion), duplications and/or insertions (e.g., a micro-duplication, micro-insertion) of a segment of a chromosome. A duplication may comprise an insertion. An insertion may be a duplication. An insertion may not be a duplication.

A copy number variation may be a fetal copy number variation. Often, a fetal copy number variation is a copy number variation in the genome of a fetus. A copy number variation may be a maternal and/or fetal copy number variation. A maternal and/or fetal copy number variation may be a copy number variation within the genome of a pregnant female (e.g., a female subject bearing a fetus), a female subject that gave birth or a female capable of bearing a fetus. A copy number variation can be a heterozygous copy number variation where the variation (e.g., a duplication or deletion) is present on one allele of a genome. A copy number variation can be a homozygous copy number variation where the variation is present on both alleles of a genome. A copy number variation may be a heterozygous or homozygous fetal copy number variation. A copy number variation may be a heterozygous or homozygous maternal and/or fetal copy number variation. A copy number variation sometimes is present in a maternal genome and a fetal genome, a maternal genome and not a fetal genome, or a fetal genome and not a maternal genome.

"Ploidy" is a reference to the number of chromosomes present in a fetus or mother. "Ploidy" may be the same as "chromosome ploidy". In humans, for example, autosomal chromosomes are often present in pairs. For example, in the absence of a genetic variation, most humans have two of each autosomal chromosome (e.g., chromosomes 1-22). The presence of the normal complement of 2 autosomal chromosomes in a human is often referred to as euploid or diploid. "Microploidy" is similar in meaning to ploidy. "Microploidy" often refers to the ploidy of a segment of a chromosome. The term "microploidy" sometimes is a reference to the presence or absence of a copy number variation (e.g., a deletion, duplication and/or an insertion) within a chromosome (e.g., a homozygous or heterozygous deletion, duplication, or insertion, the like or absence thereof).

The microploidy of a fetus may match the microploidy of the mother of the fetus (e.g., the pregnant female subject). The microploidy of a fetus may match the microploidy of the mother of the fetus and both the mother and fetus may carry the same heterozygous copy number variation, homozygous copy number variation or both may be euploid. The microploidy of a fetus may be different than the microploidy of the mother of the fetus. For example, sometimes the microploidy of a fetus is heterozygous for a copy number variation, the mother is homozygous for a copy number variation and the microploidy of the fetus does not match (e.g., does not equal) the microploidy of the mother for the specified copy number variation.

A genetic variation for which the presence or absence may be identified for a subject is associated with a medical condition. Thus, technology described herein can be used to identify the presence or absence of one or more genetic variations that are associated with a medical condition or medical state. Non-limiting examples of medical conditions include those associated with intellectual disability (e.g., Down Syndrome), aberrant cell-proliferation (e.g., cancer), presence of a micro-organism nucleic acid (e.g., virus, bacterium, fungus, yeast), and preeclampsia.

Non-limiting examples of genetic variations, medical conditions and states are described hereafter.

*Fetal Gender*

The prediction of a fetal gender or gender related disorder (e.g., sex chromosome aneuploidy) may be determined by a method, machine and/or apparatus described herein. Gender determination generally is based on a sex chromosome. In humans, there are two sex chromosomes, the X and Y chromosomes. The Y chromosome contains a gene, SRY, which triggers embryonic development as a male. The Y chromosomes of humans and other mammals also contain other genes needed for normal sperm production. Individuals with XX are female and XY are male and non-limiting variations, often referred to as sex chromosome aneuploidies, include X0, XYY, XXX and XXY. Males may have two X chromosomes and one Y chromosome (XXY; Klinefelter's Syndrome), or one X chromosome and two Y chromosomes (XYY syndrome; Jacobs Syndrome), and some females may have three X chromosomes (XXX; Triple X Syndrome) or a single X chromosome instead of two (X0; Turner Syndrome). Only a portion of cells in an individual may be affected by a sex chromosome aneuploidy which may be referred to as a mosaicism (e.g., Turner mosaicism). Other cases include those where SRY is damaged (leading to an XY female), or copied to the X (leading to an XX male).

It may be beneficial to determine the gender of a fetus in utero. For example, a patient (e.g., pregnant female) with a family history of one or more sex-linked disorders may wish to determine the gender of the fetus she is carrying to help assess the risk of the fetus inheriting such a disorder. Sex-linked disorders include, without limitation, X-linked and Y-linked disorders. X-linked disorders include X-linked recessive and X-linked dominant disorders. Examples of X-linked recessive disorders include, without limitation, immune disorders (e.g., chronic granulomatous disease (CYBB), Wiskott-Aldrich syndrome, X-linked severe combined immunodeficiency, X-linked agammaglobulinemia, hyper-IgM syndrome type 1, IPEX, X-linked lymphoproliferative disease, Properdin deficiency), hematologic disorders (e.g., Hemophilia A, Hemophilia B, X-linked sideroblastic anemia), endocrine disorders (e.g., androgen insensitivity syndrome/Kennedy disease, KAL1 Kallmann syndrome, X-linked adrenal hypoplasia congenital), metabolic disorders (e.g., ornithine transcarbamylase deficiency, oculocerebrorenal syndrome, adrenoleukodystrophy, glucose-6-phosphate dehydrogenase deficiency, pyruvate dehydrogenase deficiency, Danon disease/glycogen storage disease Type lib, Fabry's disease, Hunter syndrome, Lesch-Nyhan syndrome, Menkes disease/occipital horn syndrome), nervous system disorders (e.g., Coffin-Lowry syndrome, MASA syndrome, X-linked alpha thalassemia mental retardation syndrome, Siderius X-linked mental retardation syndrome, color blindness, ocular albinism, Norrie disease, choroideremia, Charcot-Marie-Tooth disease (CMTX2-3), Pelizaeus-Merzbacher disease, SMAX2), skin and related tissue disorders (e.g., dyskeratosis congenital, hypohidrotic ectodermal dysplasia (EDA), X-linked ichthyosis, X-linked endothelial corneal dystrophy), neuromuscular disorders (e.g., Becker's muscular dystrophy/Duchenne, centronuclear myopathy (MTM1), Conradi-Hünermann syndrome, Emery-Dreifuss muscular dystrophy 1), urologic disorders (e.g., Alport syndrome, Dent's disease, X-linked nephrogenic diabetes insipidus), bone/tooth disorders (e.g., AMELX Amelogenesis imperfecta), and other disorders (e.g., Barth syndrome, McLeod syndrome, Smith-Fineman-Myers syndrome, Simpson-Golabi-Behmel syndrome, Mohr-Tranebjærg syndrome, Nasodigitoacoustic syndrome). Examples of X-linked dominant disorders include, without limitation, X-linked hypophosphatemia, Focal dermal hypoplasia, Fragile X syndrome, Aicardi syndrome, Incontinentia pigmenti, Rett syndrome, CHILD syndrome, Lujan-Fryns syndrome, and Orofaciodigital syndrome 1. Examples of Y-linked disorders include, without limitation, male infertility, retinitis pigmentosa, and azoospermia.

*Chromosome Abnormalities*

The presence or absence of a fetal chromosome abnormality may be determined by using a method, machine and/or apparatus described herein. Chromosome abnormalities include, without limitation, a gain or loss of an entire chromosome or a region of a chromosome comprising one or more genes. Chromosome abnormalities include monosomies, trisomies, polysomies, loss of heterozygosity, translocations, deletions and/or duplications of one or more nucleotide sequences (e.g., one or more genes), including deletions and duplications caused by unbalanced translocations. The term "chromosomal abnormality" or "aneuploidy" as used herein refers to a deviation between the structure of the subject chromosome and a normal homologous chromosome. The term "normal" refers to the predominate karyotype or banding pattern found in healthy individuals of a particular species, for example, a euploid genome (e.g., diploid in humans, e.g., 46,XX or 46,XY). As different organisms have widely varying chromosome complements, the term "aneuploidy" does not refer to a particular number of chromosomes, but rather to the situation in which the chromosome content within a given cell or cells of an organism is abnormal. The term "aneuploidy" herein may refer to an imbalance of genetic material caused by a loss or gain of a whole chromosome, or part of a chromosome. An "aneuploidy" can refer to one or more deletions and/or insertions of a segment of a chromosome. The term "euploid" may refer to a normal complement of chromosomes.

The term "monosomy" as used herein refers to lack of one chromosome of the normal complement. Partial monosomy can occur in unbalanced translocations or deletions, in which only a segment of the chromosome is present in a single copy. Monosomy of sex chromosomes (45, X) causes Turner syndrome, for example. The term "disomy" refers to the presence of two copies of a chromosome. For organisms such as humans that have two copies of each chromosome (those that are diploid or "euploid"), disomy is the normal condition. For organisms that normally have three or more copies of each chromosome (those that are triploid or above), disomy is an aneuploid chromosome state. In uniparental disomy, both copies of a chromosome come from the same parent (with no contribution from the other parent).

The term "trisomy" as used herein refers to the presence of three copies, instead of two copies, of a particular chromosome. The presence of an extra chromosome 21, which is found in human Down syndrome, is referred to as "Trisomy 21." Trisomy 18 and Trisomy 13 are two other human autosomal trisomies. Trisomy of sex chromosomes can be seen in females (e.g., 47, XXX in Triple X Syndrome) or males (e.g., 47, XXY in Klinefelter's Syndrome; or 47,XYY in Jacobs Syndrome). A trisomy may be a duplication of most or all of an autosome. A trisomy may be a whole chromosome aneuploidy resulting in three instances (e.g., three copies) of a particular type of chromosome (e.g., instead of two instances (e.g., a pair) of a particular type of chromosome for a euploid).

The terms "tetrasomy" and "pentasomy" as used herein refer to the presence of four or five copies of a chromosome, respectively. Although rarely seen with autosomes, sex chromosome tetrasomy and pentasomy have been reported in humans, including XXXX, XXXY, XXYY, XYYY, XXXXX, XXXXY, XXXYY, XXYYY and XYYYY.

Chromosome abnormalities can be caused by a variety of mechanisms. Mechanisms include, but are not limited to (i) nondisjunction occurring as the result of a weakened mitotic checkpoint, (ii) inactive mitotic checkpoints causing non-disjunction at multiple chromosomes, (iii) merotelic attachment occurring when one kinetochore is attached to both mitotic spindle poles, (iv) a multipolar spindle forming when more than two spindle poles form, (v) a monopolar spindle forming when only a single spindle pole forms, and (vi) a tetraploid intermediate occurring as an end result of the monopolar spindle mechanism.

The terms "partial monosomy" and "partial trisomy" as used herein refer to an imbalance of genetic material caused by loss or gain of part of a chromosome. A partial monosomy or partial trisomy can result from an unbalanced translocation, where an individual carries a derivative chromosome formed through the breakage and fusion of two different chromosomes. In this situation, the individual would have three copies of part of one chromosome (two normal copies and the segment that exists on the derivative chromosome) and only one copy of part of the other chromosome involved in the derivative chromosome.

The term "mosaicism" as used herein refers to aneuploidy in some cells, but not all cells, of an organism. Certain chromosome abnormalities can exist as mosaic and non-mosaic chromosome abnormalities. For example, certain trisomy 21 individuals have mosaic Down syndrome and some have non-mosaic Down syndrome. Different mechanisms can lead to mosaicism. For example, (i) an initial zygote may have three 21st chromosomes, which normally would result in simple trisomy 21, but during the course of cell division one or more cell lines lost one of the 21st chromosomes; and (ii) an initial zygote may have two 21st chromosomes, but during the course of cell division one of the 21st chromosomes were duplicated. Somatic mosaicism likely occurs through mechanisms distinct from those typically associated with genetic syndromes involving complete or mosaic aneuploidy. Somatic mosaicism has been identified in certain types of cancers and in neurons, for example. In certain instances, trisomy 12 has been identified in chronic lymphocytic leukemia (CLL) and trisomy 8 has been identified in acute myeloid leukemia (AML). Also, genetic syndromes in which an individual is predisposed to breakage of chromosomes (chromosome instability syndromes) are frequently associated with increased risk for various types of cancer, thus highlighting the role of somatic aneuploidy in carcinogenesis. Methods and protocols described herein can identify presence or absence of non-mosaic and mosaic chromosome abnormalities.

Tables 1A and 1B present a non-limiting list of chromosome conditions, syndromes and/or abnormalities that can be potentially identified by methods, machines and/or an apparatus described herein. Table 1B is from the DECIPHER database as of October 6, 2011 (e.g., version 5.1, based on positions mapped to GRCh37; available at uniform resource locator (URL) dechipher.sanger.ac.uk).

Table 1A

| Chromosome | Abnormality | Disease Association |
|---|---|---|
| X | XO | Turner's Syndrome |
| Y | XXY | Klinefelter syndrome |
| Y | XYY | Double Y syndrome |
| Y | XXX | Trisomy X syndrome |
| Y | XXXX | Four X syndrome |
| Y | Xp21 deletion | Duchenne's/Becker syndrome, congenital adrenal hypoplasia, chronic granulomatus disease |
| Y | Xp22 deletion | steroid sulfatase deficiency |
| Y | Xq26 deletion | X-linked lymphoproliferative disease |
| 1 | 1 p (somatic) monosomy trisomy | neuroblastoma |
| 2 | monosomy trisomy 2q | growth retardation, developmental and mental delay, and minor physical abnormalities |
| 3 | monosomy trisomy (somatic) | Non-Hodgkin's lymphoma |
| 4 | monosomy trisomy (somatic) | Acute non lymphocytic leukemia (ANLL) |
| 5 | 5p | Cri du chat; Lejeune syndrome |
| 5 | 5q (somatic) monosomy trisomy | myelodysplastic syndrome |
| 6 | monosomy trisomy (somatic) | clear-cell sarcoma |
| 7 | 7q11.23 deletion | William's syndrome |
| 7 | monosomy trisomy | monosomy 7 syndrome of childhood; somatic: renal cortical adenomas; myelodysplastic syndrome |
| 8 | 8q24.1 deletion | Langer-Giedon syndrome |
| 8 | monosomy trisomy | myelodysplastic syndrome; Warkany syndrome; somatic: chronic myelogenous leukemia |
| 9 | monosomy 9p | Alfi's syndrome |
| 9 | monosomy 9p partial trisomy | Rethore syndrome |
| 9 | trisomy | complete trisomy 9 syndrome; mosaic trisomy 9 syndrome |
| 10 | Monosomy trisomy (somatic) | ALL or ANLL |
| 11 | 11p- | Aniridia; Wilms tumor |
| 11 | 11q- | Jacobsen Syndrome |

(continued)

| Chromosome | Abnormality | Disease Association |
|---|---|---|
| 11 | monosomy (somatic) trisomy | myeloid lineages affected (ANLL, MDS) |
| 12 | monosomy trisomy (somatic) | CLL, Juvenile granulosa cell tumor (JGCT) |
| 13 | 13q- | 13q-syndrome; Orbeli syndrome |
| 13 | 13q14 deletion | retinoblastoma |
| 13 | monosomy trisomy | Patau's syndrome |
| 14 | monosomy trisomy (somatic) | myeloid disorders (MDS, ANLL, atypical CML) |
| 15 | 15q11-q13 deletion monosomy | Prader-Willi, Angelman's syndrome |
| 15 | trisomy (somatic) | myeloid and lymphoid lineages affected, e.g., MDS, ANLL, ALL, CLL) |
| 16 | trisomy | Full Trisomy 16 Mosaic Trisomy 16 |
| 16 | 16q13.3 deletion | Rubenstein-Taybi |
| 3 | monosomy trisomy (somatic) | papillary renal cell carcinomas (malignant) |
| 17 | 17p-(somatic) | 17p syndrome in myeloid malignancies |
| 17 | 17q11.2 deletion | Smith-Magenis |
| 17 | 17q13.3 | Miller-Dieker |
| 17 | monosomy trisomy (somatic) | renal cortical adenomas |
| 17 | 17p11.2-12 trisomy | Charcot-Marie Tooth Syndrome type 1; HNPP |
| 18 | 18p- | 18p partial monosomy syndrome or Grouchy Lamy Thieffry syndrome |
| 18 | 18q- | Grouchy Lamy Salmon Landry Syndrome |
| 18 | monosomy trisomy | Edwards Syndrome |
| 19 | monosomy trisomy | |
| 20 | 20p- | trisomy 20p syndrome |
| 20 | 20p11.2-12 deletion | Alagille |
| 20 | 20q- | somatic: MDS, ANLL, polycythemia vera, chronic neutrophilic leukemia |
| 20 | monosomy trisomy (somatic) | papillary renal cell carcinomas (malignant) |
| 21 | monosomy trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
| 22 | monosomy trisomy | complete trisomy 22 syndrome |

Table 1B

| Syndrome | Chromosome | Start | End | Interval (Mb) | Grade |
|---|---|---|---|---|---|
| 12q14 microdeletion syndrome | 12 | 65,071,919 | 68,645,525 | 3.57 | |
| 15q13.3 microdeletion syndrome | 15 | 30,769,995 | 32,701,482 | 1.93 | |
| 15q24 recurrent microdeletion syndrome | 15 | 74,377,174 | 76,162,277 | 1.79 | |
| 15q26 overgrowth syndrome | 15 | 99,357,970 | 102,521,392 | 3.16 | |
| 16p11.2 microduplication syndrome | 16 | 29,501,198 | 30,202,572 | 0.70 | |
| 16p11.2-p12.2 microdeletion syndrome | 16 | 21,613,956 | 29,042,192 | 7.43 | |
| 16p13.11 recurrent microdeletion (neurocognitive disorder susceptibility locus) | 16 | 15,504,454 | 16,284,248 | 0.78 | |
| 16p13.11 recurrent microduplication (neurocognitive disorder susceptibility locus) | 16 | 15,504,454 | 16,284,248 | 0.78 | |
| 17q21.3 recurrent microdeletion syndrome | 17 | 43,632,466 | 44,210,205 | 0.58 | 1 |
| 1p36 microdeletion syndrome | 1 | 10,001 | 5,408,761 | 5.40 | 1 |
| 1q21.1 recurrent microdeletion (susceptibility locus for neurodevelopmental disorders) | 1 | 146,512,930 | 147,737,500 | 1.22 | 3 |
| 1q21.1 recurrent microduplication (possible susceptibility locus for neurodevelopmental disorders) | 1 | 146,512,930 | 147,737,500 | 1.22 | 3 |
| 1q21.1 susceptibility locus for Thrombocytopenia-Absent Radius (TAR) syndrome | 1 | 145,401,253 | 145,928,123 | 0.53 | 3 |
| 22q11 deletion syndrome (Velocardiofacial / DiGeorge syndrome) | 22 | 18,546,349 | 22,336,469 | 3.79 | 1 |
| 22q11 duplication syndrome | 22 | 18,546,349 | 22,336,469 | 3.79 | 3 |
| 22q11.2 distal deletion syndrome | 22 | 22,115,848 | 23,696,229 | 1.58 | |
| 22q13 deletion syndrome (Phelan-Mcdermid syndrome) | 22 | 51,045,516 | 51,187,844 | 0.14 | 1 |
| 2p15-16.1 microdeletion syndrome | 2 | 57,741,796 | 61,738,334 | 4.00 | |
| 2q33.1 deletion syndrome | 2 | 196,925,089 | 205,206,940 | 8.28 | 1 |
| 2q37 monosomy | 2 | 239,954,693 | 243,102,476 | 3.15 | 1 |
| 3q29 microdeletion syndrome | 3 | 195,672,229 | 197,497,869 | 1.83 | |
| 3q29 microduplication syndrome | 3 | 195,672,229 | 197,497,869 | 1.83 | |
| 7q11.23 duplication syndrome | 7 | 72,332,743 | 74,616,901 | 2.28 | |
| 8p23.1 deletion syndrome | 8 | 8,119,295 | 11,765,719 | 3.65 | |
| 9q subtelomeric deletion syndrome | 9 | 140,403,363 | 141,153,431 | 0.75 | 1 |
| Adult-onset autosomal dominant leukodystrophy (ADLD) | 5 | 126,063,045 | 126,204,952 | 0.14 | |
| Angelman syndrome (Type 1) | 15 | 22,876,632 | 28,557,186 | 5.68 | 1 |

(continued)

| Syndrome | Chromosome | Start | End | Interval (Mb) | Grade |
|---|---|---|---|---|---|
| Angelman syndrome (Type 2) | 15 | 23,758,390 | 28,557,186 | 4.80 | 1 |
| ATR-16 syndrome | 16 | 60,001 | 834,372 | 0.77 | 1 |
| AZFa | Y | 14,352,761 | 15,154,862 | 0.80 | |
| AZFb | Y | 20,118,045 | 26,065,197 | 5.95 | |
| AZFb+AZFc | Y | 19,964,826 | 27,793,830 | 7.83 | |
| AZFc | Y | 24,977,425 | 28,033,929 | 3.06 | |
| Cat-Eye Syndrome (Type I) | 22 | 1 | 16,971,860 | 16.97 | |
| Charcot-Marie-Tooth syndrome type 1A (CMT1A) | 17 | 13,968,607 | 15,434,038 | 1.47 | 1 |
| Cri du Chat Syndrome (5p deletion | 5 | 10,001 | 11,723,854 | 11.71 | 1 |
| Early-onset Alzheimer disease with cerebral amyloid angiopathy | 21 | 27,037,956 | 27,548,479 | 0.51 | |
| Familial Adenomatous Polyposis | 5 | 112,101,596 | 112,221,377 | 0.12 | |
| Hereditary Liability to Pressure Palsies (HNPP) | 17 | 13,968,607 | 15,434,038 | 1.47 | 1 |
| Leri-Weill dyschondrostosis (LWD) - SHOX deletion | X | 751,878 | 867,875 | 0.12 | |
| Leri-Weill dyschondrostosis (LWD) - SHOX deletion | X | 460,558 | 753,877 | 0.29 | |
| Miller-Dieker syndrome (MDS) | 17 | 1 | 2,545,429 | 2.55 | 1 |
| NF1-microdeletion syndrome | 17 | 29,162,822 | 30,218,667 | 1.06 | 1 |
| Pelizaeus-Merzbacher disease | X | 102,642,051 | 103,131,767 | 0.49 | |
| Potocki-Lupski syndrome (17p11.2 duplication syndrome) | 17 | 16,706,021 | 20,482,061 | 3.78 | |
| Potocki-Shaffer syndrome | 11 | 43,985,277 | 46,064,560 | 2.08 | 1 |
| Prader-Willi syndrome (Type 1) | 15 | 22,876,632 | 28,557,186 | 5.68 | 1 |
| Prader-Willi Syndrome (Type 2) | 15 | 23,758,390 | 28,557,186 | 4.80 | 1 |
| RCAD (renal cysts and diabetes) | 17 | 34,907,366 | 36,076,803 | 1.17 | |
| Rubinstein-Taybi Syndrome | 16 | 3,781,464 | 3,861,246 | 0.08 | 1 |
| Smith-Magenis Syndrome | 17 | 16,706,021 | 20,482,061 | 3.78 | 1 |
| Sotos syndrome | 5 | 175,130,402 | 177,456,545 | 2.33 | 1 |
| Split hand/foot malformation 1 (SHFM1) | 7 | 95,533,860 | 96,779,486 | 1.25 | |
| Steroid sulphatase deficiency (STS) | X | 6,441,957 | 8,167,697 | 1.73 | |
| WAGR 11p13 deletion syndrome | 11 | 31,803,509 | 32,510,988 | 0.71 | |
| Williams-Beuren Syndrome (WBS) | 7 | 72,332,743 | 74,616,901 | 2.28 | 1 |
| Wolf-Hirschhorn Syndrome | 4 | 10,001 | 2,073,670 | 2.06 | 1 |
| Xq28 (MECP2) duplication | X | 152,749,900 | 153,390,999 | 0.64 | |

Grade 1 conditions often have one or more of the following characteristics; pathogenic anomaly; strong agreement amongst geneticists; highly penetrant; may still have variable phenotype but some common features; all cases in the literature have a clinical phenotype; no cases of healthy individuals with the anomaly; not reported on DVG databases or found in healthy population; functional data confirming single gene or multi-gene dosage effect; confirmed or strong candidate genes; clinical management implications defined; known cancer risk with implication for surveillance; multiple sources of information (OMIM, Gene reviews, Orphanet, Unique, Wikipedia); and/or available for diagnostic use (reproductive counseling).

Grade 2 conditions often have one or more of the following characteristics; likely pathogenic anomaly; highly penetrant; variable phenotype with no consistent features other than DD; small number of cases/ reports in the literature; all reported cases have a clinical phenotype; no functional data or confirmed pathogenic genes; multiple sources of information (OMIM, Gene reviews, Orphanet, Unique, Wikipedia); and/or may be used for diagnostic purposes and reproductive counseling.

Grade 3 conditions often have one or more of the following characteristics; susceptibility locus; healthy individuals or unaffected parents of a proband described; present in control populations; non penetrant; phenotype mild and not specific; features less consistent; no functional data or confirmed pathogenic genes; more limited sources of data; possibility of second diagnosis remains a possibility for cases deviating from the majority or if novel clinical finding present; and/or caution when using for diagnostic purposes and guarded advice for reproductive counseling.

*Medical disorders and medical conditions*

Methods described herein can be applicable to any suitable medical disorder or medical condition. Non-limiting examples of medical disorders and medical conditions include cell proliferative disorders and conditions, wasting disorders and conditions, degenerative disorders and conditions, autoimmune disorders and conditions, pre-eclampsia, chemical or environmental toxicity, liver damage or disease, kidney damage or disease, vascular disease, high blood pressure, and myocardial infarction.

A cell proliferative disorder or condition may be a cancer of the liver, lung, spleen, pancreas, colon, skin, bladder, eye, brain, esophagus, head, neck, ovary, testes, prostate, the like or combination thereof. Non-limiting examples of cancers include hematopoietic neoplastic disorders, which are diseases involving hyperplastic/neoplastic cells of hematopoietic origin (e.g., arising from myeloid, lymphoid or erythroid lineages, or precursor cells thereof), and can arise from poorly differentiated acute leukemias (e.g., erythroblastic leukemia and acute megakaryoblastic leukemia). Certain myeloid disorders include, but are not limited to, acute promyeloid leukemia (APML), acute myelogenous leukemia (AML) and chronic myelogenous leukemia (CML). Certain lymphoid malignancies include, but are not limited to, acute lymphoblastic leukemia (ALL), which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Certain forms of malignant lymphomas include, but are not limited to, non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease. A cell proliferative disorder sometimes is a nonendocrine tumor or endocrine tumor. Illustrative examples of non-endocrine tumors include, but are not limited to, adenocarcinomas, acinar cell carcinomas, adenosquamous carcinomas, giant cell tumors, intraductal papillary mucinous neoplasms, mucinous cystadenocarcinomas, pancreatoblastomas, serous cystadenomas, solid and pseudopapillary tumors. An endocrine tumor sometimes is an islet cell tumor.

A wasting disorder or condition, or degenerative disorder or condition may be cirrhosis, amyotrophic lateral sclerosis (ALS), Alzheimer's disease, Parkinson's disease, multiple system atrophy, atherosclerosis, progressive supranuclear palsy, Tay-Sachs disease, diabetes, heart disease, keratoconus, inflammatory bowel disease (IBD), prostatitis, osteoarthritis, osteoporosis, rheumatoid arthritis, Huntington's disease, chronic traumatic encephalopathy, chronic obstructive pulmonary disease (COPD), tuberculosis, chronic diarrhea, acquired immune deficiency syndrome (AIDS), superior mesenteric artery syndrome, the like or combination thereof.

An autoimmune disorder or condition may be acute disseminated encephalomyelitis (ADEM), Addison's disease, alopecia areata, ankylosing spondylitis, antiphospholipid antibody syndrome (APS), autoimmune hemolytic anemia, autoimmune hepatitis, autoimmune inner ear disease, bullous pemphigoid, celiac disease, Chagas disease, chronic obstructive pulmonary disease, Crohns Disease (a type of idiopathic inflammatory bowel disease "IBD"), dermatomyositis, diabetes mellitus type 1, endometriosis, Goodpasture's syndrome, Graves' disease, Guillain-Barre syndrome (GBS), Hashimoto's disease, hidradenitis suppurativa, idiopathic thrombocytopenic purpura, interstitial cystitis, Lupus erythematosus, mixed connective tissue disease, morphea, multiple sclerosis (MS), myasthenia gravis, narcolepsy, euromyotonia, pemphigus vulgaris, pernicious anaemia, polymyositis, primary biliary cirrhosis, rheumatoid arthritis, schizophrenia, scleroderma, Sjögren's syndrome, temporal arteritis (also known as "giant cell arteritis"), ulcerative colitis (a type of idiopathic inflammatory bowel disease "IBD"), vasculitis, vitiligo, Wegener's granulomatosis, the like or combination thereof.

*Cancers*

The presence or absence of an abnormal cell proliferation condition (e.g., cancer, tumor, neoplasm) may be determined by using a method or apparatus described herein. For example, levels of cell-free nucleic acid in serum can be elevated in patients with various types of cancer compared with healthy patients. Patients with metastatic diseases, for example, can sometimes have serum DNA levels approximately twice as high as non-metastatic patients. Patients with metastatic diseases may also be identified by cancer-specific markers and/or certain single nucleotide polymorphisms or short tandem repeats, for example. Non-limiting examples of cancer types that may be positively correlated with elevated levels of circulating DNA include breast cancer, colorectal cancer, gastrointestinal cancer, hepatocellular cancer, lung cancer, melanoma, non-Hodgkin lymphoma, leukemia, multiple myeloma, bladder cancer, hepatoma, cervical cancer, esophageal cancer, pancreatic cancer, and prostate cancer. Various cancers can possess, and can sometimes release into the bloodstream, nucleic acids with characteristics that are distinguishable from nucleic acids from non-cancerous healthy cells, such as, for example, epigenetic state and/or sequence variations, duplications and/or deletions. Such characteristics can, for example, be specific to a particular type of cancer. Thus, it is further contemplated that a method provided herein can be used to identify a particular type of cancer.

*Preeclampsia*

The presence or absence of preeclampsia may be determined by using a method, machine or apparatus described herein. Preeclampsia is a condition in which hypertension arises in pregnancy (e.g., pregnancy-induced hypertension) and is associated with significant amounts of protein in the urine. Preeclampsia also may be associated with elevated levels of extracellular nucleic acid and/or alterations in methylation patterns. For example, a positive correlation between extracellular fetal-derived hypermethylated RASSF1A levels and the severity of pre-eclampsia has been observed. In certain examples, increased DNA methylation is observed for the H19 gene in preeclamptic placentas compared to normal controls.

Preeclampsia is one of the leading causes of maternal and fetal/neonatal mortality and morbidity worldwide. Circulating cell-free nucleic acids in plasma and serum are novel biomarkers with promising clinical applications in different medical fields, including prenatal diagnosis. Quantitative changes of cell-free fetal (cff)DNA in maternal plasma as an indicator for impending preeclampsia have been reported in different studies, for example, using real-time quantitative PCR for the male-specific SRY or DYS 14 loci. In cases of early onset preeclampsia, elevated levels may be seen in the first trimester. The increased levels of cffDNA before the onset of symptoms may be due to hypoxia/reoxygenation within the intervillous space leading to tissue oxidative stress and increased placental apoptosis and necrosis. In addition to the evidence for increased shedding of cffDNA into the maternal circulation, there is also evidence for reduced renal clearance of cffDNA in preeclampsia. As the amount of fetal DNA is currently determined by quantifying Y-chromosome specific sequences, alternative approaches such as measurement of total cell-free DNA or the use of gender-independent fetal epigenetic markers, such as DNA methylation, offer an alternative. Cell-free RNA of placental origin is another alternative biomarker that may be used for screening and diagnosing preeclampsia in clinical practice. Fetal RNA is associated with subcellular placental particles that protect it from degradation. Fetal RNA levels sometimes are tenfold higher in pregnant females with preeclampsia compared to controls, and therefore is an alternative biomarker that may be used for screening and diagnosing preeclampsia in clinical practice.

*Pathogens*

The presence or absence of a pathogenic condition may be determined by a method, machine or apparatus described herein. A pathogenic condition can be caused by infection of a host by a pathogen including, but not limited to, a bacterium, virus or fungus. Since pathogens typically possess nucleic acid (e.g., genomic DNA, genomic RNA, mRNA) that can be distinguishable from host nucleic acid, methods, machines and apparatus provided herein can be used to determine the presence or absence of a pathogen. Often, pathogens possess nucleic acid with characteristics unique to a particular pathogen such as, for example, epigenetic state and/or one or more sequence variations, duplications and/or deletions. Thus, methods provided herein may be used to identify a particular pathogen or pathogen variant (e.g., strain).

Examples

The examples set forth below illustrate certain embodiments and do not limit the technology.

*Example 1: Chromosome count normalization features not requiring an alignment*

Methods described in this example provide an alternative way of calculating chromosome representation as pertaining to whole-genome sequencing analyses, without using multiple chromosomes in the normalization. Various types of molecular diagnostics, such as non-invasive prenatal diagnostics, rely on comparing standardized values of genomic representation of a sample of interest to a pre-established cutoff. In some instances, this genomic representation is derived from whole-genome sequencing experiments, where the sequenced reads are first aligned to a reference genome. For some sequencing platforms, there is significant variability in the total number of sequencing reads as function of the experimental conditions themselves and not as an intrinsic biological property in itself. For this reason, often the genomic representation involves a normalization step where reads aligned to a certain region are divided by reads aligned to other regions (which might also include the very region of interest). For example, in the MaterniT21 test (Sequenom, Inc., San Diego, California), the chromosome representation is calculated as the ratio between reads aligned on a chromosome of interest versus the reads aligned on all autosomes. The various types of ratios that can be constructed in this normalization step might be of various relevance to the overall accuracy of the diagnostics derived from these ratios. To date, such ratios have been calculated based on aligned reads (using various sequence alignment tools and reference genomes).

Described hereafter are ways of inferring chromosome representation in the absence of a classical alignment step with respect to a generic reference genome.

a. Chromosome representation defined as the ratio between reads aligned to a chromosome of interest (e.g., chr 21) and the number of sequencing reads (prior to any alignment).

b. Chromosome representation defined as the ratio between reads aligned to a chromosome of interest (e.g., chr 21) and the number of sequencing reads (prior to any alignment), as filtered by any quality control metric (e.g., reads which pass the chastity filter)

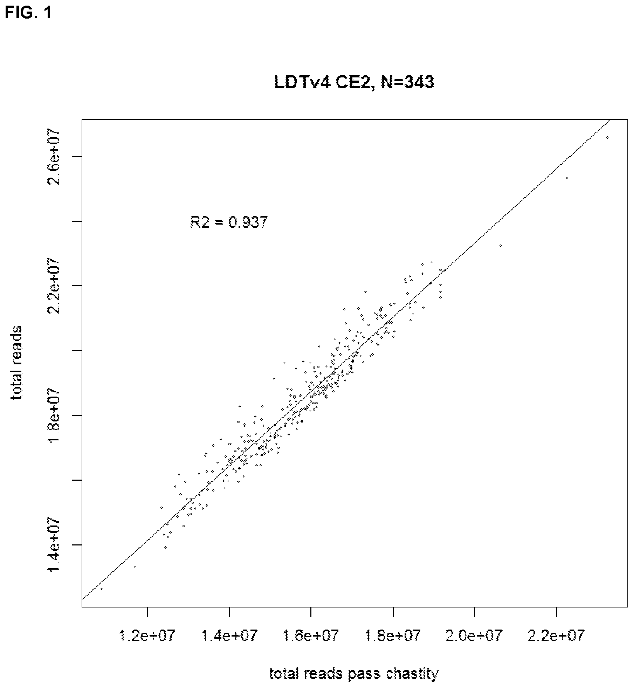

FIG. 1 shows a comparison between the total number of reads (prior to alignment) and total number of reads (prior to alignment) which pass the chastity filtered, as observed in a recent study (LDTv4CE2).

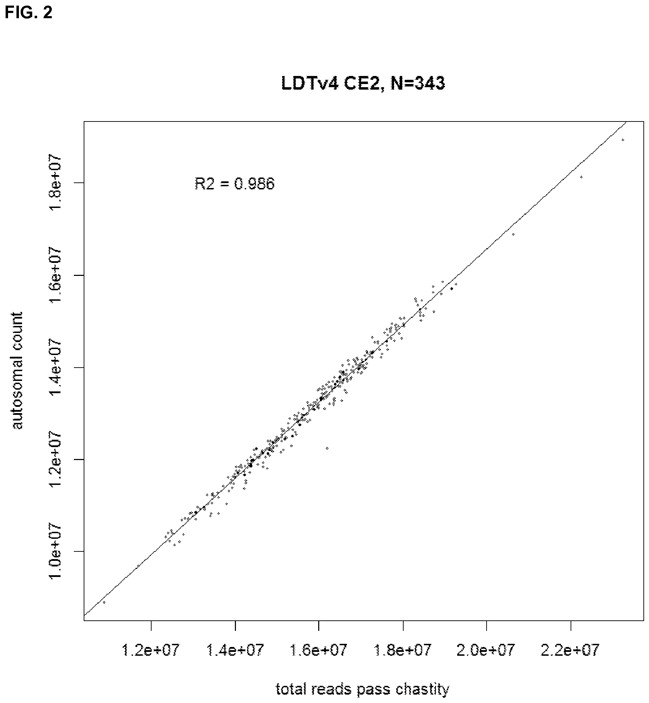

FIG. 2 shows a comparison between the total number of reads (prior to alignment) which pass the chastity filtered and the reads which are aligned to all autosomes, as observed in a recent study (LDTv4CE2).

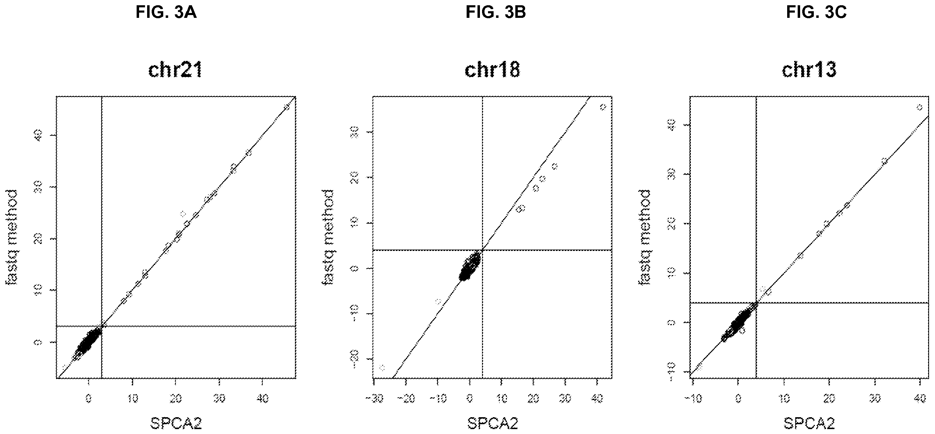

FIG. 3A, FIG. 3B and FIG. 3C show a comparison of z-scores derived from the chromosome representation calculated using autosomes and calculated using pre-alignment reads, passing chastity-filter, using a GC-LOESS normalization followed by a principal component normalization, for chromosomes 21, 13, and 18.

The accuracy of aneuploidy detection as determined based on chromosome representation calculated with pass-filtered pre-alignment reads is shown in Tables 2 through 4 below and was found to be identical to the accuracy from the LDTv4CE2 study.

TABLE 2

|  |  | TRUTH |  |
| --- | --- | --- | --- |
|  |  | T21 | EUPLOID |
| LDTv4 | T21 | 21 | 0 |
|  | EUPLOID | 0 | 313 |
|  | TOTAL | 21 | 313 |

TABLE 3

|  |  | TRUTH |  |
| --- | --- | --- | --- |
|  |  | T18 | EUPLOID |
| LDTv4 | T18 | 6 | 0 |
|  | EUPLOID | 1 | 328 |
|  | TOTAL | 7 | 328 |

TABLE 4

| | TRUTH | | |
|---|---|---|---|
| | | T13 | EUPLOID |
| LDTv4 | T13 | 7 | 0 |
| | EUPLOID | 0 | 328 |
| | TOTAL | 7 | 328 |

*Example 2: Further chromosome count normalization features not requiring an alignment*

As alternatives to the methods described in Example 1, also described hereafter are methods of inferring chromosome representation in the absence of a classical alignment step with respect to a generic reference genome. Some of these methods provide alternative ways of calculating chromosome representation without requiring that aligned reads are used for both the numerator and the denominator.

a. Chromosome representation defined as the ratio between a subset of reads aligned to a chromosome of interest (e.g., chr 21) and the number of sequencing reads (prior to any alignment) from a given subset, filtered or not by any quality control metric (e.g., reads which pass the chastity filter)
b. Chromosome representation defined as the ratio between a subset of reads aligned to a chromosome of interest (e.g., chr 21) and the number of sequencing reads (prior to any alignment) from a given subset, filtered by nucleotide composition (e.g., reads with GC content within a specified range).
c. Chromosome representation defined as the ratio between a subset of reads which match a custom dictionary of reads (obtained from previously sequenced samples and previously aligned to a chromosome of interest) and any of the variables defined in the above a -d.
d. Chromosome representation defined as the ratio of reads either aligned to a chromosome of interest or matching a custom dictionary and the reads which are not aligned to a subset of a reference genome ("unalignable").

FIG. 4 shows an example of a method making use of a custom dictionary described in (c) and (d) above for generating a count A (referred to as Ntarget, 480). As shown in FIG. 4, the number of reads for the denominator, Ntot, is generated by obtaining raw files for reads from a sequencer (410). The process includes converting the files to individual FASTQ files for each test sample (430), and counting the total number of reads for the test sample less reads filtered out according to a chastity filter (image quality filter, 440) to generate the Ntot count. Other filters can be used in place of, or in addition to, the chastity filter. For example, a filter based on GC percentage (e.g., GC percentage between 30% and 60%) can be used to filter the reads (440). Also, a filter that removes low complexity reads (e.g., reads with more than 50% repeats) can be used to filter the reads (440).

As shown in FIG. 4, reads from a reference sample or set of reference samples are aligned to a human reference genome (450) and a dictionary of reads (sub-listing) is prepared for each chromosome. Each of the dictionaries contains reads (polynucleotides; k-mers) uniquely mapped to the particular chromosome for which the dictionary is generated (460). A dictionary for a chromosome of interest is selected for a target chromosome, reads from the test sample (430) are compared to the polynucleotides in the dictionary (470) and reads that match polynucleotides in the dictionary are counted (Ntarget numerator, 480). The comparison (470) generally does not return the mapped position of each read, and gives a binary result as to whether a read belongs to the target chromosome or not. The Ntot count is utilized as the denominator and the Ntarget count is utilized as the numerator for a count representation (chromosome fraction, normalized chromosome count) determination for a target chromosome (490).

*Example 3: Examples of certain items*

Also described herein are the following items.

A1. A method for determining a sequence read count representation of a genome segment for a diagnostic test, comprising:

(a) generating a count of nucleic acid sequence reads for a genome segment, which sequence reads are reads of nucleic acid from a test sample from a subject having the genome, thereby providing a count A for the segment;
(b) generating a count of nucleic acid sequence reads for the genome or a subset of the genome, thereby providing a count B for the genome or subset of the genome, wherein the count B is a count of sequence reads not aligned to a reference genome; and
(c) determining a count representation for the segment as a ratio of the count A to the count B.

A1.1. The method of item A1, wherein the subset of the genome in (b) is larger than the segment in (a).

A1.2. The method of item A1 or A1.1, wherein the count B is determined by a process that does not include aligning the sequence reads to a reference genome.

A2. The method of any one of items A1 to A1.2, wherein the count B is:

(i) a count of total reads generated by a nucleic acid sequencing process used to sequence the nucleic acid from the test sample;
(ii) a count of a fraction of total reads generated by a nucleic acid sequencing process used to sequence the nucleic acid from the test sample;
(iii) a count of the total reads of (i) or the fraction of the total reads of (ii), less reads filtered according to a quality control metric for the sequencing process;
(iv) a count of the total reads of (i) or the fraction of the total reads of (ii), weighted according to a quality control metric for the sequencing process;
(v) a count of the total reads of (i) or the fraction of the total reads of (ii), less reads filtered according to read base content;
(vi) a count of the total reads of (i) or the fraction of the total reads of (ii), weighted according to read base content; or
(vii) a count of reads that match polynucleotides in a listing, wherein the reads are determined to match or not match the polynucleotides in the listing in a process comprising comparing reads to the polynucleotides in the listing, wherein the reads are the total reads in (i), the fraction of total reads in (ii), the total reads of (i) or the fraction of the total reads of (ii) less the reads filtered according to the quality control metric of (iii), the total reads of (i) or the fraction of the total reads of (ii) weighted according to the quality control metric of (iv), the total reads of (i) or the fraction of the total reads of (ii) less the reads filtered according to the read base content of (v), or the total reads of (i) or the fraction of the total reads of (ii) weighted according to the read base content of (vi).

A3. The method of item A2, wherein the fraction is a fraction of randomly selected reads from the total reads.

A4. The method of item A2 or A3, wherein the fraction is about 10% to about 90% of the total reads.

A5. The method of any one of item A2 to A4, wherein the nucleic acid sequencing process comprises image processing and the quality control metric is based on image quality.

A6. The method of item A5, wherein the quality control metric is based on an assessment of image overlap.

A7. The method of any one of items A2 to A6, wherein the read base content is guanine and cytosine (GC) content.

A8. The method of item A7, wherein the reads filtered in (v) have a GC content less than a first GC threshold.

A8.1. The method of item A7, wherein the reads filtered in (v) have a GC content greater than a second GC threshold.

A9. The method of any one of items A2 to A8.1, wherein the count in (vii) is a count of reads that exactly match sequence and size of the polynucleotides in the listing.

A9.1. The method of any one of items A2 to A9, wherein the polynucleotides in the listing were aligned, prior to (a), to a reference genome, or the subset in a reference genome.

A9.2. The method of item A9.1, wherein the subset in the reference genome is all autosomes or a subset of all autosomes.

A9.3. The method of item A9.1 or A9.2, wherein the comparing does not include tracking (i) a chromosome to which each polynucleotide aligns, and/or (ii) a chromosome position number at which each polynucleotide aligns.

A10. The method of any one of items A1 to A9.3, comprising subjecting the reads to an alignment process that aligns reads with a reference genome, wherein the count B is determined prior to subjecting the reads to the alignment process.

A11. The method of item A1, comprising subjecting the reads to an alignment process that aligns reads with a reference genome, wherein the count B is a count of reads not aligned to the reference genome by the alignment process.

A12. The method of any one of items A1 to A11, comprising subjecting the reads to an alignment process that aligns reads with a reference genome, wherein the count A is a count of reads aligned to the segment in the reference genome.

A13. The method of any one of items A1 to A11, wherein the count A is determined by a process that does not include aligning the sequence reads to a reference genome.

A14. The method of item A13, wherein the count A is a count of reads that match polynucleotides in a listing or a subset of a listing, wherein the reads are determined to match or not match the polynucleotides in the listing or the subset of the listing in a process comprising comparing reads to the polynucleotides in the listing or the subset of the listing.

A14.1. The method of item A14, wherein the reads compared to the polynucleotides in the listing or the subset of the listing are the total reads in item A2(i); the fraction of total reads in item A2(ii); the total reads of item A2(i) or the fraction of the total reads of item A2(ii) less the reads filtered according to the quality control metric of item A2(iii); the total reads of item A2(i) or the fraction of the total reads of item A2(ii) weighted according to the quality control metric of item A2(iv); the total reads of item A2(i) or the fraction of the total reads of item A2(ii) less the reads filtered according to the read base content of item A2(v); or the total reads of item A2(i) or the fraction of the total reads of item A2(ii) weighted according to the read base content of item A2(vi).

A14.2. The method of item A14 or A14.1, wherein the count A is a count of reads that exactly match sequence and size of the polynucleotides in the listing or the subset of the listing.

A14.3. The method of any one of items A14 to A14.2, wherein the polynucleotides in the listing or the subset of the listing were aligned, prior to (a), to the segment in a reference genome.

A14.4. The method of item A14.3, wherein the comparing does not include tracking (i) a chromosome to which each polynucleotide aligns, and/or (ii) a chromosome position number at which each polynucleotide aligns.

A14.5. The method of any one of items A1 to A9.3 and A13 to A14.4, wherein the sequence reads are not subjected to an alignment process that aligns the sequence reads to the reference genome in (a), (b) and (c).

A14.6. The method of any one of items A1 to A9.3 and A13 to A14.4, wherein the sequence reads are not subjected to an alignment process that aligns the sequence reads to the reference genome in the diagnostic test.

A15. The method of any one of items A1 to A14.6, wherein the segment is a chromosome.

A16. The method of item A15, wherein the chromosome is chosen from chromosome 13, chromosome 18 and chromosome 21.

A17. The method of any one of items A1 to A14, wherein the segment is a segment of a chromosome.

A18. The method of item A17, wherein the segment is a microduplication or microdeletion region.

A19. The method of any one of items A1 to A18, wherein the ratio in (c) is the count A divided by the count B.

A20. The method of any one of items A1 to A18, wherein the ratio in (c) is the count B divided by the count A.

A21. The method of any one of items A1 to A20, wherein the nucleic acid is circulating cell-free nucleic acid.

A22. The method of any one of items A1 to A21, wherein the diagnostic test is a prenatal diagnostic test and the test sample is from a pregnant female bearing a fetus. A23. The method of any one of items A1 to A21, wherein the diagnostic test is a test for presence, absence, increased risk, or decreased risk of a cell proliferative condition.

A24. The method of any one of items A1 to A23, comprising determining a statistic of the count representation for the segment.

A25. The method of item A24, wherein the statistic is a z-score.

A26. The method of item A25, wherein the z-score is a quotient of (a) a subtraction product of (i) the count representation for the segment for the test sample, less (ii) a median of a count representation for the segment for a sample set, divided by (b) a MAD of the count representation for the segment for the sample set.

A27. The method of item A26, wherein: the diagnostic test is a prenatal diagnostic test, the test sample is from a pregnant female bearing a fetus, and the sample set is a set of samples for subjects having euploid fetus pregnancies.

A28. The method of item A26, wherein: the diagnostic test is a prenatal diagnostic test, the test sample is from a pregnant female bearing a fetus, and the sample set is a set of samples for subjects having trisomy fetus pregnancies.

A29. The method of item A26, wherein: the diagnostic test is for presence, absence, increased risk, or decreased risk of a cell proliferative condition, and the sample set is a set of samples for subjects having the cell proliferative condition.

A30. The method of item A26, wherein: the diagnostic test is for presence, absence, increased risk, or decreased risk of a cell proliferative condition, and the sample set is a set of samples for subjects not having the cell proliferative condition.

A31. The method of any one of items A1 to A30, wherein the count A is of normalized counts.

A32. The method of any one of items A1 to A31, wherein the count B is of normalized counts.

A33. The method of item A31 or A32, wherein the normalized counts are generated by a normalization process comprising a LOESS normalization process.

A34. The method of any one of items A31 to A33, wherein the normalized counts are generated by a normalization process comprising a guanine and cytosine (GC) bias normalization.

A35. The method of any one of items A31 to A34, wherein the normalized counts are generated by a normalization process comprising LOESS normalization of GC bias (GC-LOESS).

A36. The method of any one of items A31 to A35, wherein the normalized counts are generated by a normalization process comprising principal component normalization.

A37. The method of any one of items A1 to A36, wherein: the diagnostic test is a prenatal diagnostic test, the test sample is from a pregnant female bearing a fetus, and the diagnostic test comprises determining presence of absence of a genetic variation.

A38. The method item A37, wherein the genetic variation is a chromosome aneuploidy.

A39. The method of item A38, wherein the chromosome aneuploidy is one, three or four copies of a whole chromosome.

A40. The method of item A37, wherein the genetic variation is a microduplication or microdeletion.

A41. The method of any one of items A37 to A40, wherein the genetic variation is a fetal genetic variation.

A42. The method of any one of items A1 to A36, wherein: the diagnostic test is for presence, absence, increased risk, or decreased risk of a cell proliferative condition, and the diagnostic test comprises determining presence of absence of a genetic variation.

A43. The method of item A42, wherein the genetic variation is a microduplication or microdeletion.

A44. The method of any one of items A1 to A43, wherein one or more or all of (a), (b) and (c) are performed by a microprocessor in a system.

A45. The method of any one of claims A1 to A44, wherein one or more or all of (a), (b) and (c) are performed in conjunction with memory in a system.

A46. The method of any one of items A1 to A45, wherein one or more or all of (a), (b) and (c) are performed by a computer.

B1. A system comprising one or more microprocessors and memory, which memory comprises instructions executable by the one or more microprocessors and which memory comprises nucleotide sequence reads, which sequence reads are reads of nucleic acid from a test sample from a subject, and which instructions executable by the one or more microprocessors are configured to:

> (a) generate, using a microprocessor, a count of nucleic acid sequence reads for a genome segment, which sequence reads are reads of nucleic acid from a test sample from a subject having the genome, thereby providing a count A for the segment;
> (b) generate, using a microprocessor, a count of nucleic acid sequence reads for the genome or a subset of the genome, thereby providing a count B for the genome or subset of the genome, wherein the count $B$ is a count of sequence reads not aligned to a reference genome; and
> (c) determine a count representation for the segment as a ratio of the count A to the count B.

B2. A machine comprising one or more microprocessors and memory, which memory comprises instructions executable by the one or more microprocessors and which memory comprises nucleotide sequence reads, which sequence reads are reads of nucleic acid from a test sample from a subject, and which instructions executable by the one or more microprocessors are configured to:

> (a) generate, using a microprocessor, a count of nucleic acid sequence reads for a genome segment, which sequence reads are reads of nucleic acid from a test sample from a subject having the genome, thereby providing a count $A$ for the segment;
> (b) generate, using a microprocessor, a count of nucleic acid sequence reads for the genome or a subset of the genome, thereby providing a count $B$ for the genome or subset of the genome, wherein the count $B$ is a count of sequence reads not aligned to a reference genome; and
> (c) determine a count representation for the segment as a ratio of the count $A$ to the count $B$.

B3. A non-transitory computer-readable storage medium with an executable program stored thereon, wherein the program instructs a microprocessor to perform the following:

> (a) access nucleotide sequence reads, which sequence reads are reads of nucleic acid from a test sample from a subject;
> (b) generate, using a microprocessor, a count of nucleic acid sequence reads for a genome segment, which sequence reads are reads of nucleic acid from a test sample from a subject having the genome, thereby providing a count $A$ for the segment;
> (c) generate, using a microprocessor, a count of nucleic acid sequence reads for the genome or a subset of the genome, thereby providing a count $B$ for the genome or subset of the genome, wherein the count $B$ is a count of sequence reads not aligned to a reference genome; and
> (d) determine a count representation for the segment as a ratio of the count $A$ to the count $B$.

The drawings illustrate certain embodiments of the technology and are not limiting. For clarity and ease of illustration, the drawings are not made to scale and, in some instances, various aspects may be shown exaggerated or enlarged to facilitate an understanding of particular embodiments.

The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%).

Certain embodiments of the technology are set forth in the claim(s) that follow(s).

**Claims**

1. A method for determining a sequence read count representation of a genome segment for a diagnostic test, comprising:

   (a) generating a count of nucleic acid sequence reads for a genome segment, which sequence reads are reads of nucleic acid from a test sample from a subject having the genome, thereby providing a count *A* for the segment;
   (b) generating a count of nucleic acid sequence reads for the genome or a subset of the genome, thereby providing a count *B* for the genome or subset of the genome, wherein the count *B* is a count of sequence reads that is determined by a process that does not include aligning the sequence reads to a reference genome; and
   (c) determining a count representation for the segment as a ratio of the count *A* to the count *B,*

   wherein one or more or all of (a), (b) and (c) are performed by a computer, wherein the count *B* is:

   (i) a count of total reads generated by a nucleic acid sequencing process used to sequence the nucleic acid from the test sample;
   (ii) a count of a fraction of total reads generated by a nucleic acid sequencing process used to sequence the nucleic acid from the test sample;
   (iii) a count of the total reads of (i) or the fraction of the total reads of (ii), less reads filtered according to a quality control metric for the sequencing process;
   (iv) a count of the total reads of (i) or the fraction of the total reads of (ii), weighted according to a quality control metric for the sequencing process;
   (v) a count of the total reads of (i) or the fraction of the total reads of (ii), less reads filtered according to read base content;
   (vi) a count of the total reads of (i) or the fraction of the total reads of (ii), weighted according to read base content; or
   (vii) a count of reads that match polynucleotides in a listing, wherein the reads are determined to match or not match the polynucleotides in the listing in a process comprising comparing reads to the polynucleotides in the listing, wherein the reads are the total reads in (i), the fraction of total reads in (ii), the total reads of (i) or the fraction of the total reads of (ii) less the reads filtered according to the quality control metric of (iii), the total reads of (i) or the fraction of the total reads of (ii) weighted according to the quality control metric of (iv), the total reads of (i) or the fraction of the total reads of (ii) less the reads filtered according to the read base content of (v), or the total reads of (i) or the fraction of the total reads of (ii) weighted according to the read base content of (vi).

2. The method of claim 1, wherein the fraction is a fraction of randomly selected reads from the total reads.

3. The method of claim 1 or 2, wherein the read base content is guanine and cytosine (GC) content.

4. The method of claim 1, 2 or 3, wherein the polynucleotides in the listing were aligned, prior to (a), to a reference genome, or the subset in a reference genome.

5. The method of claim 4, wherein the comparing does not include tracking (i) a chromosome to which each polynucleotide aligns, and/or (ii) a chromosome position number at which each polynucleotide aligns.

6. The method of any one of claims 1 to 5, comprising subjecting the reads to an alignment process that aligns reads with a reference genome, wherein the count *B* is determined prior to subjecting the reads to the alignment process.

7. The method of any one of claims 1 to 6, comprising subjecting the reads to an alignment process that aligns reads with a reference genome, wherein the count *A is* a count of reads aligned to the segment in the reference genome.

8. The method of any one of claims 1 to 6, wherein the count *A* is determined by a process that does not include aligning the sequence reads to a reference genome.

9. The method of claim 8, wherein the count $A$ is a count of reads that match polynucleotides in a listing or a subset of a listing, wherein the reads are determined to match or not match the polynucleotides in the listing or the subset of the listing in a process comprising comparing reads to the polynucleotides in the listing or the subset of the listing.

10. The method of claim 9, wherein the reads compared to the polynucleotides in the listing or the subset of the listing are

   (i) a count of total reads generated by a nucleic acid sequencing process used to sequence the nucleic acid from the test sample;
   (ii) a count of a fraction of total reads generated by a nucleic acid sequencing process used to sequence the nucleic acid from the test sample;
   (iii) a count of the total reads of (i) or the fraction of the total reads of (ii), less reads filtered according to a quality control metric for the sequencing process;
   (iv) a count of the total reads of (i) or the fraction of the total reads of (ii), weighted according to a quality control metric for the sequencing process;
   (v) a count of the total reads of (i) or the fraction of the total reads of (ii), less reads filtered according to read base content; or
   (vi) a count of the total reads of (i) or the fraction of the total reads of (ii), weighted according to read base content.

11. The method of claim 9 or 10, wherein the polynucleotides in the listing or the subset of the listing were aligned, prior to (a), to the segment in a reference genome.

12. The method of claim 11, wherein the comparing does not include tracking (i) a chromosome to which each polynucleotide aligns, and/or (ii) a chromosome position number at which each polynucleotide aligns.

13. The method of any one of claims 1 to 5 and 8 to 12, wherein the sequence reads are not subjected to an alignment process that aligns the sequence reads to the reference genome in (a), (b) and (c).

14. The method of any one of claims 1 to 13, wherein the count $A$ is of normalized counts, the count $B$ is of normalized counts, or the count $A$ is of normalized counts and the count $B$ is of normalized counts; wherein the normalized counts are generated by one or more normalization processes selected from a LOESS normalization process, a guanine and cytosine (GC) bias normalization, LOESS normalization of GC bias (GC-LOESS), and principal component normalization.

15. The method of any one of claims 1 to 14, wherein count B is a count of sequence reads that is determined by a comparison process that determines whether the sequence reads match or not match the polynucleotides in a listing.


**Patentansprüche**

1. Verfahren zur Bestimmung einer Darstellung der Anzahl gelesener Sequenzen eines genomischen Segments für einen diagnostischen Test, umfassend:

   (a) Generierung der Anzahl gelesener Sequenzen der Nukleinsäure für ein genomisches Segment, wobei die gelesenen Sequenzen Lesungen der Nukleinsäure aus einer Testprobe von einem Subjekt sind, das das Genom hat, wodurch eine Anzahl $A$ für das Segment bereitgestellt wird;
   (b) Generierung einer Anzahl gelesener Sequenzen der Nukleinsäure für das Genom oder ein Subset des Genoms, wodurch eine Anzahl $B$ für das Genom oder das Subset des Genoms bereitgestellt wird, wobei die Anzahl $B$ eine Anzahl von gelesenen Sequenzen ist, die durch ein Verfahren bestimmt wird, das keine Aneinanderlagerung der gelesenen Sequenzen an ein Referenzgenom umfasst; und
   (c) Bestimmung einer Darstellung der Anzahl für das Segment als Verhältnis der Anzahl $A$ zur Anzahl $B$,

   wobei eine oder mehrere oder alle von (a), (b) und (c) von einem Computer ausgeführt werden, wobei die Anzahl $B$ ist:

   (i) eine Anzahl der gesamten Lesungen, die durch einen Nukleinsäuresequenzierungsprozess generiert werden, verwendet um die Nukleinsäure aus der Testprobe zu sequenzieren;
   (ii) eine Anzahl eines Anteils der gesamten Lesungen, die durch einen Nukleinsäuresequenzierungsprozess generiert werden, verwendet um die Nukleinsäure aus der Testprobe zu sequenzieren;

(iii) eine Anzahl der gesamten Lesungen nach (i) oder des Anteils der gesamten Lesungen nach (ii), abzüglich der Lesungen, die nach einer Qualitätskontrollmetrik für den Sequenzierungsprozess gefiltert wurden;

(iv) eine Anzahl der gesamten Lesungen nach (i) oder des Anteils der gesamten Lesungen nach (ii), gewichtet nach einer Qualitätskontrollmetrik für den Sequenzierungsprozess;

(v) eine Anzahl der gesamten Lesungen nach (i) oder des Anteils der gesamten Lesungen nach (ii), abzüglich der Lesungen, die nach dem Basisgehalt der Lesungen gefiltert wurden;

(vi) eine Anzahl der gesamten Lesungen nach (i) oder des Anteils der gesamten Lesungen nach (ii), gewichtet nach dem Basisgehalt der Lesungen; oder

(vii) eine Anzahl von Lesungen, die Polynukleotiden in einer Liste entsprechen, wobei die Lesungen als den Polynukleotiden in einer Liste entsprechend oder nicht entsprechend bestimmt werden in einem Verfahren, das den Vergleich der Lesungen mit den Polynukleotiden in der Liste umfasst, wobei die Lesungen die gesamten Lesungen nach (i), der Anteil der gesamten Lesungen nach (ii), die gesamten Lesungen nach (i) oder der Anteil der gesamten Lesungen nach (ii), abzüglich der nach der Qualitätskontrollmetrik gefilterten Lesungen nach (iii), die gesamten Lesungen nach (i) oder der Anteil der gesamten Lesungen nach (ii), gewichtet nach der Qualitätskontrollmetrik nach (iv), die gesamten Lesungen nach (i) oder der Anteil der gesamten Lesungen nach (ii), abzüglich der nach dem Basisgehalt der Lesungen gefilterten Lesungen nach (v), oder die gesamten Lesungen nach (i) oder der Anteil der gesamten Lesungen nach (ii), gewichtet nach dem Basisgehalt der Lesungen nach (vi) sind.

2. Verfahren nach Anspruch 1, wobei die Fraktion eine Fraktion zufällig ausgewählter Lesungen der gesamten Lesungen ist.

3. Verfahren nach Anspruch 1 oder 2, wobei der Basisgehalt der Lesungen der Guanin und Cytosin (GC) Gehalt ist.

4. Verfahren nach Anspruch 1, 2 oder 3, wobei die Polynukleotide in der Liste vor dem Schritt (a) an ein Referenzgenom angelagert wurden oder das Subset in einem Referenzgenom.

5. Verfahren nach Anspruch 4, wobei das Vergleichen nicht das Nachverfolgen (i) eines Chromosoms, an das sich jedes Polynukleotid anlagert und/oder (ii) einer Chromosomenpositionsnummer, an die sich jedes Polynukleotid anlagert umfasst.

6. Verfahren nach einem der Ansprüche 1 bis 5, das das Einbringen der Lesungen in einen Aneinanderlagerungsprozess umfasst, der Lesungen mit einem Referenzgenom aneinanderlagert, wobei die Anzahl *B* vor dem Einbringen der Lesungen in den Aneinanderlagerungsprozess bestimmt wird.

7. Verfahren nach einem der Ansprüche 1 bis 6, das das Einbringen der Lesungen in einen Aneinanderlagerungsprozess umfasst, der Lesungen mit einem Referenzgenom aneinanderlagert, wobei die Anzahl *A* eine Anzahl von Lesungen ist, die an das Segment in dem Referenzgenom angelagert werden.

8. Verfahren nach einem der Ansprüche 1 bis 6, wobei die Anzahl *A* durch ein Verfahren bestimmt wurde, das die Aneinanderlagerung der Sequenzlesungen mit einem Referenzgenom nicht umfasst.

9. Verfahren nach Anspruch 8, wobei die Anzahl *A* eine Anzahl von Lesungen ist, die Polynukleotiden in einer Liste oder einem Subset der Liste entsprechen, wobei die Lesungen als den Polynukleotiden in der Liste oder dem Subset der Liste entsprechend oder nicht entsprechend bestimmt werden in einem Verfahren, das den Vergleich der Lesungen mit den Polynukleotiden in der Liste oder dem Subset der Liste umfasst.

10. Verfahren nach Anspruch 9, wobei die Lesungen, die mit den Polynukleotiden in der Liste oder dem Subset der Liste verglichen werden, sind

(i) eine Anzahl der gesamten Lesungen, die durch einen Nukleinsäuresequenzierungsprozess generiert werden, verwendet um die Nukleinsäure aus der Testprobe zu sequenzieren;

(ii) eine Anzahl eines Anteils der gesamten Lesungen, die durch einen Nukleinsäuresequenzierungsprozess generiert werden, verwendet um die Nukleinsäure aus der Testprobe zu sequenzieren;

(iii) eine Anzahl der gesamten Lesungen nach (i) oder des Anteils der gesamten Lesungen nach (ii), abzüglich der Lesungen, die nach einer Qualitätskontrollmetrik für den Sequenzierungsprozess gefiltert wurden;

(iv) eine Anzahl der gesamten Lesungen nach (i) oder des Anteils der gesamten Lesungen nach (ii), gewichtet nach einer Qualitätskontrollmetrik für den Sequenzierungsprozess;

(v) eine Anzahl der gesamten Lesungen nach (i) oder des Anteils der gesamten Lesungen nach (ii), abzüglich der Lesungen, die nach dem Basisgehalt der Lesungen gefiltert wurden; oder

(vi) eine Anzahl der gesamten Lesungen nach (i) oder des Anteils der gesamten Lesungen nach (ii), gewichtet nach dem Basisgehalt der Lesungen.

11. Verfahren nach Anspruch 9 oder 10, wobei die Polynukleotide in der Liste oder dem Subset der Liste vor dem Schritt (a), an das Segment in einem Referenzgenom aneinandergelagert wurden.

12. Verfahren nach Anspruch 11, wobei das Vergleichen nicht das Nachverfolgen (i) eines Chromosoms, an das sich jedes Polynukleotid anlagert und/oder (ii) einer Chromosomenpositionsnummer, an die sich jedes Polynukleotid anlagert umfasst.

13. Verfahren nach einem der Ansprüche 1 bis 5 und 8 bis 12, wobei die Sequenzlesungen keinem Aneinanderlagerungsprozess ausgesetzt werden, der die Sequenzlesungen an das Referenzgenom in (a), (b) und (c) aneinanderlagert.

14. Verfahren nach einem der Ansprüche 1 bis 13, wobei die Anzahl $A$ aus normalisierten Anzahlen besteht, die Anzahl $B$ aus normalisierten Anzahlen besteht, oder die Anzahl A aus normalisierten Anzahlen besteht und die Anzahl B aus normalisierten Anzahlen besteht, wobei die normalisierten Anzahlen durch eine oder mehrere Normalisierungsverfahren generiert sind ausgewählt aus einem LOESS Normalisierungsverfahren, einer Guanin und Cytosin (GC) systemischer Fehler Normalisierung, LOESS Normalisierung des GC systemischen Fehlers (GC-LOESS) und Hauptkomponentenormalisierung.

15. Verfahren nach einem der Ansprüche 1 bis 14, wobei die Anzahl $B$ eine Anzahl von Sequenzlesungen ist, die durch ein Vergleichsverfahren bestimmt wird, das bestimmt, ob die Sequenzlesungen mit den Polynukleotiden in einer Liste übereinstimmen oder nicht.

## Revendications

1. Méthode pour déterminer une représentation de compte de lectures de séquence d'un segment génomique pour un test diagnostique, comprenant :

(a) la génération d'un compte de lectures de séquence d'acide nucléique pour un segment génomique, lesquelles lectures de séquence sont des lectures d'acide nucléique provenant d'un échantillon de test issu d'un sujet ayant le génome, ce qui donne ainsi un compte $A$ pour le segment ;

(b) la génération d'un compte de lectures de séquence d'acide nucléique pour le génome ou un sous-ensemble du génome, ce qui donne ainsi un compte $B$ pour le génome ou le sous-ensemble du génome, lequel compte $B$ est un compte de lectures de séquence qui est déterminé par un procédé qui ne comprend pas un alignement des lectures de séquence avec un génome de référence ; et

(c) la détermination d'une représentation de compte pour le segment sous la forme du rapport du compte $A$ au compte $B$,

dans laquelle une ou plusieurs ou la totalité parmi (a), (b) et (c) sont effectuées par un ordinateur, dans laquelle le compte $B$ est :

(i) un compte des lectures totales générées par un procédé de séquençage d'acide nucléique utilisé pour séquencer l'acide nucléique provenant de l'échantillon de test ;

(ii) un compte d'une fraction des lectures totales générées par un procédé de séquençage d'acide nucléique utilisé pour séquencer l'acide nucléique provenant de l'échantillon de test ;

(iii) un compte des lectures totales de (i) ou de la fraction des lectures totales de (ii), moins les lectures filtrées en fonction d'une métrique de contrôle de qualité pour le procédé de séquençage ;

(iv) un compte des lectures totales de (i) ou de la fraction des lectures totales de (ii) pondérées en fonction d'une métrique de contrôle de qualité pour le procédé de séquençage ;

(v) un compte des lectures totales de (i) ou de la fraction des lectures totales de (ii), moins les lectures filtrées en fonction de la teneur en bases lues ;

(vi) un compte des lectures totales de (i) ou de la fraction des lectures totales de (ii) pondérées en fonction de la teneur en bases lues ; ou

(vii) un compte de lectures qui correspondent à des polynucléotides dans une liste, lesquelles lectures sont déterminées comme correspondant ou ne correspondant pas aux polynucléotides dans la liste dans un procédé comprenant la comparaison des lectures avec les polynucléotides dans la liste, lesquelles lectures sont les lectures totales dans (i), la fraction des lectures totales dans (ii), les lectures totales de (i) ou la fraction des lectures totales de (ii) moins les lectures filtrées en fonction de la métrique de contrôle de qualité de (iii), les lectures totales de (i) ou la fraction des lectures totales de (ii) pondérées en fonction de la métrique de contrôle de qualité de (iv), les lectures totales de (i) ou la fraction des lectures totales de (ii) moins les lectures filtrées en fonction de la teneur en bases lues de (v), ou les lectures totales de (i) ou la fraction des lectures totales de (ii) pondérées en fonction de la teneur en bases lues de (vi).

2. Méthode selon la revendication 1, dans laquelle la fraction est une fraction de lectures sélectionnées au hasard parmi les lectures totales.

3. Méthode selon la revendication 1 ou 2, dans laquelle la teneur en bases lues est la teneur en guanine et en cytosine (GC).

4. Méthode selon la revendication 1, 2 ou 3, dans laquelle les polynucléotides dans la liste étaient alignés, avant (a), avec un génome de référence, ou le sous-ensemble dans un génome de référence.

5. Méthode selon la revendication 4, dans laquelle la comparaison ne comprend pas le pistage (i) d'un chromosome avec lequel chaque polynucléotide s'aligne, et/ou (ii) d'un numéro de position de chromosome au niveau de laquelle chaque polynucléotide s'aligne.

6. Méthode selon l'une quelconque des revendications 1 à 5, comprenant la soumission des lectures à un procédé d'alignement qui aligne les lectures avec un génome de référence, dans laquelle le compte $B$ est déterminé avant soumission des lectures au procédé d'alignement.

7. Méthode selon l'une quelconque des revendications 1 à 6, comprenant la soumission des lectures à un procédé d'alignement qui aligne les lectures avec un génome de référence, dans laquelle le compte $A$ est un compte de lectures alignées avec le segment dans le génome de référence.

8. Méthode selon l'une quelconque des revendications 1 à 6, dans laquelle le compte $A$ est déterminé par un procédé qui ne comprend pas l'alignement des lectures de séquence avec un génome de référence.

9. Méthode selon la revendication 8, dans laquelle le compte $A$ est un compte de lectures qui correspondent à des polynucléotides dans une liste ou un sous-ensemble d'une liste, dans laquelle les lectures sont déterminées comme correspondant ou ne correspondant pas aux polynucléotides dans la liste ou le sous-ensemble de la liste dans un procédé comprenant la comparaison avec lectures avec les polynucléotides dans la liste ou le sous-ensemble de la liste.

10. Méthode selon la revendication 9, dans laquelle les lectures comparées aux polynucléotides dans la liste ou le sous-ensemble de la liste sont

(i) un compte de lectures totales générées par un procédé de séquençage d'acide nucléique utilisé pour séquencer l'acide nucléique provenant de l'échantillon de test ;
(ii) un compte d'une fraction des lectures totales générées par un procédé de séquençage d'acide nucléique utilisé pour séquencer l'acide nucléique provenant de l'échantillon de test ;
(iii) un compte des lectures totales de (i) ou de la fraction des lectures totales de (ii), moins les lectures filtrées en fonction d'une métrique de contrôle de qualité pour le procédé de séquençage ;
(iv) un compte des lectures totales de (i) ou de la fraction des lectures totales de (ii) pondérées en fonction d'une métrique de contrôle de qualité pour le procédé de séquençage;
(v) un compte des lectures totales de (i) ou de la fraction des lectures totales de (ii), moins les lectures filtrées en fonction de la teneur en bases lues ; ou
(vi) un compte des lectures totales de (i) ou de la fraction des lectures totales de (ii) pondérées en fonction de la teneur en bases lues.

11. Méthode selon la revendication 9 ou 10, dans laquelle les polynucléotides dans la liste ou le sous-ensemble de la liste étaient alignés, avant (a), avec le segment dans un génome de référence.

12. Méthode selon la revendication 11, dans laquelle la comparaison ne comprend pas le pistage (i) d'un chromosome avec lequel chaque polynucléotide s'aligne, et/ou (ii) d'un numéro de position de chromosome au niveau de laquelle chaque polynucléotide s'aligne.

13. Méthode selon l'une quelconque des revendications 1 à 5 et 8 à 12, dans laquelle les lectures de séquence ne sont pas soumises à un procédé d'alignement qui aligne les lectures de séquence avec le génome de référence dans (a), (b) et (c).

14. Méthode selon l'une quelconque des revendications 1 à 13, dans laquelle le compte *A* est celui de comptes normalisés, le compte *B* est celui de comptes normalisés, ou bien le compte *A* est celui de comptes normalisés et le compte *B* est celui de comptes normalisés ; dans laquelle les comptes normalisés sont générés par un ou plusieurs procédés de normalisation choisis parmi un procédé de normalisation LOESS, un de biais de normalisation de guanine et de cytosine (GC), un biais de normalisation LOESS de GC (GC-LOESS), et une normalisation des composantes principales.

15. Méthode selon l'une quelconque des revendications 1 à 14, dans laquelle le compte B est un compte de séquences lues qui est déterminé par un procédé de comparaison qui détermine si les lectures de séquence correspondent ou ne correspondent pas aux polynucléotides dans une liste.

**FIG. 1**

LDTv4 CE2, N=343

FIG. 2

LDTv4 CE2, N=343

FIG. 3A

FIG. 3B

FIG. 3C

chr21

chr18

chr13

## FIG. 4

```
┌─────────────────────────────┐              ┌─────────────────────────────────────┐
│   Raw files from sequencer  │              │       Human reference genome        │
└─────────────────────────────┘              └─────────────────────────────────────┘
              │                                                 │               450
              ▼            410                                  ▼
┌─────────────────────────────┐              ┌─────────────────────────────────────┐
│  Bclconvert and Demultiplex │              │  Identify unique k-mers for each    │
└─────────────────────────────┘              │      chromosome (dictionary)        │
              │                               └─────────────────────────────────────┘
              ▼            420                                  │               460
┌─────────────────────────────┐              ┌─────────────────────────────────────┐
│ Individual fastq files for  │─────────────▶│   Look up reads belonging to target │
│        each sample          │              │  chromosome using dictionary[2]     │
└─────────────────────────────┘              └─────────────────────────────────────┘
              │            430                                  │               470
              ▼                                                 ▼
┌─────────────────────────────┐              ┌─────────────────────────────────────┐
│ Count the total number of   │              │  Count the number of reads on target│
│ reads pass chastity filter[1]│             │      chromosome $N_{target}$        │
│         $N_{tot}$           │              └─────────────────────────────────────┘
└─────────────────────────────┘                                │               480
         440  │                                                 │
             └──────────────────────┬──────────────────────────┘
                                    ▼
                  ┌─────────────────────────────────────┐
                  │  Chr_fraction= $N_{target} / N_{tot}$│
                  └─────────────────────────────────────┘
                                               490
```

EP 3 149 640 B1

# FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20130130921 A
- US 20130150253 A
- US 2014039389 W
- WO 2014190286 A
- US 2014058885 W
- WO 2015051163 A
- WO 2013177086 A
- US 20100105049 A
- US 0769991 W
- US 2007071232 W
- US 60968876 B
- US 60968878 B
- EP 05012707 W
- US 20050112590 A
- US 6927028 B
- WO 2007140417 A
- WO 2007147063 A
- WO 2009032779 A
- WO 2009032781 A
- WO 2010033639 A
- WO 2011034631 A
- WO 2006056480 A
- WO 2011143659 A
- US 20090317818 A
- WO 2010115016 A
- US 20130012399 A
- WO 2013052907 A
- US 1259123 W
- WO 2013052913 A
- US 2012059123 W
- US 20130085681 A

### Non-patent literature cited in the description

- **T. STRACHAN.** The Human Genome. BIOS Scientific Publishers, 1992
- **D. N. COOPER ; M. KRAWCZAK.** Human Genome Mutations. BIOS Publishers, 1993
- **SAMBROOK ; RUSSELL.** Molecular Cloning: A Laboratory Manual. 2001
- Current Protocols in Molecular Biology. John Wiley & Sons, 1989, 6.3.1-6.3.6
- Illumina's Genome Analyzer; Genome Analyzer II. Illumina, 2000
- Illumina's Genome Analyzer; Genome Analyzer II. 2000